# EUROPEAN PATENT SPECIFICATION

(11) **EP 3 494 228 B1**
(45) Date of publication and mention of the grant of the patent: **07.07.2021**
(21) Application number: 17751048.4
(22) Date of filing: 27.07.2017
(51) Int. Cl.: C12Q 1/68

(54) **METHOD FOR PROGNOSING AND DIAGNOSING TUMORS**
VERFAHREN ZUR PROGNOSE VON TUMOREN
PROCÉDÉ DE PRONOSTIC DE TUMEURS

(30) Priority: 03.08.2016 EP 16182557; 12.04.2017 EP 17166256
(43) Date of publication of application: 12.06.2019
(73) Proprietor: CBmed GmbH Center for Biomarker Research in Medicine, 8010 Graz (AT)
(72) Inventor: HAYBAECK, Johannes, 4020 Linz (AT); GOLOB-SCHWARZL, Nicole, 8010 Graz (AT); KRASSNIG, Stefanie, 8010 Graz (AT); THALER, Nadine, 9490 Vaduz (LI); UNTERLUGGAUER, Julia Judith, 9411 St. Michael im Lavanttal (AT); DEUTSCH, Alexander, 8045 Graz (AT)
(74) Representative: Geling, Andrea
(86) International application number: PCT/EP2017/069055
(87) International publication number: WO 2018/024609

(56) References cited:
- WO-A1-2016/033250
- YUGANG WANG ET AL: "Amino Acid Deprivation Promotes Tumor Angiogenesis through the GCN2/ATF4 Pathway", NEOPLASIA, vol. 15, no. 8, 1 August 2013 (2013-08-01) , pages 989-997, XP055429431, US ISSN: 1476-5586, DOI: 10.1593/neo.13262
- JIANGBIN YE ET AL: "The GCN2-ATF4 pathway is critical for tumour cell survival and proliferation in response to nutrient deprivation", EMBO (EUROPEAN MOLECULAR BIOLOGY ORGANIZATION) JOURNAL, vol. 29, no. 12, 16 June 2010 (2010-06-16) , pages 2082-2096, XP055429392, DE ISSN: 0261-4189, DOI: 10.1038/emboj.2010.81
- FLOWERS A ET AL: "Eukaryotic initiation factor 4E overexpression in triple-negative breast cancer predicts a worse outcome", SURGERY, MOSBY, INC, US, vol. 146, no. 2, 1 August 2009 (2009-08-01), pages 220-226, XP026336279, ISSN: 0039-6060, DOI: 10.1016/J.SURG.2009.05.010 [retrieved on 2009-07-21]
- TU LUXIA ET AL: "Over-expression of eukaryotic translation initiation factor 4 gamma 1 correlates with tumor progression and poor prognosis in nasopharyngeal carcinoma", MOLECULAR CANCER, BIOMED CENTRAL, LONDON, GB, vol. 9, no. 1, 16 April 2010 (2010-04-16), page 78, XP021078049, ISSN: 1476-4598, DOI: 10.1186/1476-4598-9-78
- SEKI N ET AL: "Prognostic significance of expression of eukaryotic initiation factor 4E and 4E binding protein 1 in patients with pathological stage I invasive lung adenocarcinoma", LUNG CANCER, ELSEVIER, AMSTERDAM, NL, vol. 70, no. 3, 1 December 2010 (2010-12-01), pages 329-334, XP027455996, ISSN: 0169-5002, DOI: 10.1016/J.LUNGCAN.2010.03.006 [retrieved on 2010-11-01]
- TOMMY ALAIN ET AL: "mTOR inhibitor efficacy is determined by the eIF4E/4E-BP ratio", ONCOTARGET, vol. 3, no. 12, 1 January 2012 (2012-01-01), pages 1491-1492, XP055324109, DOI: 10.18632/oncotarget.799
- DEBORAH SILVERA ET AL: "Translational control in cancer", NATURE REVIEWS CANCER, vol. 10, no. 4, 1 April 2010 (2010-04-01), pages 254-266, XP055194575, ISSN: 1474-175X, DOI: 10.1038/nrc2824

## Description

The present invention is in the field of tumor prognosis. It relates to a method of providing a prognosis to an individual suffering from a tumor. Moreover, the present invention relates to the use of a kit for conducting the above-mentioned method.

### BACKGROUND OF THE INVENTION

Proteins are crucial for the survival of every cell. They are synthesized from the genetic code (delivered in form of mRNA) by ribosomes, a process termed translation. Eukaryotic translation Initiation Factors (eIFs) are necessary for the first steps of the translation process, including especially the loading of the ribosome onto the mRNA by stabilizing the formation of the functional ribosome around the start codon. Furthermore, elFs provide also regulatory mechanisms in translation initiation.

A particularly important role in ribosome-mRNA attachment carries the eIF4F-complex, comprising the mRNA cap-binding protein eIF4E, the RNA helicase eIF4A and the scaffolding protein eIF4G, which mediates 40S ribosome binding through interaction with eIF3. After ribosome loading the ribosomal subunit and attached factors thereof scan the mRNA for the start codon. EIF1 considerably contributes to this scanning process. Following initiation codon recognition eIF5 and eIF5B then promote tRNA binding to the start codon, featured by eIF2, and as a consequence elF release and 60S ribosomal subunit binding and start of translation.

In a cellular context elF action is partly regulated by the mTOR-pathway, which incorporates both extracellular and intracellular signaling signals and is a central regulator of cell metabolism, growth, proliferation and survival.

Tumor cells may show an elF expression pattern which is different from that of healthy cells. Even between the various types of tumor elF expression may vary. In tumor cells some eIFs are upregulated whereas some other elFs are downregulated. Thus, elFs may be used as tumor markers.

For example, WO 2016/033250 A1 refers to the detection of levels of a breast cancer survivor population gene panel including eIF2AK4 to determine recurrence. Further, Tommy Alain et al. ("mTOR inhibitor efficacy is determined by the eIF4E/4E-BP ratio", Oncotarget, Vol. 3, No. 12, 2012, pages 1491 to 1492) relate to prognostic significance of ratios of eukaryotic initiation factors in liver cancer cells. Furthermore, Deborah Silvera et al. ("Translational control in cancer", Nature Reviews Cancer, Vol. 10, No. 4, 2010, pages 254 to266) relate to prognostic significance of eukaryotic initiation factors in liver cancer and lymphoma.

There is (still) a need for new and reliable elF tumor markers. Comprehensive studies analyzing the relationship between the whole range of elFs and patient outcome and analyzing the potential of the whole range of elFs as markers allowing the diagnosis of tumors or differential diagnosis between specific tumor entities have not been performed yet. The present inventors have carried out theses analysis and identified elFs which performed best in the prognosis of an individual suffering from a tumor, in the diagnosis of a tumor, or differential diagnosis between specific tumor identities. The identified elFs allow a reliable prognosis with respect to the life expectancy of an individual suffering from a tumor, tumor diagnosis in an individual or differential diagnosis between specific tumor entities in an individual.

### SUMMARY OF THE INVENTION

In a first aspect, the present invention relates to a method of providing a prognosis to an individual suffering from a tumor comprising the steps of:
a) determining the level of eIF2AK4 (Gene: EIF2AK4, GCN2; Gene ID (GenBank): 440275) in a sample from an individual, and
b) comparing the level of eIF2AK4 in said sample to a reference level A of eIF2AK4 determined in samples of patients suffering from the same tumor or to a reference level B of eIF2AK4 determined in samples of healthy individuals, wherein a decreased level of eIF2AK4 in the sample of said individual compared to the reference level A and/or B indicates a good prognosis,
wherein the tumor is a lymphoma or a hepatocellular carcinoma.

In a second aspect, the present invention relates to the use of a kit comprising means for determining the level of eIF2AK4 in a sample from an individual for providing a prognosis to the individual suffering from a tumor, wherein the tumor is a lymphoma or a hepatocellular carcinoma.

This summary of the invention does not necessarily describe all features of the present invention. Other embodiments will become apparent from a review of the ensuing detailed description.

### BRIEF DESCRIPTION OF THE FIGURES

**Figure 1** shows the expression/survival correlation for the 1^{st} quartile cut off level between high and low expression of eIF2C_3, eIF3g, eIF-4G1 and eIF-5 in DLBCL.
**Figure 2** shows the expression/survival correlation for the Median cut off level between high and low expression of eIF2AK3/HsPEK, eIF2B4 /eIF-2B subunit delta, eIF3c and 4E-BP1 in DLBCL.
**Figure 3** shows the expression/survival correlation for the 3^{rd} quartile cut off level between high and low expression of eIF2AK4, eIF2d, eIF-2α and eIF-2-beta/eIF2S2 in DLBCL.
**Figure 4** shows the expression/survival correlation for the 3^{rd} quartile cut off level between high and low expression of eIF3b, eIF3d, eIF3f and eIF31 in DLBCL.
**Figure 5** shows the expression/survival correlation for the 3^{rd} quartile cut off level between high and low expression of eIF-4B, eIF-4E3 and eIF-5A in DLBCL.

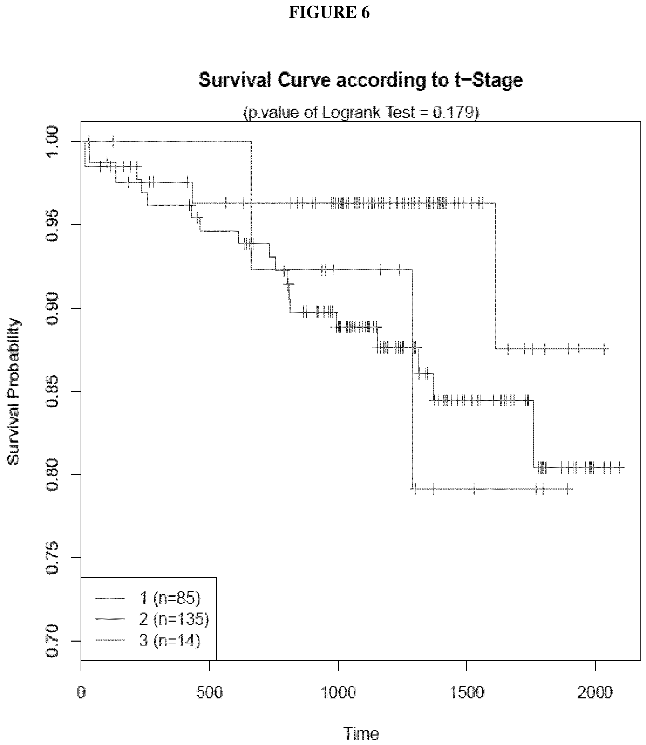

**Figure 6** shows a survival curve according to t-stage; 14 patients with a score of 3, 135 patients with score 2 and 85 patients with a score of 1.

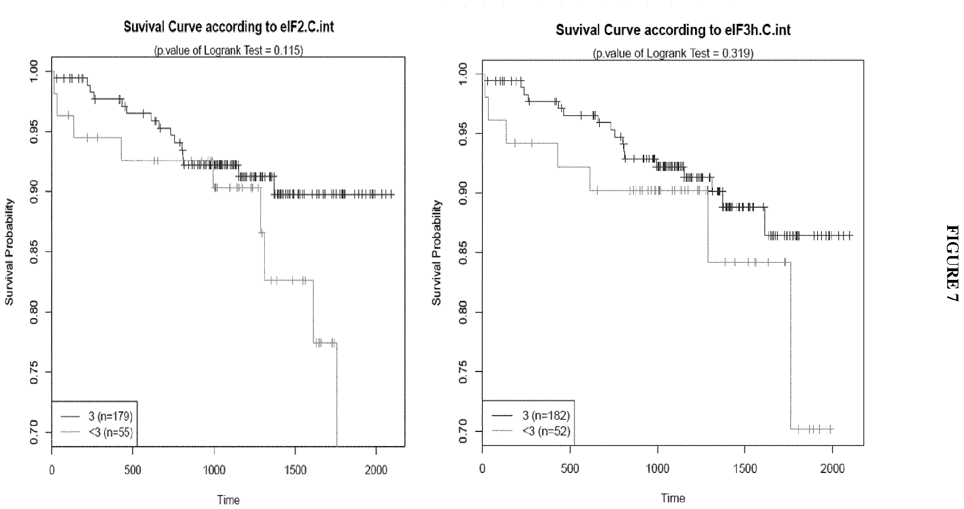

**Figure 7** shows a survival curve according to the eIF2α intensity. Survival curve according to the eIF3H intensity.

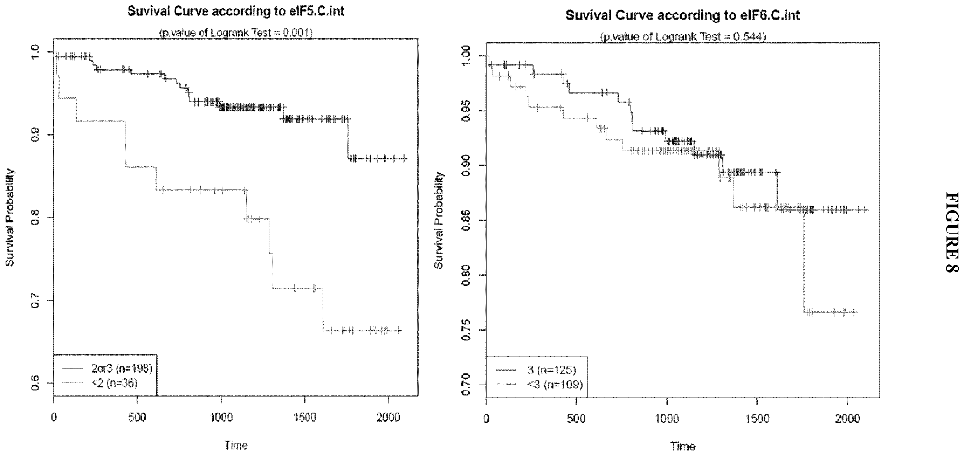

**Figure 8** shows a survival curve according to the eIF5 intensity. Survival curve according to the eIF6 intensity.

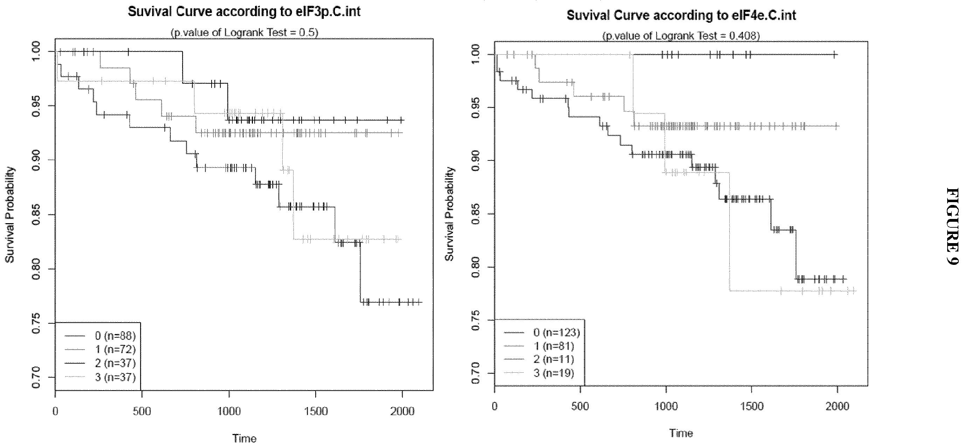

**Figure 9** shows a survival curve according to the eIF3p intensity. Survival curve according to the eIF4e intensity.

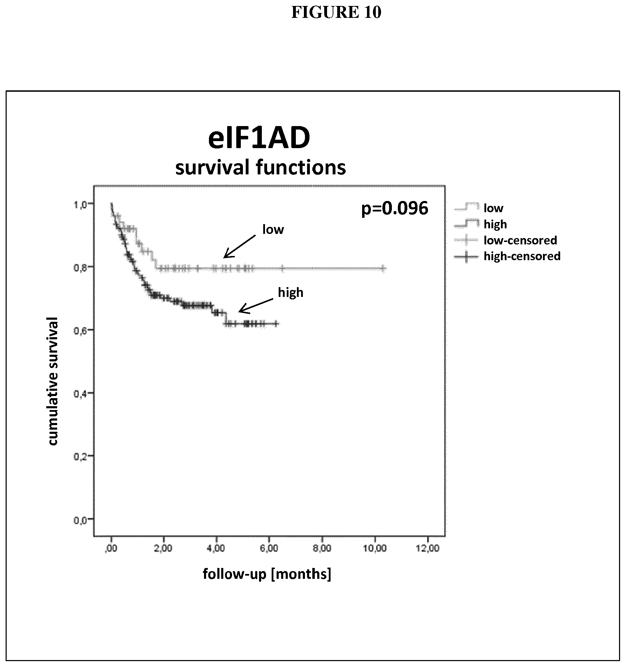

**Figure 10** shows the expression/survival correlation for the 1^{st} quartile cut off level between high and low expression of eIF1AD in DLCBL.

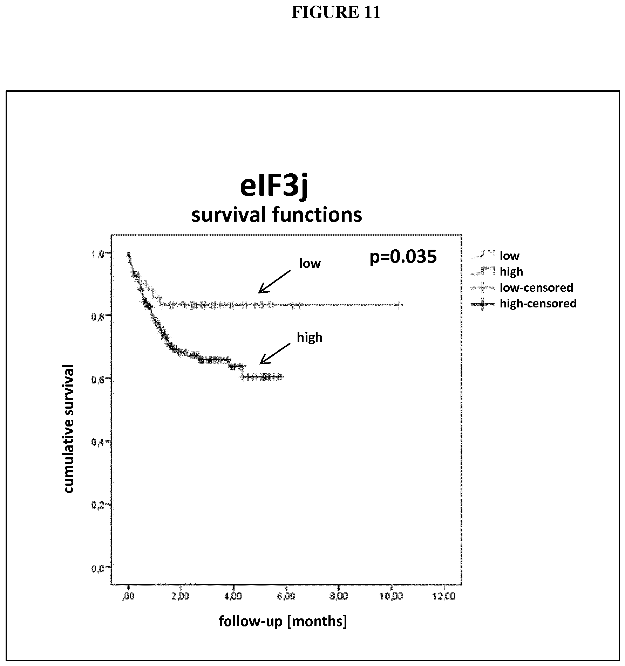

**Figure 11** shows the expression/survival correlation for the 1^{st} quartile cut off level between high and low expression of eIF3j in DLCBL.

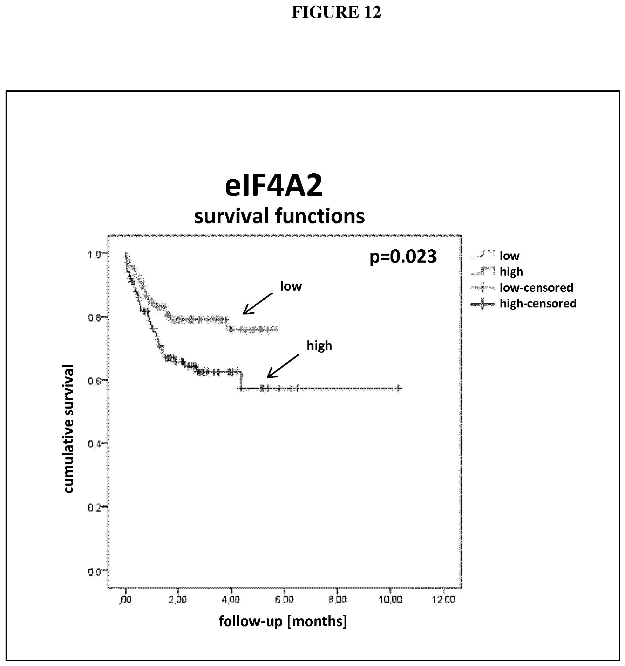

**Figure 12** shows the expression/survival correlation for the Median cut off level between high and low expression of eIF4A2 in DLBCL.

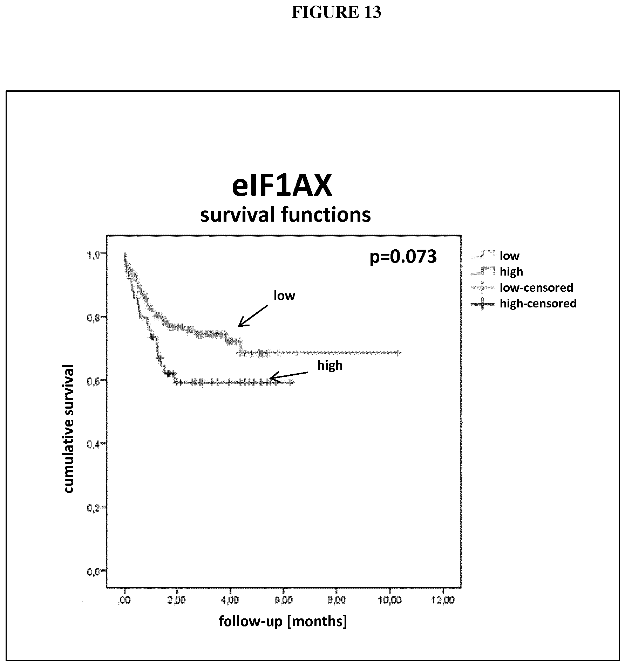

**Figure 13** shows the expression/survival correlation for the 3^{rd} quartile cut off level between high and low expression of eIF1AX in DLCBL.

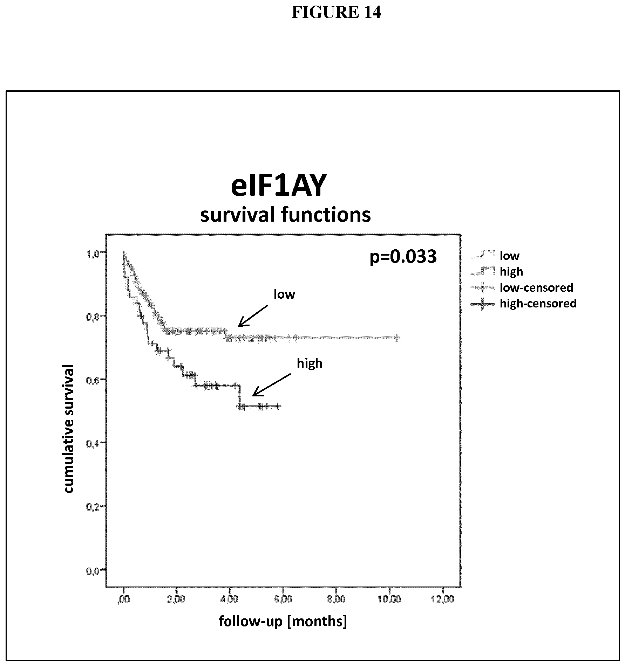

**Figure 14** shows the expression/survival correlation for the 3^{rd} quartile cut off level between high and low expression of eIF1AY in DLBCL.

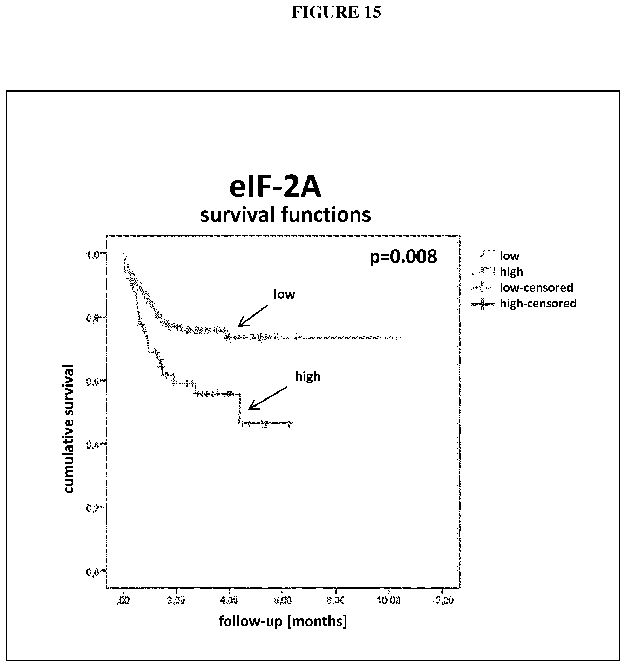

**Figure 15** shows the expression/survival correlation for the 3^{rd} quartile cut off level between high and low expression of eIF-2A in DLCBL.

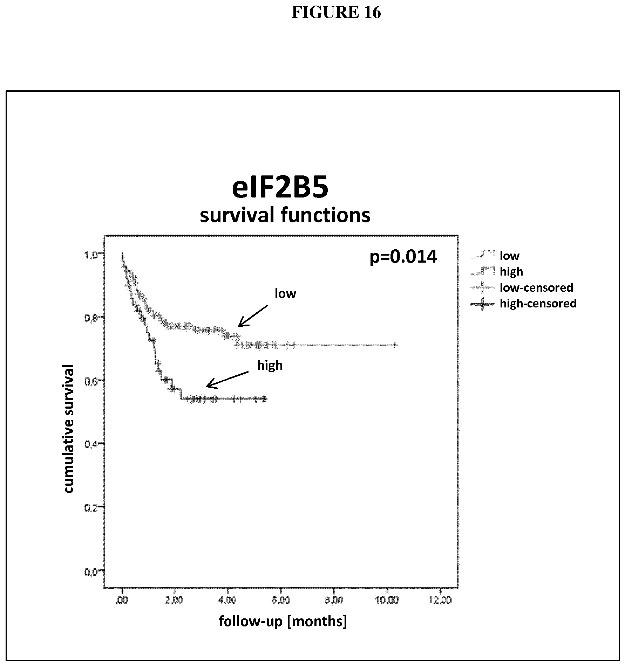

**Figure 16** shows the expression/survival correlation for the 3^{rd} quartile cut off level between high and low expression of eIF2B5 in DLCBL.

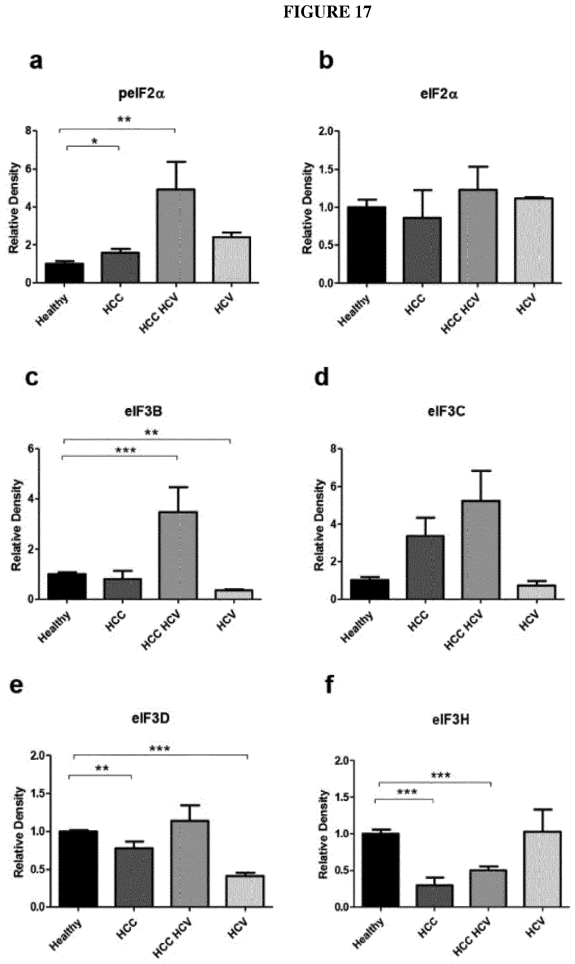

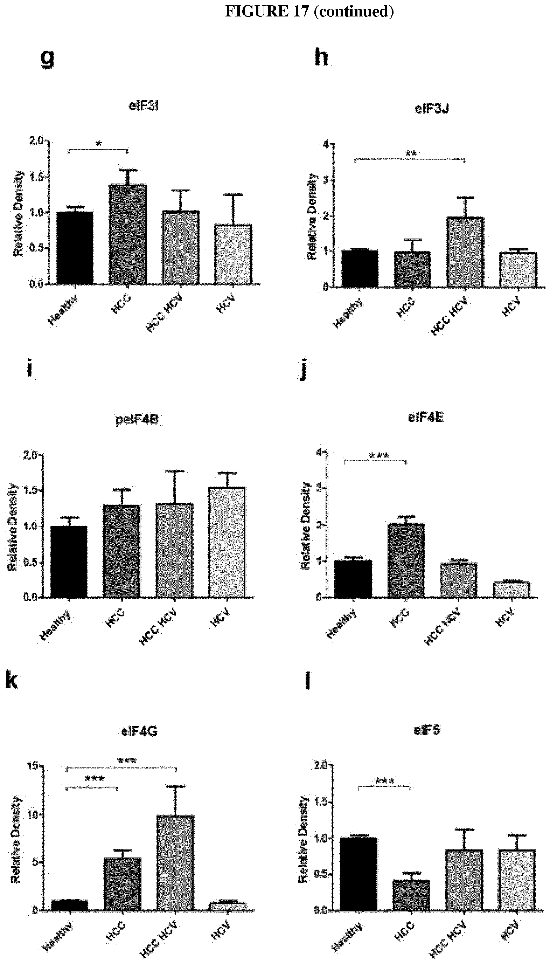

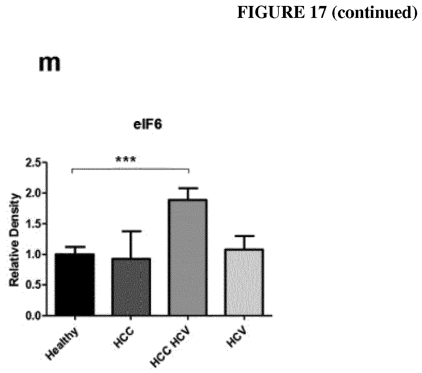

**Figure 17** shows elF protein expression in HCV - induced chronic hepatitis, HCV-associated HCC and HCC without viral infection. elF protein expression in HCV, HCV-associated HCC and HCC compared to healthy controls was analyzed using immunoblot analyses. Alterations in protein expression pattern of peIF2α (a), eIF2α (b), eIF3B (c), eIF3C (d), eIF3D (e), eIF3H (f), eIF3I (g), eIF3J (h), peIF4B (i), eIF4E (j), eIF4G (k), eIF5 (1) and eIF6 (m) are shown. Densitometry analyses of immunoblots were performed using ImageJ software (NIH, MD, United States). Relative densities were normalized to the loading control (GAPDH). Statistical analyses: 1-way ANOVA with Bonferroni post-test *p< 0.05; **p< 0.01 and ***p< 0.001.

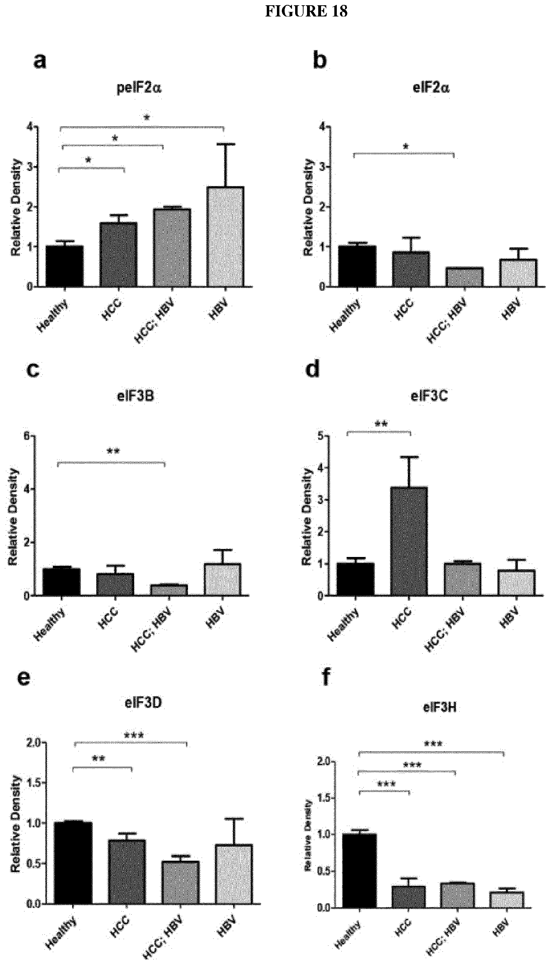

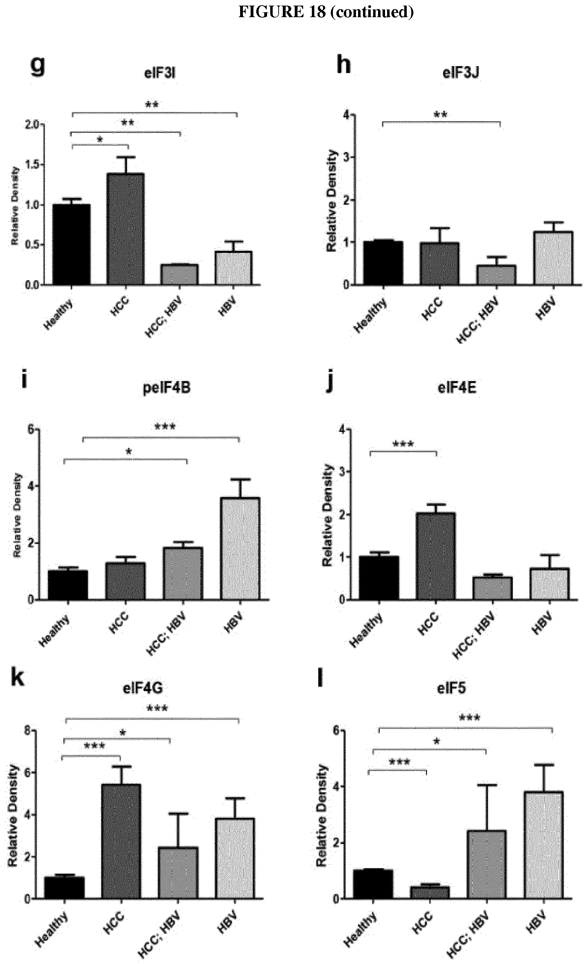

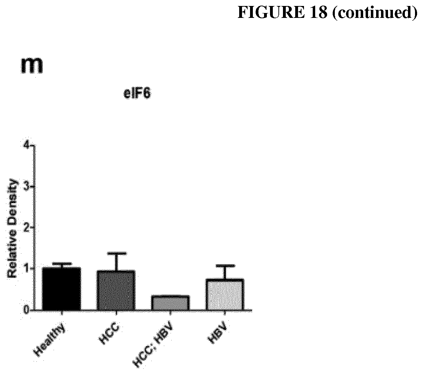

**Figure 18** shows elF protein expression in chronic hepatitis B, HBV-associated HCC and HCC without viral infection. eIF protein expression in HBV induced chronic hepatitis, HBV-associated HCC and HCC without viral infection compared to healthy controls was analyzed using immunoblot analyses. Alterations in protein expression pattern of peIF2α (a), eIF2α (b), eIF3B (c), eIF3C (d), eIF3D (e), eIF3H (f), eIF3I (g), eIF3J (h), peIF4B (i), eIF4E (j), eIF4G (k), eIF5 (1) and eIF6 (m) are shown. Densiometric analyses of immunoblots were performed using ImageJ software (NIH, MD, United States). Relative densities normalized to the loading control (GAPDH). Statistical analyses: 1-way ANOVA with Bonferroni post-test *p< 0.05; **p< 0.01 and ***p< 0.001.
**Figure 19** shows single elFs and sets of elFs which level is preferably determined in a method of diagnosing a hepatocellular carcinoma (HCC) in an individual. These sets comprise 2, 3, 4, 5, 6, 7, or 8 elFs.
**Figure 20** shows single elFs and sets of elFs which level is preferably determined in a method of diagnosing a hepatitis B virus (HBV) infection in an individual. These sets comprise 2, 3, 4, 5, or 6 elFs.
**Figure 21** shows single elFs and sets of elFs which level is preferably determined in a method of diagnosing a viral induced hepatocellular carcinoma (HCC) in an individual. These elFs are particularly preferred as they allow to determine whether the individual suffers from a hepatitis C virus induced hepatocellular carcinoma (HCC-HCV) or hepatitis B virus induced hepatocellular carcinoma (HCC-HBV). These sets comprise 2, 3, 4, or 5 elFs.

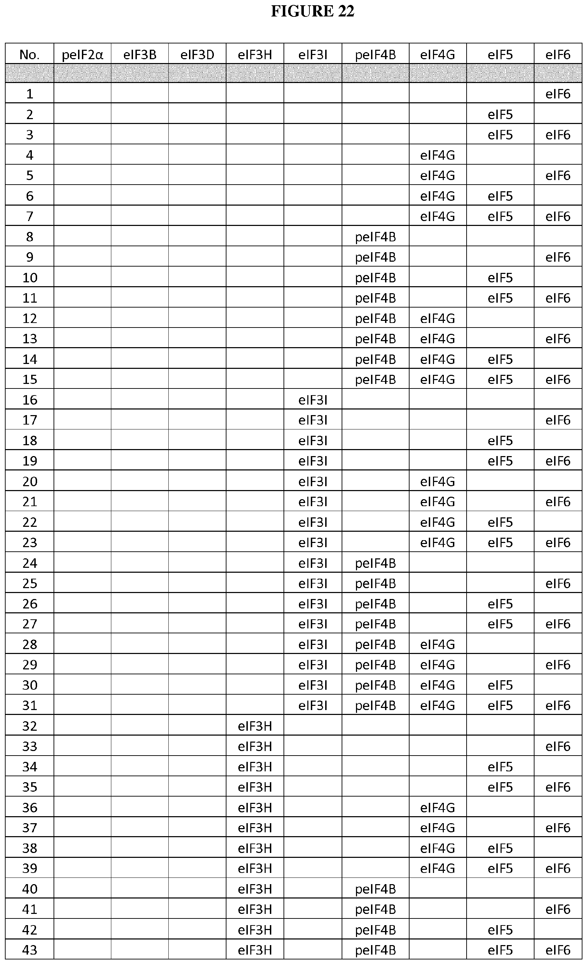

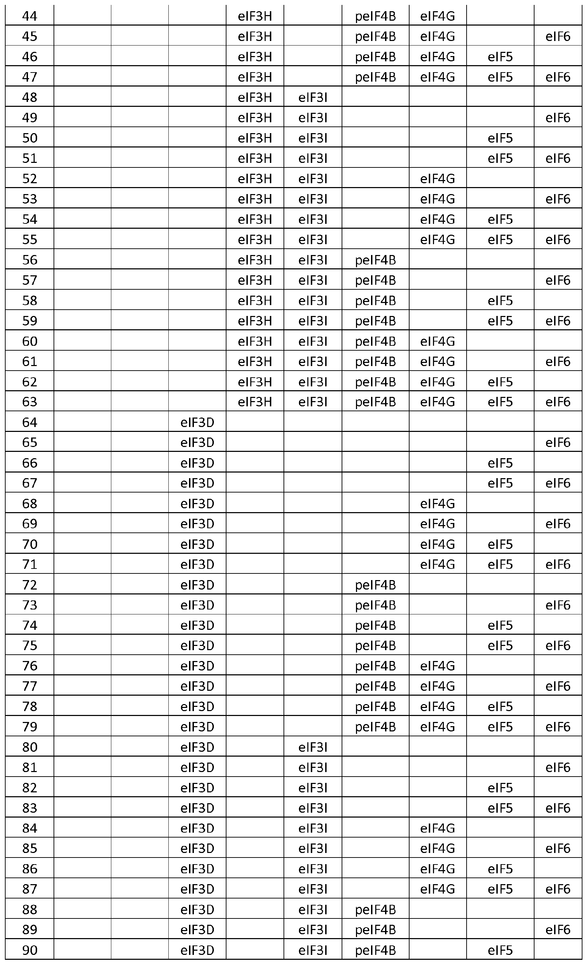

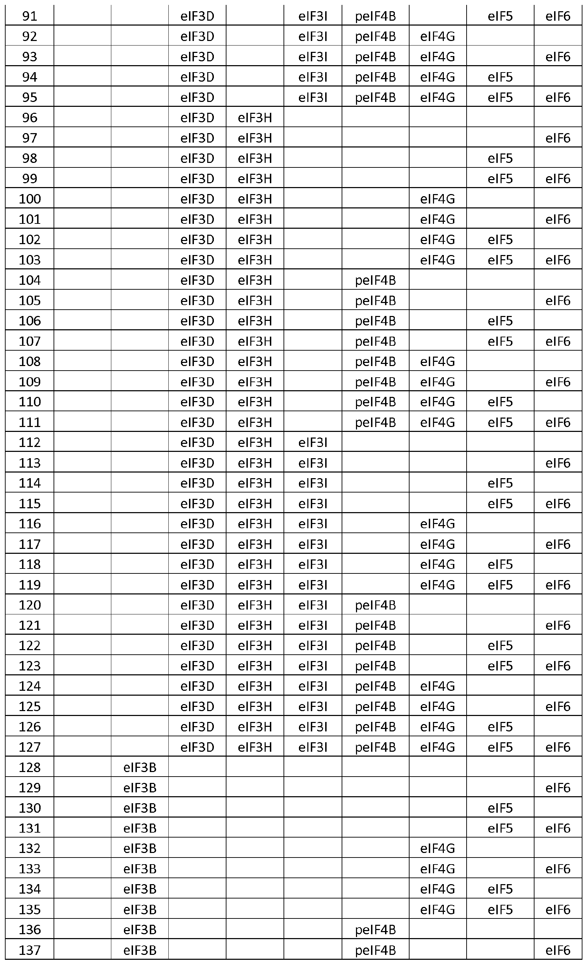

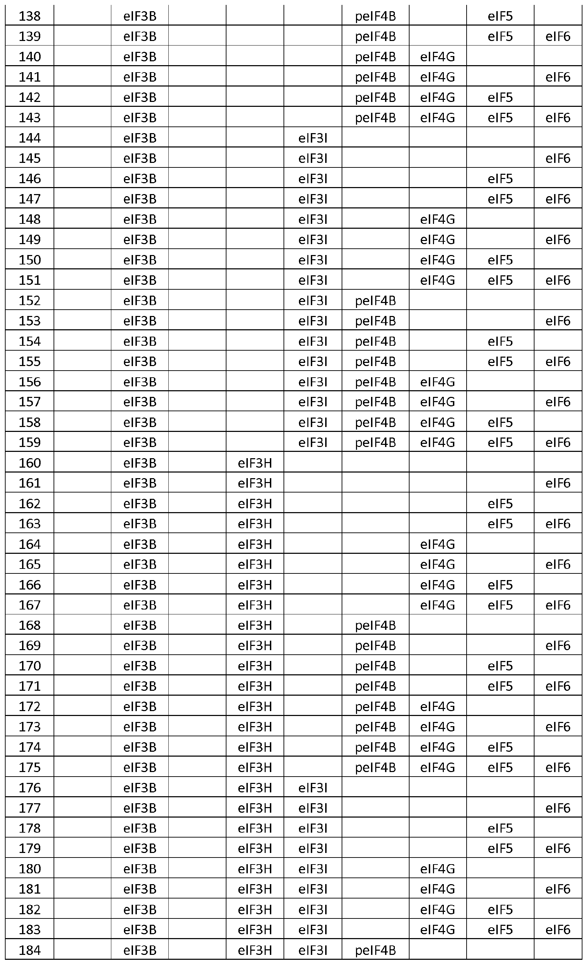

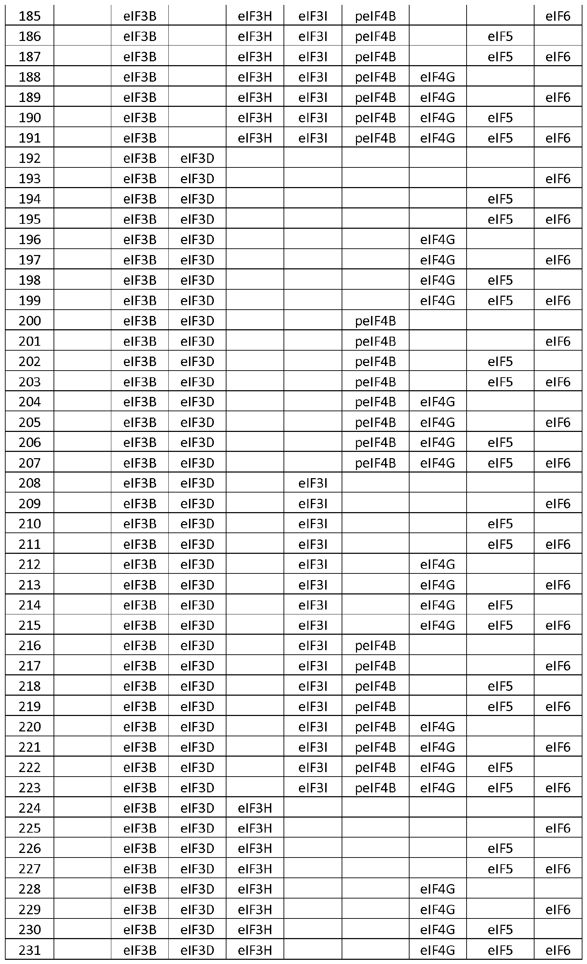

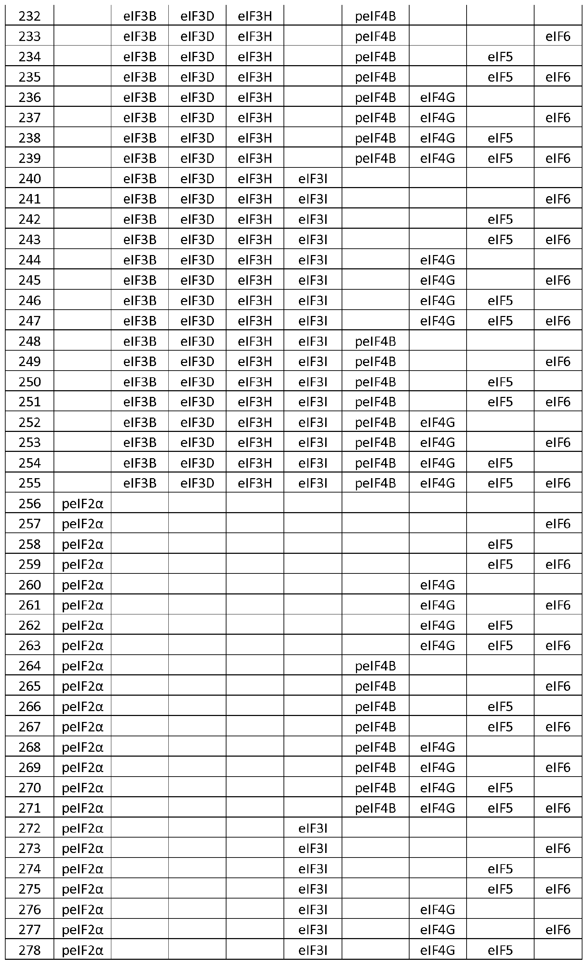

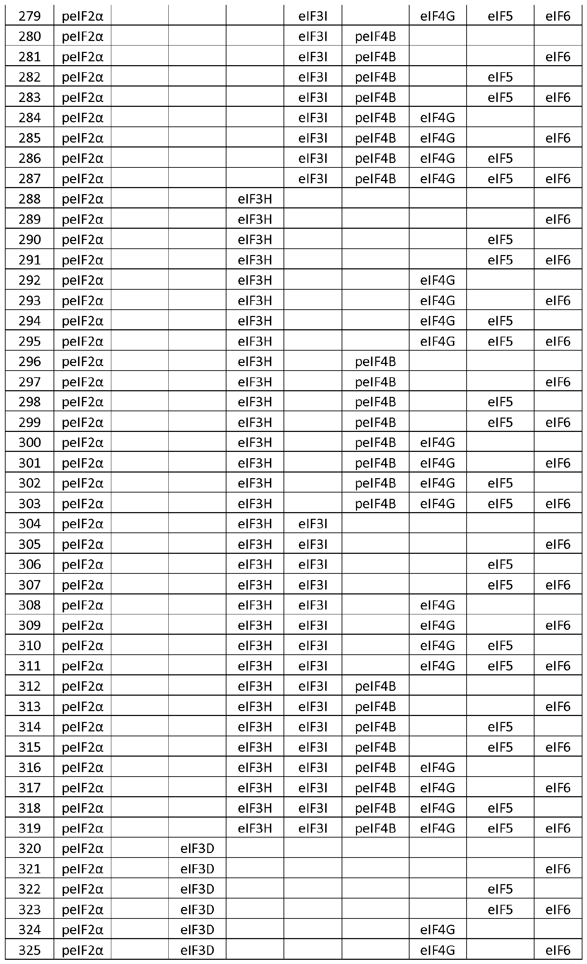

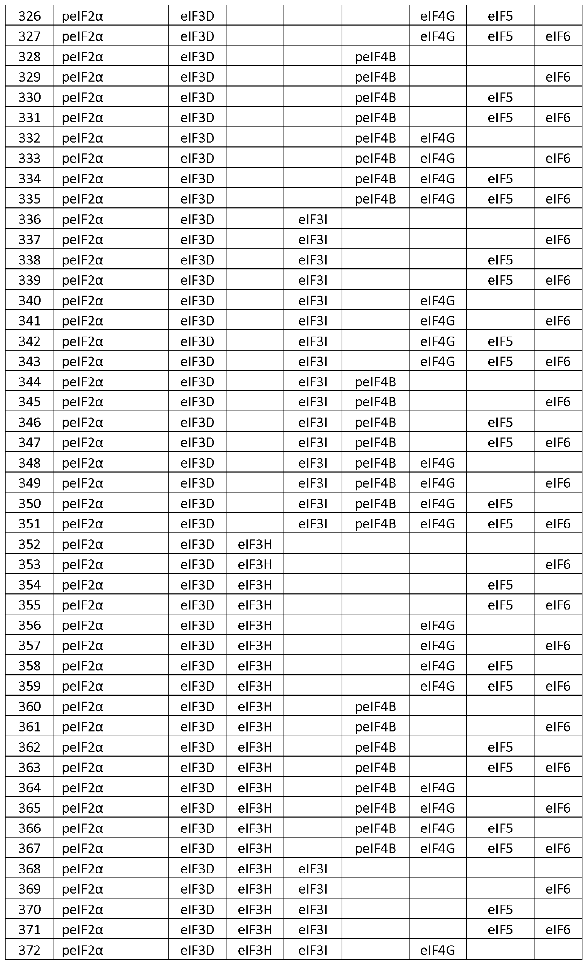

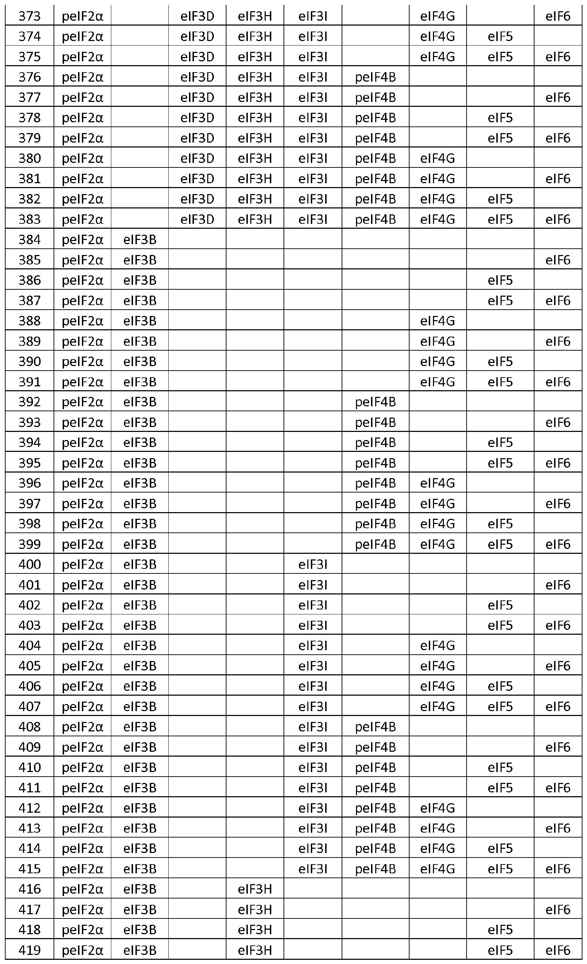

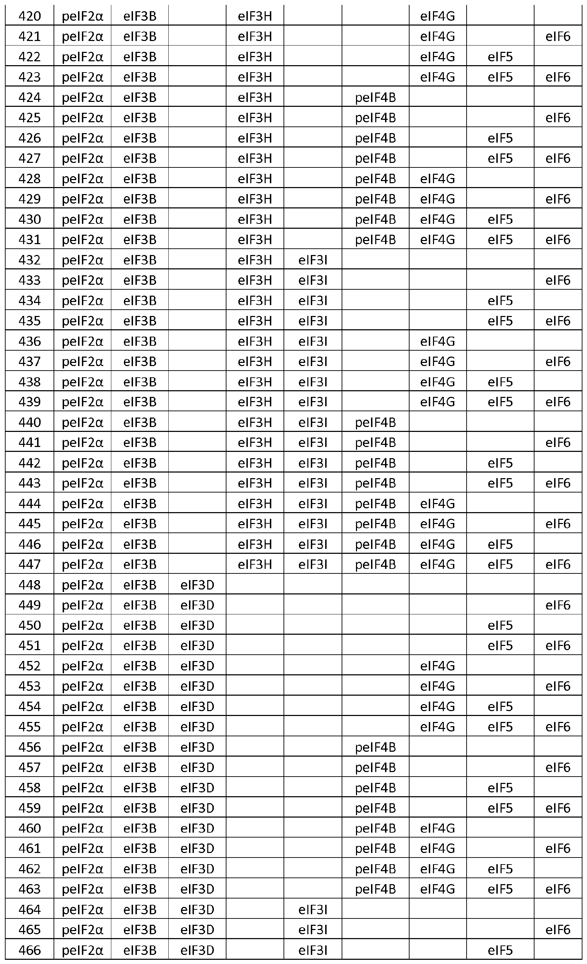

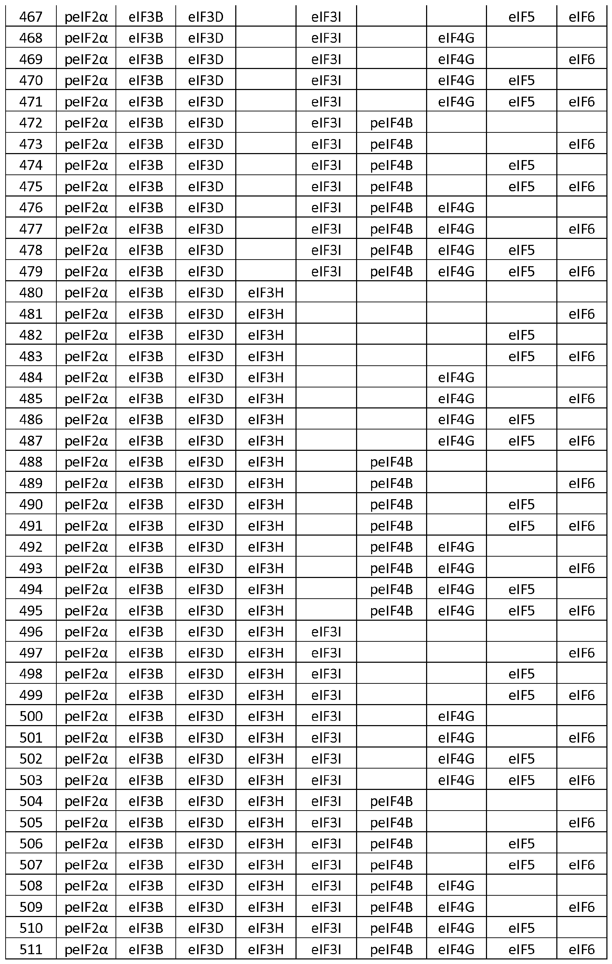

**Figure 22** shows single elFs and sets of elFs which level is preferably determined in a method of differentiating between at least two conditions in an individual, wherein the at least two conditions are selected from the group consisting of a hepatocellular carcinoma (HCC), hepatitis C virus (HCV) infection, hepatitis B virus (HBV) infection, and a viral induced hepatocellular carcinoma (HCC). The viral induced hepatocellular carcinoma is preferably a hepatitis C virus induced hepatocellular carcinoma (HCC-HCV) or hepatitis B virus induced hepatocellular carcinoma (HCC-HBV). These sets comprise 2, 3, 4, 5, 6, 7, 8, or 9 elFs.
**Figure 23** shows the expression/survival correlation for the Median cut off level between high and low expression of eIF1AX/Y in DLBCL (local patient cohort). To compare and depict differential survival outcome the log rank test and the Kaplan-Meier method were used.
**Figure 24** shows the expression/survival correlation for the Median cut off level between high and low expression of eIF2B5 in DLBCL (local patient cohort). To compare and depict differential survival outcome the log rank test and the Kaplan-Meier method were used.
**Figure 25** shows the expression/survival correlation for the 3^{rd} quartile cut off level between high and low expression of eIF1AX/Y in DLBCL (local patient cohort). To compare and depict differential survival outcome the log rank test and the Kaplan-Meier method were used.
**Figure 26** shows the expression/survival correlation for the 3^{rd} quartile cut off level between high and low expression of eIF2B5 in DLBCL (local patient cohort). To compare and depict differential survival outcome the log-rank test and the Kaplan-Meier method were used.

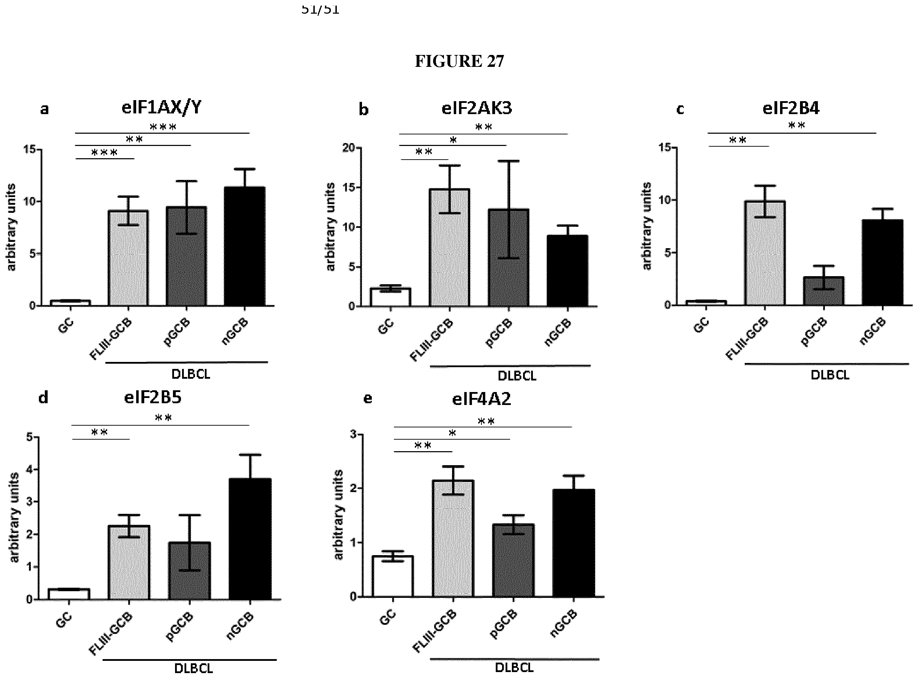

**Figure 27** shows the mRNA expression levels of eIF1AX/Y (a), eIF2AK3 (b), eIF2B4 (c), eIF2B5 (d) and eIF4A2 (e) in non-neoplastic germinal center B-cells (GC) and different subtypes of Diffuse Large B-cell Lymphoma (DLBCL): the germinal center B-cell subtype of DLBCL, separated into primary germinal center B-cell disease (pGCB) and secondary germinal center B-cell disease, that arose/going out from a Follicular Lymphoma grade III (FLIII-GCB), and the non-germinal center B-cell subtype of DLBCL (nGCB). Depicted are always absolute gene expression levels. Actin and GAPDH were used as housekeeping genes. For statistical analysis first the Shapiro-Wilk test was used to test for normality of distribution. According to the test result, a t-test or its nonparametric counterpart, the Mann-Whitney U Test, was used afterwards. Significance levels: *p<0.05, **p<0.01, ***p<0.001. Numbers: GC: n= 5, FLIII-GCB: n= 22, pGCB: n= 12, nGCB: n= 19.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölb1, H. eds. (1995), Helvetica Chimica Acta, CH-4010 Basel, Switzerland).

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of' according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "hepatocellular carcinoma (HCC)", as used herein, refers to the most common type of liver cancer. Most cases of hepatocellular carcinoma occur in individuals who already have symptoms of chronic liver disease and present either with worsening of symptoms or during surveillance that is used to screen individuals who are at risk the most. In other cases, HCC may directly present with yellow skin, bloating from fluid in the abdomen, easy bruising from blood clotting abnormalities, loss of appetite, unintentional weight loss, abdominal pain especially in the right upper quadrant, nausea, vomiting, or feeling tired.

The term "diagnosing a hepatocellular carcinoma (HCC)", as used herein, means determining whether an individual shows signs of or suffers from HCC.

The term "viral induced hepatocellular carcinoma (HCC)", as used herein, refers to a hepatocellular carcinoma triggered/induced by a virus, in particular a hepatitis C or hepatitis B virus. Thus, in a preferred embodiment, the viral induced hepatocellular carcinoma is a hepatitis C virus induced hepatocellular carcinoma (HCC-HCV) or hepatitis B virus induced hepatocellular carcinoma (HCC-HBV). It is known that a HCV or HBV infection is a risk factor of developing a HCC.

The term "hepatitis C virus (HCV) infection", as used herein, refers to a liver disease caused by the hepatitis C virus. The virus can cause both acute and chronic hepatitis infection, ranging in severity from a mild illness lasting a few weeks to a serious, lifelong illness. The hepatitis C virus is a blood borne virus and the most common modes of infection are through unsafe injection practices, inadequate sterilization of medical equipment, and the transfusion of unscreened blood and blood products.

The term "diagnosing a hepatitis C virus (HCV) infection", as used herein, means determining whether an individual shows signs of or suffers from a HCV infection.

The term "hepatitis B virus (HBV) infection", as used herein, refers to a liver disease caused by the hepatitis B virus. It can cause both acute and chronic disease. The virus is transmitted through contact with the blood or other body fluids of an infection individual.

The term "diagnosing a hepatitis B virus (HBV) infection", as used herein, means determining whether an individual shows signs of or suffers from a HBV infection.

The term "differentiating between at least two conditions in an individual, wherein the at least two conditions are selected from the group consisting of a hepatocellular carcinoma (HCC), hepatitis C virus (HCV) infection, hepatitis B virus (HBV) infection, and a viral induced hepatocellular carcinoma (HCC)", as used herein, means differential diagnosing between said at least two conditions. For example, said differential diagnosing allows to decide whether an individual suffers from (i) a HCC or HCV infection, (ii) a HCC or HBV infection, (iii) a HCC or viral induced HCC, (iv) a HCV infection or HBV infection, (v) a HCV infection or viral induced HCC, (vi) a HBV infection or viral induced HCC, (vii) a HCC, HCV infection or HBV infection, (viii) a HCC, HCV or viral induced HCC, (ix) a HCC, HBV infection or viral induced HCC, (x) a HCV infection, HBV infection or viral induced HCC, or (xi) a HCC, HCV infection, HBV infection or viral induced HCC. Preferably, the viral induced HCC is a hepatitis C virus induced hepatocellular carcinoma (HCC-HCV) or hepatitis B virus induced hepatocellular carcinoma (HCC-HBV).

The term "diagnosing a lymphoma", as used herein, means determining whether an individual shows signs of or suffers from a lymphoma. Preferably, the lymphoma is a Diffuse Large B-cell Lymphoma (DLBCL). More preferably, the DLBCL is a germinal center B-cell (GCB) subtype of DLBCL or a non-germinal center B-cell (nGCB) subtype of DLBCL. Even more preferably, the germinal center B-cell (GCB) subtype is a primary germinal center B-cell (pGCB) disease or a secondary germinal center B-cell (FLIII-GCB) disease. In particular, the secondary germinal center B-cell disease arises/goes out from a Follicular Lymphoma grade III.

The term "providing a prognosis to an individual suffering from a tumor", as used herein, particularly means determining whether the individual has a good prognosis or poor prognosis. The tumor may be a hepatocellular carcinoma (HCC) or a lymphoma. An individual suffering from a tumor may be considered to have a "good prognosis" where, for example, the survival rate associated with the tumor is greater compared to the survival rate of other individuals suffering from the same tumor or a related tumor subtype and showing another expression pattern of the prognostic marker of the present invention. In certain embodiments, a "good prognosis" indicates at least an increased expected survival time. A "good prognosis" indicates a greater than 1%, preferably greater than 10%, more preferably greater than 20%, more preferably greater than 30%, more preferably greater than 40%, more preferably greater than 50%, more preferably greater than 60%, more preferably greater than 70%, more preferably greater than 80%, more preferably greater than 90%, chance that the individual will survive to a specified time point (such as one, two, three, four, five, six or twelve months or even one, two or three years).

An individual suffering from a tumor may be considered to have a "poor prognosis" where, for example, the survival rate associated with the tumor is less compared to the survival rate of other individuals suffering from the same tumor or a related tumor subtype and showing another expression pattern of the prognostic marker of the present invention. A "poor prognosis" indicates a greater than 1 %, preferably greater than 10%, more preferably greater than 20%, more preferably greater than 30%, more preferably greater than 40%, more preferably greater than 50%, more preferably greater than 60%, more preferably greater than 70%, more preferably greater than 80%, more preferably greater than 90%, chance that the individual will not survive to a specified time point (such as one, two, three, four, five, six or twelve months or even one, two or three years). This may include also a greater than 50%, preferably greater than 60%, more preferably greater than 70%, more preferably greater than 80%, more preferably greater than 90% chance that the tumor will metastasize or migrate, if the tumor is a lymphoma.

The term "lymphoma", as used herein, refers to a group of blood cell tumors that develop from lymphocytes (a type of white blood cell). Signs and symptoms may include enlarged lymph nodes, fever, drenching sweats, unintended weight loss, itching, and constantly feeling tired. The two main categories of lymphomas are Hodgkin's lymphomas (HL) and the non-Hodgkin lymphomas (NHL). The World Health Organization (WHO) includes two other categories as types of lymphoma: multiple myeloma and immunoproliferative diseases. Multiple myeloma and immunoproliferative diseases are, however, preferably not encompassed by the term "lymphoma" as defined therein. In other words, the term "lymphoma", as used herein, preferably comprises Hodgkin's lymphoma (HL) and non-Hodgkin lymphoma (NHL). In one preferred embodiment, the lymphoma is a Diffuse Large B-cell Lymphoma (DLBCL). In a more preferred embodiment, the DLBCL is a germinal center B-cell (GCB) subtype of DLBCL or a non-germinal center B-cell (nGCB) subtype of DLBL. In an even more preferred embodiment, the germinal center B-cell (GCB) subtype is a primary germinal center B-cell (pGCB) disease or a secondary germinal center B-cell (FLIII-GCB) disease. In particular, the secondary germinal center B-cell disease arises/goes out from a Follicular Lymphoma grade III.

The term "individual", as used herein, refers to any subject for whom it is desired to know whether she or he suffers from a disease or condition (e.g. a HCC, HCV infection, HBV infection, viral induced HCC, or lymphoma). In particular, the term "individual", as used herein, refers to a subject suspected to be affected by the disease or condition (e.g. the HCC, HCV infection, HBV infection, viral induced HCC, or lymphoma). The individual may be diagnosed to be affected by the disease or condition (e.g. the HCC, HCV infection, HBV infection, viral induced HCC, or lymphoma), i.e. diseased, or may be diagnosed to be not affected by the disease or condition (e.g. the HCC, HCV infection, HBV infection, viral induced HCC, or lymphoma), i.e. healthy. The term "individual", as used herein, also refers to a subject which is affected by the disease or condition (e.g. the HCC, HCV infection, HBV infection, viral induced HCC, or lymphoma), i.e. diseased. The patient may be retested for the disease or condition (e.g. the HCC, HCV infection, HBV infection, viral induced HCC, or lymphoma) and may be diagnosed to be still affected by the disease or condition (e.g. the HCC, HCV infection, HBV infection, viral induced HCC, or lymphoma), i.e. diseased, or not affected by the disease or condition (e.g. the HCC, HCV infection, HBV infection, viral induced HCC, or lymphoma) anymore, i.e. healthy, for example after therapeutic intervention. The individual may also be retested for the disease or condition (e.g. the HCC, HCV infection, HBV infection, viral induced HCC, or lymphoma) and may be diagnosed as having developed an advanced form of the disease or condition (e.g. the HCC, HCV infection, HBV infection, viral induced HCC, or lymphoma).
It should be noted that an individual that is diagnosed as being healthy, i.e. not suffering from the disease or condition in question (e.g. the HCC, HCV infection, HBV infection, viral induced HCC, or lymphoma), may possibly suffer from another disease or condition not tested/known. The term "individual", as used herein, further refers to any subject suffering from a disease or condition, in particular a tumor (e.g. a HCC or lymphoma), for whom it is desired to know whether she or he has a good prognosis with respect to the disease or condition, in particular the tumor (e.g. the HCC or lymphoma), or poor prognosis with respect to the disease or condition, in particular the tumor (e.g. the HCC or lymphoma).
The individual may be any mammal, including both a human and another mammal, e.g. an animal such as a rabbit, mouse, rat, or monkey. Human individuals are particularly preferred.

The term "(control) patient", as used herein, refers to a subject known to be affected by a disease or condition (e.g. a HCC, HCV infection, HBV infection, viral induced HCC, or lymphoma), i.e. diseased. Said (control) patient may have developed an advanced form of the disease or condition (e.g. the HCC, HCV infection, HBV infection, viral induced HCC, or lymphoma). For example, the (control) patient is a (control) patient with a HCC, HCV infection, HBV infection, a viral induced HCC (e.g. a patient with a HCC-HCV or HCC-HBV), or lymphoma.
The "(control) patient", as used herein, also refers to a patient suffering from the same tumor (e.g. a HCC or lymphoma) as the individual to be tested, in particular in cases where a prognosis of the individual suffering from the tumor (e.g. a HCC or lymphoma) is determined.
The (control) patient may be any mammal, including both a human and another mammal, e.g. an animal such as a rabbit, mouse, rat, or monkey. Human (control) patients are particularly preferred.

The term "healthy (control) individual", as used herein, refers to a subject known to be not affected by the disease or condition (e.g. the HCC, HCV infection, HBV infection, viral induced HCC, or lymphoma) (negative control), i.e. healthy.
The healthy individual, as used herein, also refers to a subject known to be not affected by a tumor (e.g. a HCC or lymphoma).
It should be noted that an individual which is known to be healthy, i.e. not suffering from the disease or condition in question (e.g. a HCC, HCV infection, HBV infection, viral induced HCC, or lymphoma), may possibly suffer from another disease or condition not tested/known. In addition, an individual which is known to be healthy, i.e. not suffering from the tumor in question (e.g. a HCC or lymphoma), may possibly suffer from another tumor not tested/known. The healthy individual may be any mammal, including both a human and another mammal, e.g. an animal such as a rabbit, mouse, rat, or monkey. Human healthy individuals are particularly preferred.

The term "treatment", in particular "therapeutic treatment", as used herein, refers to any therapy which improves the health status and/or prolongs (increases) the lifespan of an individual suffering from a disease or condition, in particular a tumor. Said therapy may eliminate the disease or condition in an individual, arrest or slow the development of a disease in an individual, inhibit or slow the development of a disease in an individual, decrease the frequency or severity of symptoms in an individual, and/or decrease the recurrence in an individual who currently has or who previously has had a disease. The treatment of the diseases or conditions described herein includes, but is not limited to, administration of a drug, surgery, chemotherapy, and/or radiotherapy.

The term "level", as used herein, refers to an amount (measured for example in grams, mole, or ion counts) or concentration (e.g. absolute or relative concentration) of the at least one elF claimed herein.

The term "level", as used herein, also comprises scaled, normalized, or scaled and normalized amounts or values. The level may also be a cut-off level.
In a preferred embodiment, the level is an expression level.

The term "eukaryotic Initiation Factor (eIF)", as used herein, refers to molecules which are involved in the initiation phase of eukaryotic translation. These factors help to stabilize the formation of the functional ribosome around the start codon and also provide regulatory mechanisms in translation initiation. The term "eukaryotic Initiation Factor (eIF)", as used herein, covers elF RNA transcripts (RNA transcript variants) such as mRNAs including splice variants of these transcripts and elF proteins encoded thereby. Thus, the level of the elFs may be determined by measuring mRNA or protein levels. The term "eukaryotic Initiation Factor (eIF)", as used herein, also covers elF isoforms. These elF isoforms are members of a set of highly similar molecules, in particular proteins, that perform the same or similar biological role. eIF4E, for example, comprises the isoforms eIF4E1, eIF4E2, and/or eIF4E3, encoded by the respective genes. In addition, eIF4G, for example, comprises the isoforms eIF4G1, eIF4G2, and/or eIF4G3, encoded by the respective genes. The level of elF isoforms may also be determined by measuring mRNA or protein levels.

The term "sample", as used herein, refers to any sample from an individual or (control) patient containing at least one of the elFs claimed herein. Preferably, the sample is a biological sample. The biological sample may be a body fluid sample. For example, biological samples encompassed by the present invention are blood (e.g. whole blood or blood fraction such as blood cell fraction, serum or plasma) samples, lymph samples, saliva samples, urine samples, or samples from other peripheral sources. Said biological samples may be mixed or pooled, e.g. a sample may be a mixture of a blood sample and a urine sample. Said biological samples may be provided by removing a body fluid from an individual or control (patient), but may also be provided by using a previously isolated sample. For example, a blood sample may be taken from an individual or (control) patient by conventional blood collection techniques. The biological sample, e.g. urine sample or blood sample, may be obtained from an individual or (control) patient prior to the initiation of a therapeutic treatment, during the therapeutic treatment, and/or after the therapeutic treatment. If the sample, in particular the biological sample, is obtained from at least one (control) patient or healthy (control) individual, e.g. from at least 1, 2, 3, 4, 5, 6, 7, 8, 9, 10, 20, 30, 40, 50, 60, 70, 80, 90, 100, 150, 200, 250, 300, 400, 500, or 1.000 (control) patient(s) or healthy (control) individual(s), it is designated as a "reference sample", in particular as a "reference biological sample". Preferably, the reference (biological) sample is from the same source than the (biological) sample of the individual to be tested, e.g. both are blood samples or urine samples. It is further preferred that both are from the same species, e.g. from a human. It is also (alternatively or additionally) preferred that the measurements of the reference (biological) sample and the (biological) sample of the individual to be tested are identical, e.g. both have an identical volume. It is particularly preferred that the reference (biological) sample and the (biological) sample are from individuals/(control) patients of the same sex and similar age, e.g. no more than 2 years apart from each other.

The term "body fluid sample", as used herein, refers to any liquid sample derived from the body of an individual or (control) patient containing at least one of the elFs claimed herein. Said body fluid sample may be a urine sample, blood sample, sputum sample, breast milk sample, cerebrospinal fluid (CSF) sample, cerumen (earwax) sample, gastric juice sample, mucus sample, lymph sample, endolymph fluid sample, perilymph fluid sample, peritoneal fluid sample, pleural fluid sample, saliva sample, sebum (skin oil) sample, semen sample, sweat sample, tears sample, cheek swab, vaginal secretion sample, liquid biopsy, or vomit sample including components or fractions thereof. The term "body fluid sample" also encompasses body fluid fractions, e.g. blood fractions, urine fractions or sputum fractions. The body fluid samples may be mixed or pooled. Thus, a body fluid sample may be a mixture of a blood and a urine sample or a mixture of a blood and cerebrospinal fluid sample. Said body fluid sample may be provided by removing a body liquid from an individual or (control) patient, but may also be provided by using previously isolated body fluid sample material. The body fluid sample allows for a noninvasive analysis of an individual. It is further preferred that the body fluid sample has a volume of between 0.01 and 20 ml, more preferably of between 0.1 and 10 ml, even more preferably of between 0.5 and 8 ml, and most preferably of between 1 and 5 ml. If the body fluid sample is obtained from at least one (control) patient or healthy (control) individual, e.g. from at least 1, 2, 3, 4, 5, 6, 7, 8, 9, 10, 20, 30, 40, 50, 60, 70, 80, 90, 100, 150, 200, 250, 300, 400, 500, or 1.000 (control) patients (s) or healthy (control) individual(s), it is designated as a "reference body fluid sample".

The term "blood sample", as used herein, encompasses a whole blood sample or a blood fraction sample such as a blood cell fraction, blood serum, or blood plasma sample. It is preferred that the blood serum or plasma sample has a volume of between 0.01 and 20 ml, more preferably of between 0.1 and 10 ml, even more preferably of between 0.5 and 8 ml and most preferably of between 1 and 5 ml.

In the context of the present invention, the term "kit of parts (in short: kit)" is understood to be any combination of at least some of the components identified herein, which are combined, coexisting spatially, to a functional unit, and which can contain further components. Said kit may allow point-of-care testing (POCT).

The term "point-of-care testing (POCT)", as used herein, refers to a medical diagnostic testing at or near the point of care that is the time and place of individual care. This contrasts with the historical pattern in which testing was wholly or mostly confined to the medical laboratory, which entailed sending off specimens away from the point of care and then waiting hours or days to learn the results, during which time care must continue without the desired information. Point-of-care tests are simple medical tests that can be performed at the bedside. The driving notion behind POCT is to bring the test conveniently and immediately to the individual to be tested. This increases the likelihood that the individual, physician, and care team will receive the results quicker, which allows for immediate clinical management decisions to be made. POCT is often accomplished through the use of transportable, portable, and handheld instruments and test kits. Small bench analyzers or fixed equipment can also be used when a handheld device is not available - the goal is to collect the specimen and obtain the results in a very short period of time at or near the location of the individual so that the treatment plan can be adjusted as necessary before the individual leaves the hospital.

### Embodiments of the invention

The present inventors performed comprehensive studies in order to analyze the relationship between the whole range of elFs and patient outcome and the potential of the whole range of elFs as markers to diagnose tumors or differential diagnose between specific tumor entities. They identified elFs which performed best in the prognosis of an individual suffering from a tumor, in the diagnosis of a tumor, or differential diagnosis between specific tumor identities. The identified elFs allow a reliable prognosis with respect to the life expectancy of an individual suffering from a tumor, tumor diagnosis in an individual or differential diagnosis between specific tumor entities in an individual.

Thus, in a first aspect, the present invention relates to a method of providing a prognosis to an individual suffering from a tumor comprising the steps of:
a) determining the level of eIF2AK4 (Gene: EIF2AK4, GCN2; Gene ID (GenBank): 440275) in a sample from an individual, and
b) comparing the level of eIF2AK4 in said sample to a reference level A of eIF2AK4 determined in samples of patients suffering from the same tumor or to a reference level B of eIF2AK4 determined in samples of healthy individuals, wherein a decreased level of eIF2AK4 in the sample of said individual compared to the reference level A and/or B indicates a good prognosis,
wherein the tumor is a lymphoma or a hepatocellular carcinoma.

Also described herein, but not encompassed by the present invention, is a method of providing a prognosis to an individual suffering from a tumor comprising the steps of:
a) obtaining a sample from said individual,
b) determining the level of at least one eukaryotic Initiation Factor (elF) selected from the group consisting of eIF2AK4 (Gene: EIF2AK4, GCN2; Gene ID (GenBank): 440275), eIF2B4/eIF-2B subunit delta (Gene: EIF2B4; Gene ID (GenBank): 8890), eIF2C 3 (Gene: AGO3/EIF2C3; Gene ID (GenBank): 192669), eIF2d (Gene: EIF2D; Gene ID (GenBank): 1939), eIF-2A/alpha/α/eIF2S1 (Gene: EIF2S1; Gene ID (GenBank): 1965), eIF-2-beta/eIF2S2 (Gene: EIF2S2; Gene ID (GenBank): 8894), eIF3b (Gene: EIF3B; Gene ID (GenBank): 8662), eIF3c (Gene: EIF3C; Gene ID (GenBank): 8663), eIF3d (Gene: EIF3D; Gene ID (GenBank): 8664), eIF3f (Gene: EIF3F; Gene ID (GenBank): 8665), eIF3g (Gene: EIF3G; Gene ID (GenBank): 8666), eIF31 (Gene: EIF3L; Gene ID (GenBank): 51386), eIF-4B (Gene: EIF4B; Gene ID (GenBank): 1975), 4E-BP1 (Gene: EIF4EBP1; Gene ID (GenBank): 1978), eIF-4G1 (Gene:EIF4G1; Gene ID (GenBank): 1981), eIF-5A (Gene: EIF5A; Gene ID (GenBank): 1984), eIF2AK3/HsPEK (Gene: EIF2AK3; Gene ID (GenBank): 9451), eIF-4E (eIF-4E3; Gene: EIF4E3; Gene ID (GenBank): 317649), eIF-5 (Gene: EIF5; Gene ID (GenBank): 1983), eIF1AD (Gene: EIF1AD, Gene ID (GenBank): 84285), eIF1AX/eIF-1A X isoform (Gene: EIF1AX, Gene ID (GenBank): 1964), eIF1AY/eIF-1A Y isoform (Gene: EIF1AY, Gene ID: (GenBank) 9086), eIF-2A (Gene: EIF2A, Gene ID (GenBank): 83939), eIF2B5 (Gene: EIF2B5, Gene ID (GenBank): 8893), eIF3j (Gene: EIF3J, Gene ID (GenBank): 8669), and eIF4A2/eIF4A-II (Gene: EIF4A2, Gene ID (GenBank): 1974) in the sample of step a), and
c) comparing the level of the at least one elF in said sample to a reference level A of the same elF determined in samples of one or more patients suffering from the same tumor or to a reference level B of the same elF determined in samples of one or more healthy individuals, wherein
   an increased level of eIF2AK3/HsPEK, eIF-4E3 and/or eIF-5 in the sample of said individual compared to the reference level A,
   an increased level of eIF-2α and/or eIF-5 in the sample of said individual compared to the reference level B,
   a decreased level of eIF-2α, eIF1AD, eIF1AX/eIF-1A X isoform, eIF1AY/eIF-1A Y isoform, eIF-2A, eIF2B5, eIF3j, and/or eIF4A2/eIF4A-II in the sample of said individual compared to the reference level A and
   a decreased level of eIF2AK4, eIF2B4, eIF2C 3, eIF2d, eIF2S2, eIF3b, eIF3c, eIF3d, eIF3f, eIF3g, eIF31, eIF-4B, 4E-BP1, eIF-4G1 and/or eIF-5A in the sample of said individual compared to the reference level A and/or B
   indicates a good prognosis.

It turned out that eIF2AK4, eIF2B4, eIF2C 3, eIF2d, eIF-2α, eIF2S2, eIF3b, eIF3c, eIF3d, eIF3f, eIF3g, eIF31, eIF-4B, 4E-BP1, eIF-4G1, eIF-5A, eIF2AK3/HsPEK, eIF-4E3, eIF-5, eIF1AD, eIF1AX/eIF-1A X isoform, eIF1AY/eIF-1A Y isoform, eIF-2A, eIF2B5, eIF3j, and/or eIF4A2/eIF4A-II can be used to provide a prognosis for an individual suffering from a tumor. The differential expression of these elFs in individuals suffering from a tumor allows providing a reliable survival prognosis.

The above method can alternatively be worded as follows: A method of providing a prognosis to an individual suffering from a tumor comprising the steps of:
a) determining the level of at least one eukaryotic Initiation Factor (elF) selected from the group consisting of eIF2AK4 (Gene: EIF2AK4, GCN2; Gene ID (GenBank): 440275), eIF2B4/eIF-2B subunit delta (Gene:EIF2B4; Gene ID (GenBank): 8890), eIF2C 3 (Gene: AGO3/EIF2C3; Gene ID (GenBank): 192669), eIF2d (Gene: EIF2D; Gene ID (GenBank): 1939), eIF-2A/alpha/α/eIF2S1 (Gene: EIF2S1; Gene ID (GenBank): 1965), eIF-2-beta/eIF2S2 (Gene: EIF2S2; Gene ID (GenBank): 8894), eIF3b (Gene: EIF3B; Gene ID (GenBank): 8662), eIF3c (Gene: EIF3C; Gene ID (GenBank): 8663), eIF3d (Gene: EIF3D; Gene ID (GenBank): 8664), eIF3f (Gene: EIF3F; Gene ID (GenBank): 8665), eIF3g (Gene: EIF3G; Gene ID (GenBank): 8666), eIF31 (Gene: EIF3L; Gene ID (GenBank): 51386), eIF-4B (Gene: EIF4B; Gene ID (GenBank): 1975), 4E-BP1 (Gene: EIF4EBP1; Gene ID (GenBank): 1978), eIF-4G1 (Gene:EIF4G1; Gene ID (GenBank): 1981), eIF-5A (Gene: EIF5A; Gene ID (GenBank): 1984), eIF2AK3/HsPEK (Gene: EIF2AK3; Gene ID (GenBank): 9451), eIF-4E (eIF-4E3; Gene: EIF4E3; Gene ID (GenBank): 317649), eIF-5 (Gene: EIF5; Gene ID (GenBank): 1983), eIF1AD (Gene: EIF1AD, Gene ID (GenBank): 84285), eIF1AX/eIF-1A X isoform (Gene: EIF1AX, Gene ID (GenBank): 1964), eIF1AY/eIF-1A Y isoform (Gene: EIF1AY, Gene ID: (GenBank) 9086), eIF-2A (Gene: EIF2A, Gene ID (GenBank): 83939), eIF2B5 (Gene: EIF2B5, Gene ID (GenBank): 8893), eIF3j (Gene: EIF3J, Gene ID (GenBank): 8669), and eIF4A2/eIF4A-II (Gene: EIF4A2, Gene ID (GenBank): 1974) in a sample from an individual, and
b) comparing the level of the at least one elF in said sample to a reference level A of the same elF determined in samples of patients suffering from the same tumor or to a reference level B of the same elF determined in samples of healthy individuals, wherein
   an increased level of eIF2AK3/HsPEK, eIF-4E3 and/or eIF-5 in the sample of said individual compared to the reference level A,
   an increased level of eIF-2α and/or eIF-5 in the sample of said individual compared to the reference level B,
   a decreased level of eIF-2α, eIF1AD, eIF1AX/eIF-1A X isoform, eIF1AY/eIF-1A Y isoform, eIF-2A, eIF2B5, eIF3j, and/or eIF4A2/eIF4A-II in the sample of said individual compared to the reference level A, and/or
   a decreased level of eIF2AK4, eIF2B4, eIF2C 3, eIF2d, eIF2S2, eIF3b, eIF3c, eIF3d, eIF3f, eIF3g, eIF31, eIF-4B, 4E-BP1, eIF-4G1, and/or eIF-5A in the sample of said individual compared to the reference level A and/or B
   indicates a good prognosis.

The expression rate/level of eIF2AK4, eIF2B4, eIF2C_3, eIF2d, eIF-2α, eIF2S2, eIF3b, eIF3c, eIF3d, eIF3f, eIF3g, eIF31, eIF-4B, 4E-BP1, eIF-4G1, eIF-5A, eIF2AK3/HsPEK, eIF-4E3, eIF-5, eIF1AD, eIF1AX/eIF-1A X isoform, eIF1AY/eIF-1A Y isoform, eIF-2A, eIF2B5, eIF3j, and/or eIF4A2/eIF4A-II in individuals suffering from a tumor is an indication of the survival rate of these individuals and therefore these markers can be used to prognose the outcome of a disease associated with a tumor.

An increased expression of eIF2AK3/HsPEK, eIF-4E3 and/or eIF-5 in an individual suffering from a tumor compared to the reference level determined in samples of one or more patients suffering from the same tumor indicates a good prognosis. This means that such patients will live longer compared to other patients where eIF2AK3/HsPEK, eIF-4E3 and/or eIF-5 show an average expression rate or even an expression rate below average.

An increased level of eIF-2α and/or eIF-5 in an individual suffering from a tumor compared to a reference level determined in samples of healthy individuals indicates also a good prognosis. Therefore, patients suffering from a tumor and expressing these two eIFs in a higher level than healthy individuals will live longer than patients showing substantially identical or even lower expression rates of eIF-2α and/or eIF-5.

Decreased levels of eIF2AK4, eIF2B4, eIF2C 3, eIF2d, eIF2S2, eIF3b, eIF3c, eIF3d, eIF3f, eIF3g, eIF31, eIF-4B, 4E-BP1, eIF-4G1, eIF-5A, eIF1AD, eIF1AX/eIF-1A X isoform, eIF1AY/eIF-1A Y isoform, eIF-2A, eIF2B5, eIF3j, and/or eIF4A2/eIF4A-II in samples of individuals suffering from a tumor compared to reference levels determined in samples of other patients suffering from the same tumor or determined in samples of healthy individuals indicate a good prognosis.

It is described herein that the level of at least one elF selected from the group consisting of eIF2AK4, eIF2B4, eIF2C 3, eIF2d, eIF-2α, eIF2S2, eIF3b, eIF3c, eIF3d, eIF3f, eIF3g, eIF31, eIF-4B, 4E-BP1, eIF-4G1, eIF-5A, eIF2AK3/HsPEK, eIF-4E3, eIF-5, eIF1AD, eIF1AX/eIF-1A X isoform, eIF1AY/eIF-1A Y isoform, eIF-2A, eIF2B5, eIF3j, and/or eIF4A2/eIF4A-II is determined. In other embodiments, the levels of at least two, preferably at least three, more preferably at least four, more preferably at least five, more preferably at least six, more preferably at least seven, more preferably at least eight, more preferably at least nine, more preferably at least ten, more preferably at least 11, more preferably at least 12, more preferably at least 13, more preferably at least 14, more preferably at least 15, more preferably at least 16, more preferably at least 17, more preferably at least 18, more preferably at least 19, more preferably at least 20, more preferably at least 21, more preferably at least 22, more preferably at least 23, more preferably at least 24, more preferably at least 25, in particular of all, of the elFs mentioned above are determined.

In order to provide a reliable prognosis, the levels of elFs determined in samples of individuals suffering from a tumor have to be compared with reference levels. These reference levels are preferably determined in patients suffering from the same tumor to give an average level of the respective elF and/or in healthy individuals. "Healthy individuals" to determine the average level of the elFs did and do not suffer from a tumor. The number of patients and individuals used to determine the respective reference levels A and B amounts to at least two, preferably at least five, more preferably at least ten, more preferably at least 20, more preferably at least 50.

According to a preferred embodiment, the tumor is a lymphoma or a hepatocellular carcinoma (HCC).

It turned out that eIF2AK4 used in the method according to the first aspect of the present invention can be used in the prognosis of individuals suffering from a lymphoma or a hepatocellular carcinoma (HCC).

According to another preferred embodiment of the above-described method, an increased level of at least one elF selected from the group consisting of eIF5 and eIF2α in a sample of an individual suffering from hepatocellular carcinoma compared to the reference level B indicates a good prognosis.

If the levels of eIF5 and/or eIF2α in a sample of an individual suffering from hepatocellular carcinoma are increased compared to the reference level B, the prognosis can be considered as good. This means that this individual has a higher survival rate compared to individuals having a marker level substantially identical, identical or below reference level B.

According to a further preferred embodiment of the above-described method, an increased level of at least one elF selected from the group consisting of eIF2AK3/HsPEK, eIF-4E3 and eIF-5 in a sample of an individual suffering from lymphoma compared to the reference level A indicates a good prognosis.

If the levels of eIF2AK3/HsPEK, eIF-4E3 and/or eIF-5 in a sample of an individual suffering from lymphoma are increased compared to the reference level A, the prognosis can be considered as good. This means that this individual has a higher survival rate compared to individuals having a marker level substantially identical, identical or below reference level A.

According to a preferred embodiment of the above-described method, a decreased level of at least one elF selected from the group consisting of eIF2AK4, eIF2B4, eIF2C 3, eIF2d, eIF-2α, eIF2S2, eIF3b, eIF3c, eIF3d, eIF3f, eIF3g, eIF31, eIF-4B, 4E-BP1, eIF-4G1, eIF-5A, eIF1AD, eIF1AX/eIF-1A X isoform, eIF1AY/eIF-1A Y isoform, eIF-2A, eIF2B5, eIF3j, and eIF4A2/eIF4A-II in a sample of an individual suffering from lymphoma compared to the reference level A indicates a good prognosis.

If the levels of eIF2AK4, eIF2B4, eIF2C 3, eIF2d, eIF-2α, eIF2S2, eIF3b, eIF3c, eIF3d, eIF3f, eIF3g, eIF31, eIF-4B, 4E-BP1, eIF-4G1, eIF-5A, eIF1AD, eIF1AX/eIF-1A X isoform, eIF1AY/eIF-1A Y isoform, eIF-2A, eIF2B5, eIF3j, and/or eIF4A2/eIF4A-II in a sample of an individual suffering from lymphoma are decreased compared to the reference level A, the prognosis can be considered as good. This means that this individual has a higher survival rate compared to individuals having a marker level substantially identical, identical or above reference level A.

Preferably, the level of at least one elF selected from the group consisting of eIF1AD, eIF1AX/eIF-1A X isoform, eIF1AY/eIF-1A Y isoform, eIF-2A, eIF2B5, eIF3j, eIF4A2/eIF4A-II, eIF2AK3/HsPEK, eIF2B4/eIF-2B subunit delta, eIF-2-beta/eIF2S2, eIF31, and 4E-BP1 is determined in a sample from an individual, and wherein
a decreased level of the at least one elF selected from the group consisting of eIF1AD, eIF1AX/eIF-1A X isoform, eIF1AY/eIF-1A Y isoform, eIF-2A, eIF2B5, eIF3j, eIF4A2/eIF4A-II, eIF2B4/eIF-2B subunit delta, eIF-2-beta/eIF2S2, eIF31, and 4E-BP1, and/or
an increased level of the eIF2AK3/HsPEK
in the sample from an individual suffering from lymphoma compared to the reference level A indicates a good prognosis.

More preferably, the level of at least one elF selected from the group consisting of eIF1AD, eIF1AX/eIF-1A X isoform, eIF1AY/eIF-1A Y isoform, eIF-2A, eIF2B5, eIF3j, eIF4A2/eIF4A-II, eIF2B4/eIF-2B subunit delta, and eIF2AK3/HsPEK is determined in a sample from an individual, and wherein
a decreased level of the at least one elF selected from the group consisting of eIF1AD, eIF1AX/eIF-1A X isoform, eIF1AY/eIF-1A Y isoform, eIF-2A, eIF2B5, eIF3j, eIF4A2/eIF4A-II, and eIF2B4/eIF-2B subunit delta, and/or
an increased level of the eIF2AK3/HsPEK
in the sample from an individual suffering from lymphoma compared to the reference level A indicates a good prognosis.

Even more preferably, the level of at least one eukaryotic Initiation Factor (elF) selected from the group consisting of eIF1AY/eIF-1A Y isoform and eIF2B5 is determined in a sample from an individual, and wherein
a decreased level of the at least one elF selected from the group consisting of eIF1AY/eIF-1A Y isoform and eIF2B5
in the sample from an individual suffering from lymphoma compared to the reference level A indicates a good prognosis.

Also described herein, but not encompassed by the present invention, is a method of diagnosing a hepatocellular carcinoma (HCC) in an individual (suspected of having a HCC) comprising the step of:
determining the level of at least one eukaryotic Initiation Factor (elF) in a sample from an individual (suspected of having a HCC),
wherein the at least one elF is selected from the group consisting of eIF2α, preferably peIF2α, eIF3C, eIF3D, eIF3H (Gene: EIF3H/EIF3S3; Gene ID (GenBank): 8667), eIF3I (eIF3S2) (Gene: EIF3I/EIF3S2; Gene ID (GenBank): 8668), eIF4E (eIF4E1, eIF4E2, eIF4E3, Gene: EIF4E1, Gene ID (GenBank): 1977, Gene: EIF4E2, Gene ID (GenBank): 9470, Gene: EIF4E3, Gene ID (GenBank): 317649), eIF4G (eIF4G1, eIF4G2, eIF4G3, Gene: EIF4G1, Gene ID (GenBank): 1981, Gene: EIF4G2, Gene ID (GenBank): 1982, Gene: EIF4G3, Gene ID (GenBank): 8672), and eIF5 (Gene: EIF5; Gene ID (GenBank): 1983).
In this respect, it should be noted that peIF2α is the phosphorylated form of eIF2α. Further, eIF4E preferably comprises the isoforms eIF4E1, eIF4E2, and/or eIF4E3. Furthermore, eIF4G preferably comprises the isoforms eIF4G1, eIF4G2, and/or eIF4G3.
As to the Gene IDs of the elFs, it is also referred to the above-described methods.

For example, the level(s) of at least 1, at least 2, at least 3, at least 4, at least 5, at least 6, at least 7 eIF(s) or of all of the elFs mentioned above is (are) determined.

In one embodiment, the level of the at least one elF is compared to a reference level of said at least one elF. Thus, in one particular embodiment, a method of diagnosing a hepatocellular carcinoma (HCC) in an individual (suspected of having a HCC) is described which comprises the steps of:
(i) determining the level of at least one eukaryotic Initiation Factor (elF) in a sample from an individual (suspected of having a HCC),
   wherein the at least one elF is selected from the group consisting of eIF2α, preferably peIF2α, eIF3C, eIF3D, eIF3H, eIF3I, eIF4E, eIF4G, and eIF5, and
(ii) comparing the level of the at least one elF to a reference level of said at least one elF.

The above comparison allows to diagnose HCC in the individual suspected of having HCC. The individual may be diagnosed as suffering from HCC, i.e. being diseased, or as not suffering from HCC, i.e. being healthy.

The reference level may be any level which allows to determine whether an individual suffers from HCC or not.

It is preferred that the reference level is the level determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference sample(s), from at least one healthy individual, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 healthy individual(s). It is more preferred that the reference level is the level determined by measuring between 2 and 500 reference samples from between 2 and 500 healthy individuals. It is even more preferred that the reference level is determined by measuring between 50 and 500 reference samples from between 50 and 500 healthy individuals. It is most preferred that the reference level is determined by measuring between 100 and 500 reference samples from between 100 and 500 healthy individuals.

It is practicable to take one reference sample per subject for analysis. If additional reference samples are required, e.g. to determine the reference level in different reference samples, the same subject may be (re)tested. Said reference level may be an average reference level. It may be determined by measuring reference levels and calculating the "average" value (e.g. mean, median or modal value) thereof.

As mentioned above, the level of the at least one elF is compared to a reference level of said at least one elF. Said reference level is the level determined by measuring a reference sample. For example, if the level of eIF5 is determined in a sample from an individual, it is compared to a reference level of eIF5 determined in a reference sample. Alternatively, if the level of eIF5 and the level of eIF4B is determined in a sample from an individual, both levels are compared to the respective reference levels, i.e. the level of eIF5 is compared to the reference level of eIF5 and the level of eIF4B is compared to the reference level of eIF4B in a reference sample.

It is further preferred that
the level of the at least one elF selected from the group consisting of eIF3D, eIF3H, and eIF5 below the reference level indicates that the individual has HCC, and/or
the level of the at least one elF selected from the group consisting of peIF2α, eIF3C, eIF3I, eIF4E, and eIF4G above the reference level indicates that the individual has HCC.

Preferably, the level of the at least one elF is at least 0.6-fold or 0.7-fold, more preferably at least 0.8-fold or 0.9-fold, even more preferably at least 1.2-fold or 1.5-fold, and most preferably at least 2.0-fold or 3.0-fold below/above the reference level. For example, the level of the at least one elF is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

It should be noted that with respect to eIF3D, it is preferred that the level is at least 0.6-fold below the reference level. It is more preferred that the level is at least 1.2-fold below the reference level.

With respect to eIF3H, it is preferred that the level is at least 0.6-fold below the reference level. It is more preferred that the level is at least 1.2-fold below the reference level.

With respect to eIF5, it is preferred that the level is at least 0.6-fold below the reference level. It is more preferred that the level is at least 1.2-fold below the reference level.

With respect to peIF2α, it is preferred that the level is at least 1.0-fold above the reference level. It is more preferred that the level is at least 2.0-fold above the reference level.

With respect to eIF3C, it is preferred that the level is at least 2.0-fold above the reference level. It is more preferred that the level is at least 3.0-fold above the reference level.

With respect to eIF3I, it is preferred that the level is at least 1.0-fold above the reference level. It is more preferred that the level is at least 2.5-fold above the reference level.

With respect to eIF4E, it is preferred that the level is at least 1.5-fold above the reference level. It is more preferred that the level is at least 3.0-fold above the reference level.

With respect to eIF4G, it is preferred that the level is at least 1.0-fold above the reference level. It is more preferred that the level is at least 3.0-fold above the reference level.

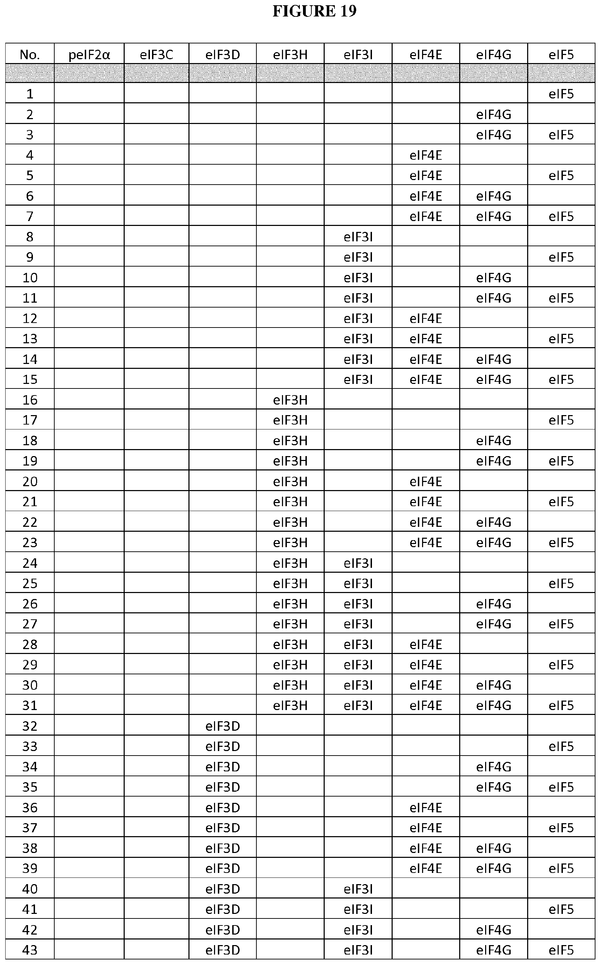

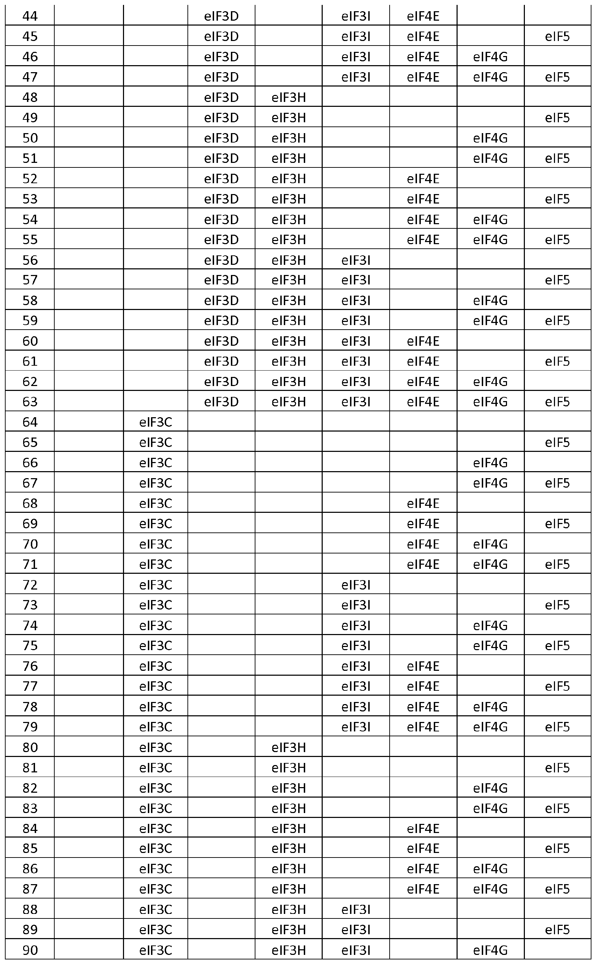

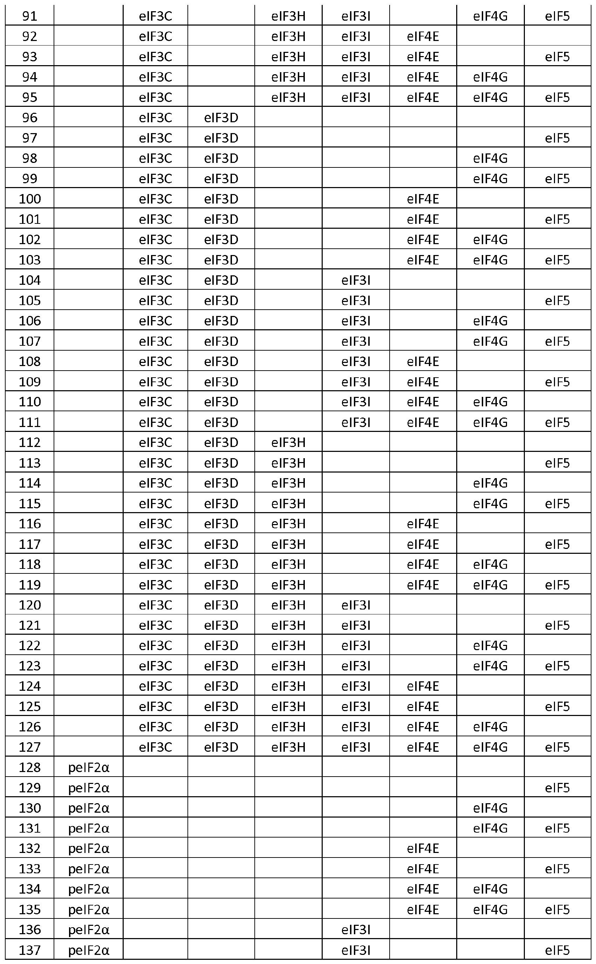

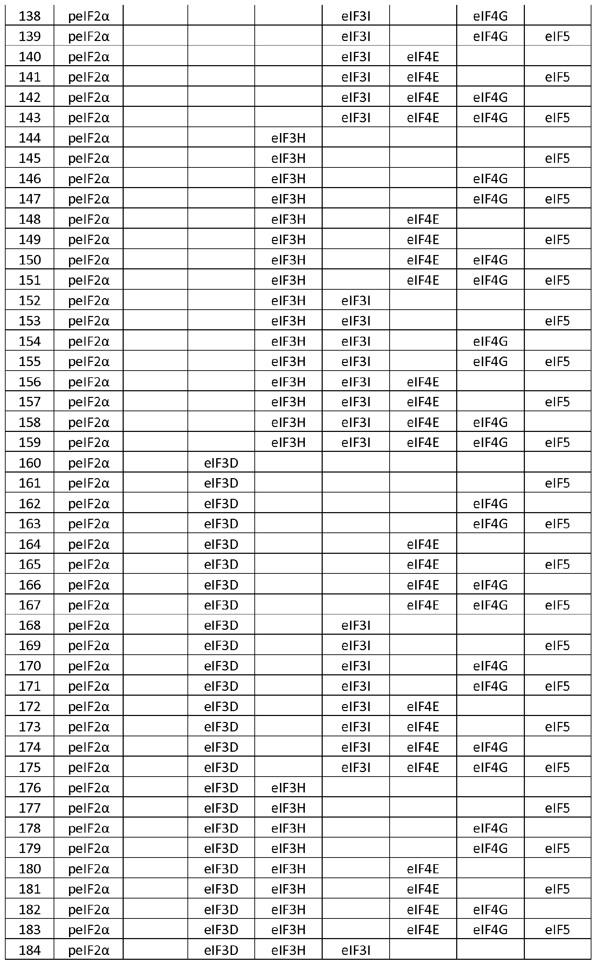

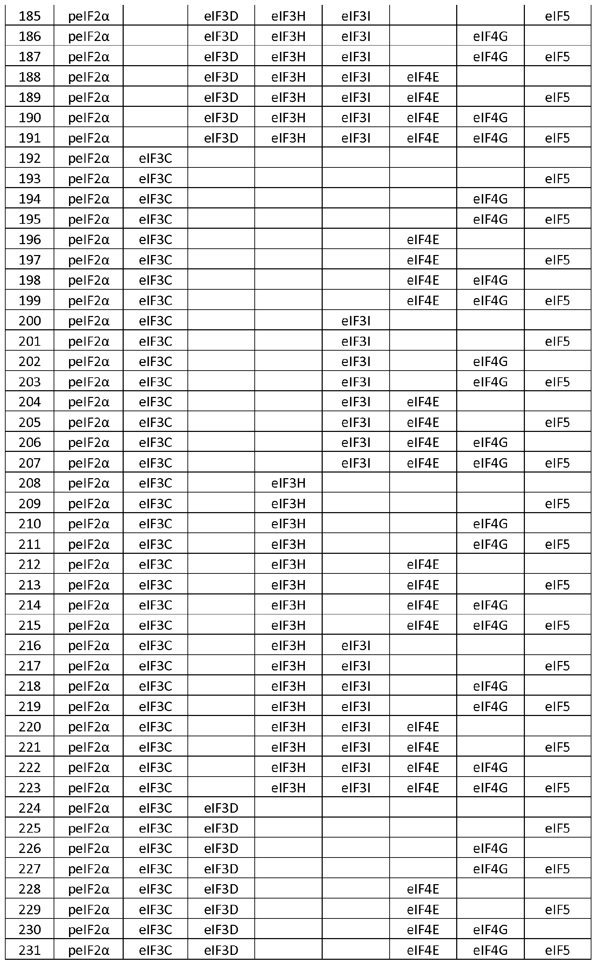

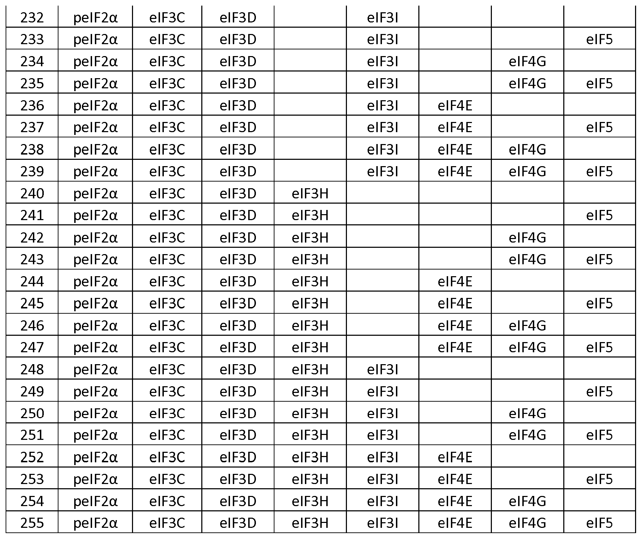

**Figure 19** shows single elFs and sets of elFs which level is preferably determined in a method of diagnosing a hepatocellular carcinoma (HCC) in an individual. These sets comprise 2, 3, 4, 5, 6, 7, or 8 elFs. Alternatively, peIF2α listed in **Figure 19** may be eIF2α.

Also described herein, but not encompassed by the present invention, is a method of diagnosing a hepatitis C virus (HCV) infection in an individual (suspected of having a HCV infection) comprising the step of:
determining the level of at least one eukaryotic Initiation Factor (elF) in a sample from an individual (suspected of having a HCV infection),
wherein the at least one elF is selected from the group consisting of eIF3B and eIF3D.
As to the Gene IDs of the elFs, it is referred to the above-described methods.

For example, the level(s) of at least 1 elF, or of all of the elFs mentioned above is (are) determined.

Preferably, the level(s) of (i) eIF3B, (ii) eIF3D, or (iii) eIF3B and eIF3D is (are) determined.

In one embodiment, the level of the at least one elF is compared to a reference level of said at least one elF. Thus, in one particular embodiment, a method of diagnosing a hepatitis C virus (HCV) infection in an individual (suspected of having a HCV infection) is described which comprises the steps of:
(i) determining the level of at least one eukaryotic Initiation Factor (elF) in a sample from an individual (suspected of having a HCV infection), and
   wherein the at least one elF is selected from the group consisting of eIF3B and eIF3D, and
(ii) comparing the level of the at least one elF to a reference level of said at least one elF.

The above comparison allows to diagnose a HCV infection in the individual suspected of having a HCV infection. The individual may be diagnosed as suffering from a HCV infection, i.e. being diseased, or as not suffering from a HCV infection, i.e. being healthy.

The reference level may be any level which allows to determine whether an individual suffers from a HCV infection or not.

It is preferred that the reference level is the level determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference sample(s), from at least one healthy individual, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 healthy individual(s). It is more preferred that the reference level is the level determined by measuring between 2 and 500 reference samples from between 2 and 500 healthy individuals. It is even more preferred that the reference level is determined by measuring between 50 and 500 reference samples from between 50 and 500 healthy individuals. It is most preferred that the reference level is determined by measuring between 100 and 500 reference samples from between 100 and 500 healthy individuals.

It is practicable to take one reference sample per subject for analysis. If additional reference samples are required, e.g. to determine the reference level in different reference samples, the same subject may be (re)tested. Said reference level may be an average reference level. It may be determined by measuring reference levels and calculating the "average" value (e.g. mean, median or modal value) thereof.

It is further preferred that the level of the at least one elF below the reference level indicates that the individual has a HCV infection.

Preferably, the level of the at least one elF is at least 0.6-fold or 0.7-fold, more preferably at least 0.8-fold or 0.9-fold, even more preferably at least 1.2-fold or 1.5-fold, and most preferably at least 2.0-fold or 3.0-fold below the reference level. For example, the level of the at least one elF is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below the reference level.

It should be noted that with respect to eIF3B, it is preferred that the level is at least 0.9-fold below the reference level. It is more preferred that the level is at least 2.5-fold below the reference level.

With respect to eIF3D, it is preferred that the level is at least 0.8-fold below the reference level. It is more preferred that the level is at least 2.0-fold below the reference level.

More preferably, the level(s) of (i) eIF3B, (ii) eIF3D, or (iii) eIF3B and eIF3D is (are) determined in the above described method.

Also described herein, but not encompassed by the present invention, is a method of diagnosing a hepatitis B virus (HBV) infection in an individual (suspected of having a HBV infection) comprising the step of:
determining the level of at least one eukaryotic Initiation Factor (elF) in a sample from an individual (suspected of having a HBV infection),
wherein the at least one elF is selected from the group consisting of eIF2α, preferably peIF2α, eIF3H, eIF3I, eIF4B, preferably peIF4B, eIF4G, and eIF5.
In this respect, it should be noted that peIF2α is the phosphorylated form of eIF2α and peIF4B is the phosphorylated form of eIF4B. In addition, eIF4G preferably comprises the isoforms eIF4G1, eIF4G2, and/or eIF4G3.
As to the Gene IDs of the elFs, it is referred to the above-described methods.

For example, the level(s) of at least 1, at least 2, at least 3, at least 4, at least 5 eIF(s) or of all of the elFs mentioned above is (are) determined.

In one embodiment, the level of the at least one elF is compared to a reference level of said at least one elF. Thus, in one particular embodiment, a method of diagnosing a hepatitis B virus (HBV) infection in an individual (suspected of having a HBV infection) is described which comprises the steps of:
(i) determining the level of at least one eukaryotic Initiation Factor (elF) in a sample from an individual (suspected of having a HBV infection),
   wherein the at least one elF is selected from the group consisting of eIF2α, preferably peIF2α, eIF3H, eIF3I, eIF4B, preferably peIF4B, eIF4G, and eIF5, and
(ii) comparing the level of the at least one elF to a reference level of said at least one elF.

The above comparison allows to diagnose a HBV infection in the individual suspected of having a HBV infection. The individual may be diagnosed as suffering from a HBV infection, i.e. being diseased, or as not suffering from a HBV infection, i.e. being healthy.

The reference level may be any level which allows to determine whether an individual suffers from a HBV infection or not.

It is preferred that the reference level is the level determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference sample(s), from at least one healthy individual, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 healthy individual(s). It is more preferred that the reference level is the level determined by measuring between 2 and 500 reference samples from between 2 and 500 healthy individuals. It is even more preferred that the reference level is determined by measuring between 50 and 500 reference samples from between 50 and 500 healthy individuals. It is most preferred that the reference level is determined by measuring between 100 and 500 reference samples from between 100 and 500 healthy individuals.

It is practicable to take one reference sample per subject for analysis. If additional reference samples are required, e.g. to determine the reference level in different reference samples, the same subject may be (re)tested. Said reference level may be an average reference level. It may be determined by measuring reference levels and calculating the "average" value (e.g. mean, median or modal value) thereof.

It is further preferred that
the level of the at least one elF selected from the group consisting of eIF3H and eIF3I below the reference level indicates that the individual has a hepatitis B virus (HBV) infection, and/or the level of the at least one elF selected from the group consisting of peIF2α, peIF4B, eIF4G, and eIF5 above the reference level indicates that the individual has a hepatitis B virus (HBV) infection.

Preferably, the level of the at least one elF is at least 0.6-fold or 0.7-fold, more preferably at least 0.8-fold or 0.9-fold, even more preferably at least 1.2-fold or 1.5-fold, and most preferably at least 2.0-fold or 3.0-fold below/above the reference level. For example, the level of the at least one elF is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

It should be noted that with respect to eIF3H, it is preferred that the level is at least 0.5-fold below the reference level. It is more preferred that the level is at least 1.5-fold below the reference level.

With respect to eIF3I, it is preferred that the level is at least 0.5-fold below the reference level. It is more preferred that the level is at least 1.0-fold below the reference level.

With respect to peIF2α, it is preferred that the level is at least 1.0-fold above the reference level. It is more preferred that the level is at least 2.5-fold above the reference level.

With respect to peIF4B, it is preferred that the level is at least 1.5-fold above the reference level. It is more preferred that the level is at least 3.0-fold above the reference level.

With respect to eIF4G, it is preferred that the level is at least 1.5-fold above the reference level. It is more preferred that the level is at least 3.0-fold above the reference level.

With respect to eIF5, it is preferred that the level is at least 1.0-fold above the reference level. It is more preferred that the level is at least 3.0-fold above the reference level.

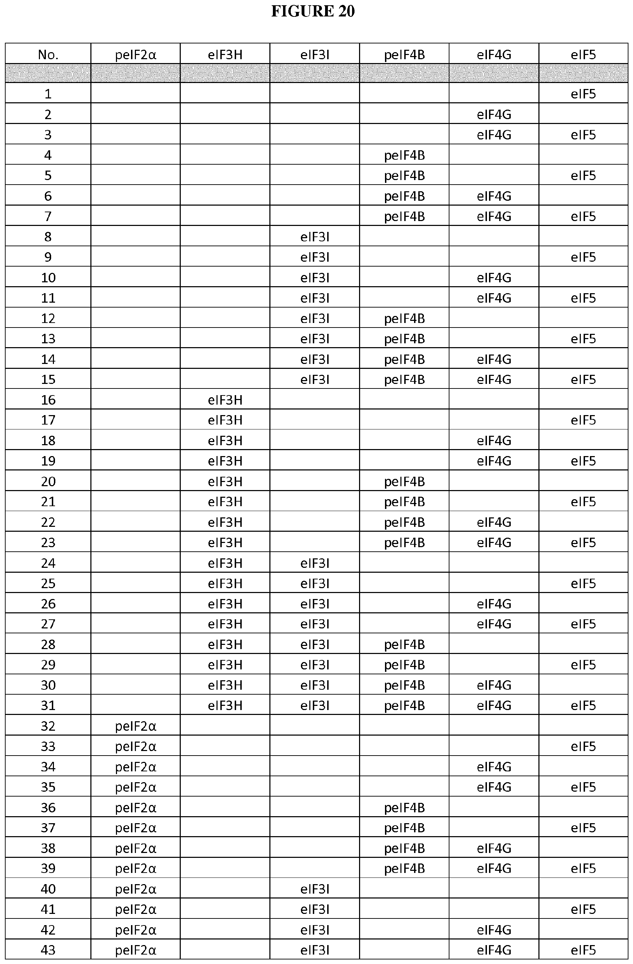

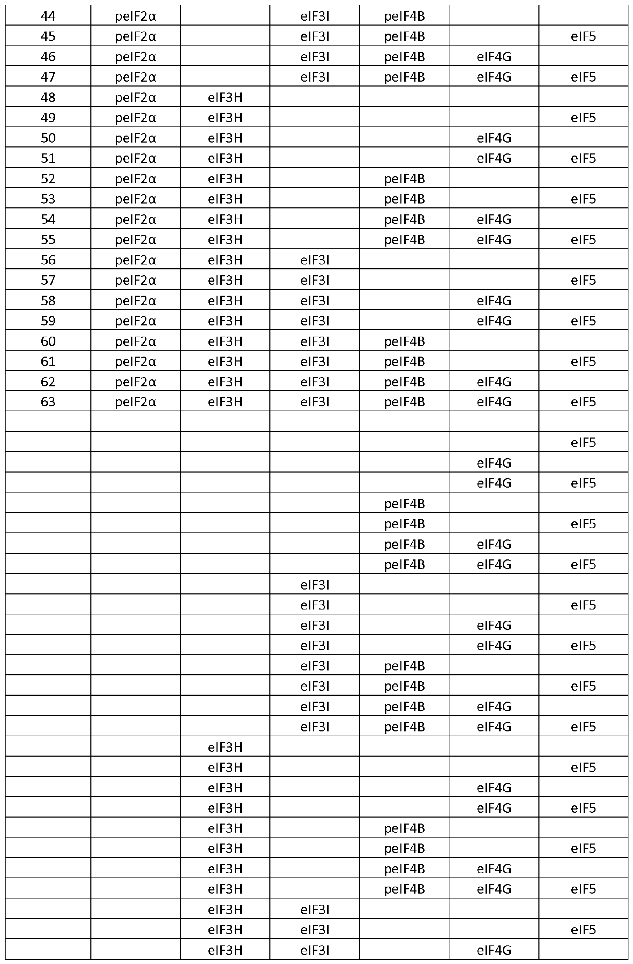

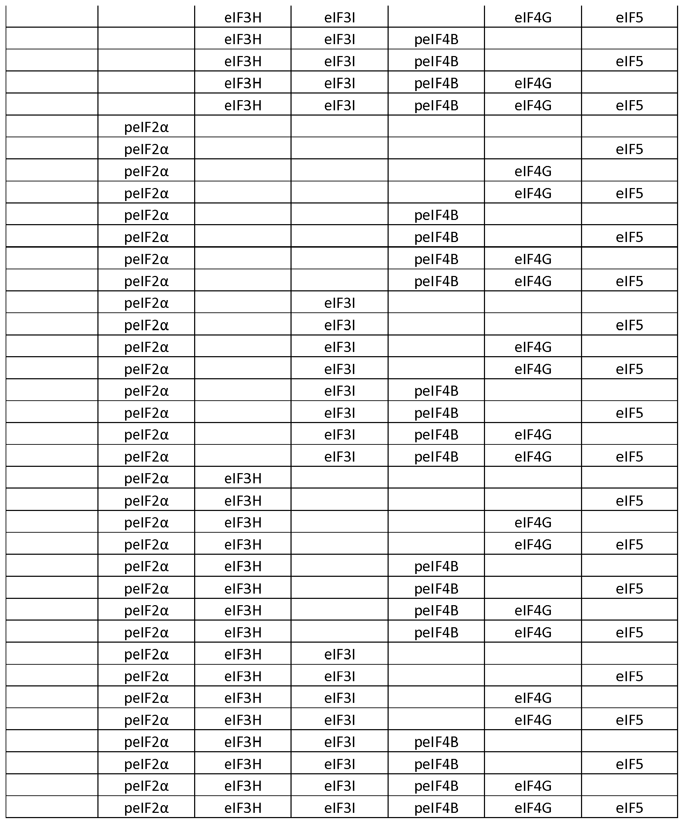

**Figure 20** shows single elFs and sets of elFs which level is preferably determined in a method of diagnosing a hepatitis B virus (HBV) infection in an individual. These sets comprise 2, 3, 4, 5, or 6 elFs. Alternatively, peIF2α listed in **Figure 20** may be eIF2α and/or peIF4B listed in **Figure 20** may be eIF4B.

Also described herein, but not encompassed by the present invention, is a method of diagnosing a viral induced hepatocellular carcinoma (HCC) in an individual (suspected of having a viral induced HCC) comprising the step of:
determining the level of at least one eukaryotic Initiation Factor (elF) in a sample from an individual (suspected of having a viral induced HCC),
wherein the at least one elF is selected from the group consisting of peIF2α, eIF2α (eIF-2A/alpha/α/eIF2S1) (Gene: EIF2S1; Gene ID (GenBank): 1965), eIF3B (Gene: EIF3B; Gene ID (GenBank): 8662), eIF3D (Gene: EIF3D; Gene ID (GenBank): 8664), eIF3H (Gene: EIF3H/EIF3S3; Gene ID (GenBank): 8667), eIF3I (eIF3S2) (Gene: EIF3I/EIF3S2; Gene ID (GenBank): 8668), eIF3J (Gene: EIF3J/EIF3S1; Gene ID (GenBank): 8669), eIF4B (Gene: EIF4B; Gene ID (GenBank): 1975), preferably peIF4B, eIF4G (eIF4G1, eIF4G2, eIF4G3, Gene: EIF4G1, Gene ID (GenBank): 1981, Gene: EIF4G2, Gene ID (GenBank): 1982, Gene: EIF4G3, Gene ID (GenBank): 8672), eIF5 (Gene: EIF5; Gene ID (GenBank): 1983) and EIF6 (Gene: eIF6; Gene ID (GenBank): 3692).
In this respect, it should be noted that peIF2α is the phosphorylated form of eIF2α and peIF4B is the phosphorylated form of eIF4B. In addition, eIF4G preferably comprises the isoforms eIF4G1, eIF4G2, and/or eIF4G3.

For example, the level(s) of at least 1, at least 2, at least 3, at least 4, at least 5, at least 6, at least 7, at least 8, at least 9, at least 10 eIF(s) or of all of the elFs mentioned above is (are) determined.

In one embodiment, the level of the at least one elF is compared to a reference level of said at least one elF. Thus, in one particular embodiment, a method of diagnosing a viral induced hepatocellular carcinoma (HCC) in an individual (suspected of having a viral induced HCC) is described which comprises the steps of:
(i) determining the level of at least one eukaryotic Initiation Factor (elF) in a sample from an individual (suspected of having a viral induced HCC),
   wherein the at least one elF is selected from the group consisting of peIF2α, eIF2α, eIF3B, eIF3D, eIF3H, eIF3I, eIF3J, eIF4B, preferably peIF4B, eIF4G, eIF5, and eIF6, and
(ii) comparing the level of the at least one elF to a reference level of said at least one elF.

The above comparison allows to diagnose a viral induced HCC in the individual suspected of having a viral induced HCC. The individual may be diagnosed as suffering from a viral induced HCC, i.e. being diseased, or as not suffering from a viral induced HCC, i.e. being healthy.

The reference level may be any level which allows to determine whether an individual suffers from a viral induced HCC or not.

It is preferred that the reference level is the level determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference sample(s), from at least one healthy individual, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 healthy individual(s). It is more preferred that the reference level is the level determined by measuring between 2 and 500 reference samples from between 2 and 500 healthy individuals. It is even more preferred that the reference level is determined by measuring between 50 and 500 reference samples from between 50 and 500 healthy individuals. It is most preferred that the reference level is determined by measuring between 100 and 500 reference samples from between 100 and 500 healthy individuals.

It is practicable to take one reference sample per subject for analysis. If additional reference samples are required, e.g. to determine the reference level in different reference samples, the same subject may be (re)tested. Said reference level may be an average reference level. It may be determined by measuring reference levels and calculating the "average" value (e.g. mean, median or modal value) thereof.

It is further preferred that the viral induced hepatocellular carcinoma is hepatitis C virus induced hepatocellular carcinoma (HCC-HCV) or hepatitis B virus induced hepatocellular carcinoma (HCC-HBV), and
wherein the at least one elF is selected from the group consisting of peIF2α, eIF3B, eIF3H, eIF3J, and eIF4G.

It is also preferred that the viral induced hepatocellular carcinoma is hepatitis C virus induced hepatocellular carcinoma (HCC-HCV), and
wherein the at least one elF is selected from the group consisting of peIF2α, eIF3B, eIF3H, eIF3J, eIF4G, and eIF6.

It is also preferred that the viral induced hepatocellular carcinoma is hepatitis B virus induced hepatocellular carcinoma (HCC-HBV), and
wherein the at least one elF is selected from the group consisting of peIF2α, eIF2α, eIF3B, eIF3D, eIF3H, eIF3I, eIF3J, peIF4B, eIF4G, and eIF5.

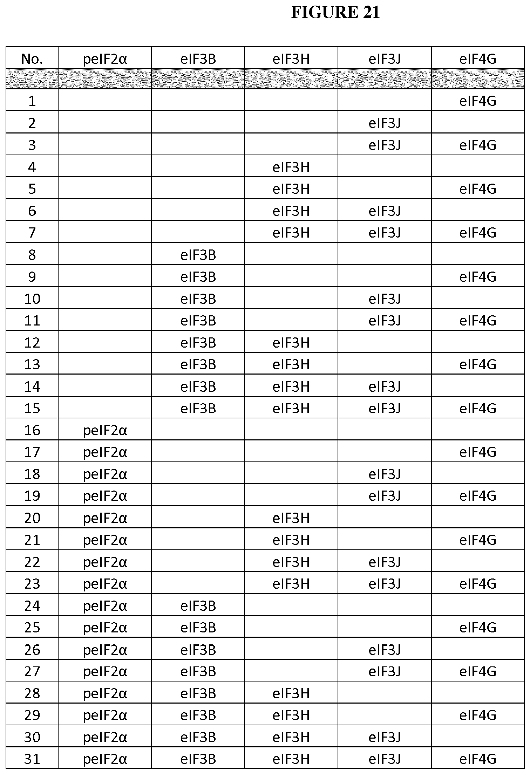

**Figure 21** shows single elFs and sets of elFs which level is preferably determined in a method of diagnosing a viral induced hepatocellular carcinoma (HCC) in an individual. These elFs are particularly preferred as they allow to determine whether the individual suffers from a hepatitis C virus induced hepatocellular carcinoma (HCC-HCV) or hepatitis B virus induced hepatocellular carcinoma (HCC-HBV). These sets comprise 2, 3, 4, or 5 elFs.

It is more preferred that
(i) the level of eIF3H below the reference level indicates that the individual has a hepatitis C virus induced hepatocellular carcinoma (HCC-HCV),
(ii) the level of the at least one elF selected from the group consisting of peIF2α, eIF3B, eIF3J, eIF4G, and eIF6 above the reference level indicates that the individual has a hepatitis C virus induced hepatocellular carcinoma (HCC-HCV),
(iii) the level of the at least one elF selected from the group consisting of eIF2α, eIF3B, eIF3D, eIF3H, eIF3I, and eIF3J below the reference level indicates that the individual has a hepatitis B virus induced hepatocellular carcinoma (HCC-HBV), or
(iv) the level of the at least one elF selected from the group consisting of peIF2α, peIF4B, eIF4G, and eIF5 above the reference level indicates that the individual has a hepatitis B virus induced hepatocellular carcinoma (HCC-HBV).

Preferably, the level of the at least one elF is at least 0.6-fold or 0.7-fold, more preferably at least 0.8-fold or 0.9-fold, even more preferably at least 1.2-fold or 1.5-fold, and most preferably at least 2.0-fold or 3.0-fold below/above the reference level. For example, the level of the at least one elF is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

Also described herein, but not encompassed by the present invention, is a method of differentiating between at least two conditions in an individual, wherein the at least two conditions are selected from the group consisting of a hepatocellular carcinoma (HCC), hepatitis C virus (HCV) infection, hepatitis B virus (HBV) infection, and a viral induced hepatocellular carcinoma (HCC) comprising the step of:
determining the level of at least one eukaryotic Initiation Factor (elF) in a sample from an individual,
wherein the at least one elF is selected from the group consisting of eIF2α, preferably peIF2α, eIF3B, eIF3D, eIF3H, eIF3I, eIF4B, preferably peIF4B, eIF4G, eIF5, and eIF6.
In this respect, it should be noted that peIF2α is the phosphorylated form of eIF2α and peIF4B is the phosphorylated form of eIF4B. In addition, eIF4G preferably comprises the isoforms eIF4G1, eIF4G2, and/or eIF4G3.
As to the Gene IDs of the elFs, it is referred to the above-described methods.

For example, the level(s) of at least 1, at least 2, at least 3, at least 4, at least 5, at least 6, at least 7, at least 8 eIF(s) or of all of the elFs mentioned above is (are) determined.

In one embodiment, the level of the at least one elF is compared to at least one reference level (e.g. at least 1, 2, 3 reference level(s), or 4 reference levels) of said at least one elF. Thus, in one particular embodiment, described herein is a method of differentiating between at least two conditions in an individual, wherein the at least two conditions are selected from the group consisting of a hepatocellular carcinoma (HCC), hepatitis C virus (HCV) infection, hepatitis B virus (HBV) infection, and a viral induced hepatocellular carcinoma (HCC) comprising the steps of:
(i) determining the level of at least one eukaryotic Initiation Factor (elF) in a sample from an individual,
   wherein the at least one elF is selected from the group consisting of eIF2α, preferably peIF2α, eIF3B, eIF3D, eIF3H, eIF3I, eIF4B, preferably peIF4B, eIF4G, eIF5, and eIF6, and
(ii) comparing the level of the at least one elF to at least one reference level (e.g. to at least 1, 2, 3 reference level(s), or 4 reference levels) of said at least one elF.

The above comparison allows to decide whether an individual suffers from (i) a HCC or HCV infection, (ii) a HCC or HBV infection, (iii) a HCC or viral induced HCC, (iv) a HCV infection or HBV infection, (v) a HCV infection or viral induced HCC, (vi) a HBV infection or viral induced HCC, (vii) a HCC, HCV infection or HBV infection, (viii) a HCC, HCV or viral induced HCC, (ix) a HCC, HBV infection or viral induced HCC, (x) a HCV infection, HBV infection or viral induced HCC, or (xi) a HCC, HCV infection, HBV infection or viral induced HCC.

Preferably, the viral induced hepatocellular carcinoma (HCC) is a hepatitis C virus induced hepatocellular carcinoma (HCC-HCV) or hepatitis B virus induced hepatocellular carcinoma (HCC-HBV).

The at least one reference level may be any level which allows to differentiate between the above described diseases or conditions.

It is preferred that the at least one reference level is the level determined by measuring at least one reference sample from
at least one patient with a hepatocellular carcinoma (HCC),
at least one patient with a hepatitis C virus (HCV) infection,
at least one patient with a hepatitis B virus (HBV) infection,
at least one patient with a hepatitis C virus induced hepatocellular carcinoma (HCC-HCV), and/or
at least one patient with a hepatitis B virus induced hepatocellular carcinoma (HCC-HBV).

It is particularly preferred that the reference level is the level determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference sample(s), from at least one patient with a HCC, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 patient(s) with a HCC. It is more preferred that the reference level is the level determined by measuring between 2 and 500 reference samples from between 2 and 500 patients with a HCC. It is even more preferred that the reference level is determined by measuring between 50 and 500 reference samples from between 50 and 500 patients with a HCC. It is most preferred that the reference level is determined by measuring between 100 and 500 reference samples from between 100 and 500 patients with a HCC.

It is further particularly preferred that the reference level is the level determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference sample(s), from at least one patient with a HCV or HBV infection, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 patient(s) with a HCV or HBV infection. It is more preferred that the reference level is the level determined by measuring between 2 and 500 reference samples from between 2 and 500 patients with a HCV or HBV infection. It is even more preferred that the reference level is determined by measuring between 50 and 500 reference samples from between 50 and 500 patients with a HCV or HBV infection. It is most preferred that the reference level is determined by measuring between 100 and 500 reference samples from between 100 and 500 patients with a HCV or HBV infection.

It is also particularly preferred that the reference level is the level determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference sample(s), from at least one patient with a HCC-HCV or HCC-HBV, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 patient(s) with a HCC-HCV or HCC-HBV. It is more preferred that the reference level is the level determined by measuring between 2 and 500 reference samples from between 2 and 500 patients with a HCC-HCV or HCC-HBV. It is even more preferred that the reference level is determined by measuring between 50 and 500 reference samples from between 50 and 500 patients with a HCC-HCV or HCC-HBV. It is most preferred that the reference level is determined by measuring between 100 and 500 reference samples from between 100 and 500 patients with a HCC-HCV or HCC-HBV.

It is practicable to take one reference sample per subject for analysis. If additional reference samples are required, e.g. to determine the reference level in different reference samples, the same subject may be (re)tested. Said reference level may be an average reference level. It may be determined by measuring reference levels and calculating the "average" value (e.g. mean, median or modal value) thereof.

It is more preferred that
(i) the at least one reference level is the level determined by measuring at least one reference sample from at least one patient with a hepatitis C virus (HCV) infection, and wherein the level of peIF2α below the reference level indicates that the individual has a hepatocellular carcinoma (HCC), or wherein the level of peIF2α above the reference level indicates that the individual has a hepatitis C virus induced hepatocellular carcinoma (HCC-HCV),
(ii) the at least one reference level is the level determined by measuring at least one reference sample from at least one patient with a hepatitis B virus induced hepatocellular carcinoma (HCC-HBV), and
   wherein the level of peIF2α below the reference level indicates that the individual has a hepatocellular carcinoma (HCC), or wherein the level of peIF2α above the reference level indicates that the individual has a hepatitis B virus (HBV) infection,
(iii) the at least one reference level is the level determined by measuring at least one reference sample from at least one patient with a hepatocellular carcinoma (HCC), and wherein the level of eIF3B below the reference level indicates that the individual has a hepatitis C virus (HCV) infection, or wherein the level of eIF3B above the reference level indicates that the individual has a hepatitis C virus induced hepatocellular carcinoma (HCC-HCV),
(iv) the at least one reference level is the level determined by measuring at least one reference sample from at least one patient with a hepatocellular carcinoma (HCC), and wherein the level of eIF3B below the reference level indicates that the individual has a hepatitis B virus induced hepatocellular carcinoma (HCC-HBV), or wherein the level of eIF3B above the reference level indicates that the individual has a hepatitis B virus (HBV) infection,
(v) the at least one reference level is the level determined by measuring at least one reference sample from at least one patient with a hepatocellular carcinoma (HCC), and wherein the level of eIF3D below the reference level indicates that the individual has a hepatitis C virus (HCV) infection, or wherein the level of eIF3D above the reference level indicates that the individual has a hepatitis C virus induced hepatocellular carcinoma (HCC-HCV),
(vi) the at least one reference level is the level determined by measuring at least one reference sample from at least one patient with a hepatitis B virus (HBV) infection, and wherein the level of eIF3D below the reference level indicates that the individual has a hepatitis B virus induced hepatocellular carcinoma (HCC-HBV), or wherein the level of eIF3D above the reference level indicates that the individual has a hepatocellular carcinoma (HCC),
(vii) the at least one reference level is the level determined by measuring at least one reference sample from at least one patient with a hepatitis C virus induced hepatocellular carcinoma (HCC-HCV), and
   wherein the level of eIF3H below the reference level indicates that the individual has a hepatocellular carcinoma (HCC), or wherein the level of eIF3H above the reference level indicates that the individual has a hepatitis C virus (HCV) infection,
(viii) the at least one reference level is the level determined by measuring at least one reference sample from at least one patient with a hepatocellular carcinoma (HCC), and wherein the level of eIF3H below the reference level indicates that the individual has a hepatitis B virus (HBV) infection, or wherein the level of eIF3H above the reference level indicates that the individual has hepatitis B virus induced hepatocellular carcinoma (HCC-HBV),
(ix) the at least one reference level is the level determined by measuring at least one reference sample from at least one patient with a hepatitis C virus induced hepatocellular carcinoma (HCC-HCV), and
   wherein the level of eIF3I below the reference level indicates that the individual has a hepatitis C virus (HCV) infection, or wherein the level of eIF3I above the reference level indicates that the individual has a hepatocellular carcinoma (HCC),
(x) the at least one reference level is the level determined by measuring at least one reference sample from at least one patient with hepatitis B virus (HBV) infection, and wherein the level of eIF3I below the reference level indicates that the individual has a hepatitis B virus induced hepatocellular carcinoma (HCC-HBV), or wherein the level of eIF3I above the reference level indicates that the individual has a hepatocellular carcinoma (HCC),
(xi) the at least one reference level is the level determined by measuring at least one reference biological sample from at least one patient with a hepatocellular carcinoma (HCC) and/or a hepatitis C virus induced hepatocellular carcinoma (HCC-HCV), and wherein the level of peIF4B above the reference level(s) indicates that the individual has a hepatitis C virus (HCV) infection,
(xii) the at least one reference level is the level determined by measuring at least one reference sample from at least one patient with a hepatitis B virus induced hepatocellular carcinoma (HCC-HBV), and
   wherein the level of peIF4B below the reference level indicates that the individual has a hepatocellular carcinoma (HCC), or wherein the level of peIF4B above the reference level indicates that the individual has a hepatitis B virus (HBV) infection,
(xiii) the at least one reference level is the level determined by measuring at least one reference sample from at least one patient with a hepatocellular carcinoma (HCC), and wherein the level of eIF4G below the reference level indicates that the individual has a hepatitis C virus (HCV) infection, or wherein the level of eIF4G above the reference level indicates that the individual has a hepatitis C virus induced hepatocellular carcinoma (HCC-HCV),
(xiv) the at least one reference level is the level determined by measuring at least one reference sample from at least one patient with hepatitis B virus (HBV) infection, and wherein the level of eIF4G below the reference level indicates that the individual has a hepatitis B virus induced hepatocellular carcinoma (HCC-HBV), or wherein the level of eIF4G above the reference level indicates that the individual has a hepatocellular carcinoma (HCC),
(xv) the at least one reference level is the level determined by measuring at least one reference sample from at least one patient with a hepatitis C virus induced hepatocellular carcinoma (HCC-HCV) and/or a hepatitis C virus (HCV) infection, and
   wherein the level of eIF5 below the reference level(s) indicates that the individual has a hepatocellular carcinoma (HCC),
(xvi) the at least one reference level is the level determined by measuring at least one reference sample from at least one patient with a hepatitis B virus induced hepatocellular carcinoma (HCC-HBV), and
   wherein the level of eIF5 below the reference level indicates that the individual has a hepatocellular carcinoma (HCC), or wherein the level of eIF5 above the reference level indicates that the individual has a hepatitis B virus (HBV) infection,
(xvii) the at least one reference level is the level determined by measuring at least one reference sample from at least one patient with a hepatitis C virus (HCV) infection, and wherein the level of eIF6 below the reference level indicates that the individual has a hepatocellular carcinoma (HCC), or wherein the level of eIF6 above the reference level indicates that the individual has a hepatitis C virus induced hepatocellular carcinoma (HCC-HCV), and/or
(xviii) the at least one reference level is the level determined by measuring at least one reference sample from at least one patient with hepatitis B virus (HBV) infection, and wherein the level of eIF6 below the reference level indicates that the individual has a hepatitis B virus induced hepatocellular carcinoma (HCC-HBV), or wherein the level of eIF6 above the reference level indicates that the individual has a hepatocellular carcinoma (HCC).

Preferably, the level of the at least one elF is at least 0.6-fold or 0.7-fold, more preferably at least 0.8-fold or 0.9-fold, even more preferably at least 1.2-fold or 1.5-fold, and most preferably at least 2.0-fold or 3.0-fold below/above the reference level. For example, the level of the at least one elF is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

**Figure 22** shows single elFs and sets of elFs which level is preferably determined in a method of differentiating between at least two conditions in an individual, wherein the at least two conditions are selected from the group consisting of a hepatocellular carcinoma (HCC), hepatitis C virus (HCV) infection, hepatitis B virus (HBV) infection, and a viral induced hepatocellular carcinoma (HCC). The viral induced hepatocellular carcinoma is preferably a hepatitis C virus induced hepatocellular carcinoma (HCC-HCV) or hepatitis B virus induced hepatocellular carcinoma (HCC-HBV). These sets comprise 2, 3, 4, 5, 6, 7, 8, or 9 elFs. Alternatively, peIF2α listed in **Figure 22** may be eIF2α and/or peIF4B listed in **Figure 22** may be eIF4B.

Also described herein, but not encompassed by the present invention, is a method of diagnosing a lymphoma in an individual (suspected of having a lymphoma) comprising the step of:
determining the level of at least one eukaryotic Initiation Factor (elF) in a sample from an individual (suspected of having a lymphoma),
wherein the at least one elF is selected from the group consisting of eIF1AX/eIF-1A X isoform, eIF1AY/eIF-1A Y isoform, eIF2AK3/HsPEK, eIF2B4/eIF-2B subunit delta, eIF2B5, and eIF4A2/eIF4A-II.
As to the Gene IDs of the elFs, it is referred to the above-described methods.

For example, the level(s) of at least 1, at least 2, at least 3, at least 4, at least 5 eIF(s) or of all of the elFs mentioned above is (are) determined.

It is preferred that the lymphoma is a Diffuse Large B-cell Lymphoma (DLBCL). More preferably, the DLBCL is a germinal center B-cell (GCB) subtype of DLBCL or a non-germinal center B-cell (nGCB) subtype of DLBCL. It is more preferred that the germinal center B-cell (GCB) subtype is a primary germinal center B-cell (pGCB) disease or a secondary germinal center B-cell (FLIII-GCB) disease. In particular, the secondary germinal center B-cell disease arises/goes out from a Follicular Lymphoma grade III.

It is particularly preferred that the lymphoma is a Diffuse Large B-cell Lymphoma (DLBCL), and
wherein the at least one elF is selected from the group consisting of eIF1AX/eIF-1A X isoform, eIF1AY/eIF-1A Y isoform, eIF2AK3/HsPEK, and eIF4A2/eIF4A-II.
It is particularly more preferred that the Diffuse Large B-cell Lymphoma (DLBCL) is a non-germinal center B-cell (nGCB) subtype of DLBL, primary germinal center B-cell (pGCB) disease, or secondary germinal center B-cell (FLIII-GCB) disease, and
wherein the at least one elF is selected from the group consisting of eIF1AX/eIF-1A X isoform, eIF1AY/eIF-1A Y isoform, eIF2AK3/HsPEK, and eIF4A2/eIF4A-II.
**Figure 27** clearly shows that a significant overexpression of eIF1AX/eIF-1A X isoform, eIF1AY/eIF-1A Y isoform, eIF2AK3/HsPEK, and eIF4A2/eIF4A-II could be determined in all different subtypes of DLBCL, e.g. in a non-germinal center B-cell (nGCB) subtype of DLBL, primary germinal center B-cell (pGCB) disease, or secondary germinal center B-cell (FLIII-GCB) disease .

It is also particularly preferred that the lymphoma is a non-germinal center B-cell (nGCB) subtype of DLBL or secondary germinal center B-cell (FLIII-GCB) disease, and wherein the at least one elF is selected from the group consisting of eIF2B4/eIF-2B subunit delta and eIF2B5.
**Figure 27** clearly shows that a significant overexpression of eIF2B4/eIF-2B subunit delta and eIF2B5 could be determined in a non-germinal center B-cell (nGCB) subtype of DLBL or secondary germinal center B-cell (FLIII-GCB) disease.

In one embodiment, the level of the at least one elF is compared to a reference level of said at least one elF. Thus, in one particular embodiment, described herein is a method of diagnosing a lympoma in an individual (suspected of having a lymphoma) comprising the steps of:
(i) determining the level of at least one eukaryotic Initiation Factor (elF) in a sample from an individual (suspected of having a lymphoma),
   wherein the at least one elF is selected from the group consisting of eIF1AX/eIF-1A X isoform, eIF1AY/eIF-1A Y isoform, eIF2AK3/HsPEK, eIF2B4/eIF-2B subunit delta, eIF2B5, and eIF4A2/eIF4A-II, and
(ii) comparing the level of the at least one elF to a reference level of said at least one elF.

The above comparison allows to diagnose a lymphoma in the individual suspected of having a lymphoma. The individual may be diagnosed as suffering from a lymphoma, i.e. being diseased, or as not suffering from a lymphoma, i.e. being healthy.

The reference level may be any level which allows to determine whether an individual suffers from a lymphoma or not.

It is preferred that the reference level is the level determined by measuring at least one reference sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference sample(s), from at least one healthy individual, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 healthy individual(s). It is more preferred that the reference level is the level determined by measuring between 2 and 500 reference samples from between 2 and 500 healthy individuals. It is even more preferred that the reference level is determined by measuring between 50 and 500 reference samples from between 50 and 500 healthy individuals. It is most preferred that the reference level is determined by measuring between 100 and 500 reference samples from between 100 and 500 healthy individuals.

It is practicable to take one reference sample per subject for analysis. If additional reference samples are required, e.g. to determine the reference level in different reference samples, the same subject may be (re)tested. Said reference level may be an average reference level. It may be determined by measuring reference levels and calculating the "average" value (e.g. mean, median or modal value) thereof.

It is further preferred that
the level of the at least one elF (eIF1AX/eIF-1A X isoform, eIF1AY/eIF-1A Y isoform, eIF2AK3/HsPEK, eIF2B4/eIF-2B subunit delta, eIF2B5, and/or eIF4A2/eIF4A-II) above the reference level indicates that the individual has a lymphoma.

Preferably, the level of the at least one elF is at least 0.5-fold, 1.0-fold, 1.2-fold, or 1.5-fold, more preferably at least 2.0-fold, 3.0-fold, or 4.0-fold, even more preferably at least 5.0-fold, 6.0-fold, or 7.0-fold, and most preferably at least 8.0-fold, 9.0-fold, or 10-fold above the reference level. For example, the level of the at least one elF is at least 0.5-fold, at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, at least 3.0-fold, at least 4.0-fold, at least 5.0-fold, at least 6.0-fold, at least 7.0-fold, at least 8.0-fold, at least 9.0-fold, or at least 10.0-fold above the reference level.

In the method of the first aspect of the present invention, it is preferred that the sample is a biological sample, in particular a (tumor) tissue or a body fluid sample. It is also preferred that the reference sample is a reference biological sample, in particular a tumor tissue or a body fluid sample.

Preferably, the body fluid sample is selected from the group consisting of a blood sample, a urine sample, a lymph sample, a saliva sample and a combination thereof. More preferably, the blood sample is a whole blood sample or a blood fraction sample. Even more preferably, the blood fraction sample is a blood cell fraction sample, a blood serum sample, or a blood plasma sample.

Preferably, the aforementioned samples are pre-treated before they are used in the method of the first aspect of the present invention. Said pre-treatment may include treatments required to separate eIF2AK4, or to remove excessive material or waste. Furthermore, pre-treatments may aim at sterilizing samples and/or removing contaminants such as undesired cells, bacteria or viruses. Suitable techniques comprise centrifugation, extraction, fractioning, ultrafiltration, protein precipitation followed by filtration and purification and/or enrichment of compounds. Moreover, other pre-treatments are carried out in order to provide eIF2AK4 in a form or concentration suitable for analysis.

In one embodiment of the method according to the first aspect of the present invention, the sample used to determine the level of eIF2AK4 can be a tumor tissue (obtainable e.g. by biopsy) or a body fluid. The body fluid is preferably whole blood, serum, lymph or saliva. eIF2AK4 of the present invention can be found in the tissue affected with the tumor and in body fluids like blood and blood components (e.g. serum), lymph and saliva.

According to another preferred embodiment of the method according to the first aspect of the present invention, the level of eIF2AK4 is determined by measuring mRNA or protein levels.

The level of eIF2AK4 in the method according to the first aspect of the present invention can be determined either by measuring mRNA molecules encoding said elF or the elF as such in form of a protein. Methods to determine mRNA levels and protein levels in a sample are well known.

mRNA expression levels are usually measured by polymerase chain reaction (PCR), in particular by reverse transcription quantitative polymerase chain reaction (RT-PCR and qPCR) or real-time PCR. RT-PCR is used to create a cDNA from the mRNA. The cDNA may be used in a qPCR assay to produce fluorescence as the DNA amplification process progresses. This fluorescence is proportional to the original mRNA amount in the samples. Other methods to be used include Northern blots, Fluorescence in situ hybridization (FISH), microarrays, and RT-PCR combined with capillary electrophoresis.

Protein levels of elFs are preferably determined using immunoassays. Such methods are based on the binding of an antibody, a derivative or a fragment thereof to its corresponding target (i.e. eIF). Polyclonal and monoclonal antibodies can be used in such methods. Derivatives or fragments of antibodies include Fab fragments, F(ab')2 fragments, Fv fragments, single chain antibodies and single domain antibodies. Preferred immunoassays include Western blot, Immunohistochemistry, ELISA (enzyme-linked immunosorbent assay), radioimmunoassays, fluorescence resonance energy transfer (FRET) or time resolved-FRET (TR-FRET). Immunoassays detection is possible in lymphoma and HCC. Already for Westernblot used antibodies in the laboratory of the present inventors include, e.g., eIF-2α (Cell Signaling), eIF3c (Cell Signaling), eIF-4B (Cell Signaling), eIF-4G (Cell Signaling), 4E-BP1 (Cell Signaling), eIF3b (Santa Cruz Biotechnology, INC.), eIF3d (Santa Cruz Biotechnology, INC.) and eIF-5 (GeneTex).

It is particularly preferred to use antibodies and derivatives or fragments of antibodies which have been obtained from a non-human source. These antigen binding molecules can be of porcine, rabbit, murine, camel or rat origin. Of course, it is also possible to use antibodies and derivatives or fragments thereof which are recombinantly produced in plants or cell cultures, in particular microbial cell cultures (e.g. bacteria, yeast).

In a second aspect, the present invention relates to the use of a kit comprising means for determining the level of eIF2AK4 in a sample from an individual for providing a prognosis to the individual suffering from a tumor, wherein the tumor is a lymphoma or a hepatocellular carcinoma.

Preferably, the sample is a tumor tissue or a body fluid.

The level of eIF2AK4 is determined by measuring mRNA or protein levels.

Also described herein, but not encompassed by the present invention, is a kit comprising/consisting of
means for determining the level of at least one elF in a sample from an individual, wherein the at least one elF is selected from the group consisting of:
(i) eIF2AK4, eIF2B4/eIF-2B subunit delta, eIF2C 3, eIF2d, eIF-2A/alpha/a/eIF2S1, eIF-2-beta/eIF2S2, eIF3b, eIF3c, eIF3d, eIF3f, eIF3g, eIF31, eIF-4B, 4E-BP1, eIF-4G1, eIF-5A, eIF2AK3/HsPEK, eIF-4E3, eIF-5, eIF1AD, eIF1AX/eIF-1A X isoform, eIF1AY/eIF-1A Y isoform, eIF-2A, eIF2B5, eIF3j, and eIF4A2/eIF4A-II,
(ii) eIF2α, preferably peIF2α, eIF3C, eIF3D, eIF3H, eIF3I, eIF4E, eIF4G, and eIF5,
(iii) eIF3B and eIF3D,
(iv) eIF2α, preferably peIF2α, eIF3H, eIF3I, eIF4B, preferably peIF4B, eIF4G, and eIF5,
(v) peIF2α, eIF2α, eIF3B, eIF3D, eIF3H, eIF3I, eIF3J, eIF4B, preferably peIF4B, eIF4G, eIF5, and eIF6,
(vi) eIF2α, preferably peIF2α, eIF3B, eIF3D, eIF3H, eIF3I, eIF4B, preferably peIF4B, eIF4G, eIF5, and eIF6, and/or
(vii) eIF1AX/eIF-1A X isoform, eIF1AY/eIF-1A Y isoform, eIF2AK3/HsPEK, eIF2B4/eIF-2B subunit delta, eIF2B5, and eIF4A2/eIF4A-II.
In this respect, it should be noted that peIF2α is the phosphorylated form of eIF2α and peIF4B is the phosphorylated form of eIF4B. Further, eIF4G preferably comprises the isoforms eIF4G1, eIF4G2, and/or eIF4G3. Furthermore, eIF4E preferably comprises the isoforms eIF4E1, eIF4E2, and/or eIF4E3.
As to the Gene IDs of the elFs, it is referred to the above-described methods.

As to the specific elF combinations, it is referred to the above-described methods.

Said means may be primers or primer pairs allowing the detecting of the above mentioned eIFs on the RNA transcript, e.g. mRNA, level and/or antibodies, antibody derivatives or fragments of antibodies allowing the detection of the above mentioned elFs on the protein level. In addition, said means encompass dipstrips or dipsticks, e.g. urine or blood dipstrips or dipsticks. Said means are tools used to determine changes in individual's urine or blood. A dipstrip or dipstick comprises different chemical pads or reagents which react (e.g. change color, in particular by applying an immune assay) when immersed in (e.g. blood or urine), and then removed from the biological sample (e.g. urine or blood sample). The result can be read after a few minutes, preferably after a few seconds.

The kit is useful for conducting the above-described methods. In particular, the kit comprising the elFs referred to in
(i) (mentioned above) is useful for carrying out the method of providing a prognosis to an individual suffering from a tumor,
(ii) (mentioned above) is useful for carrying out the method of diagnosing a hepatocellular carcinoma (HCC) in an individual (suspected of having a HCC),
(iii) (mentioned above) is useful for carrying out the method of diagnosing a hepatitis C virus (HCV) infection in an individual (suspected of having a HCV infection),
(iv) (mentioned above) is useful for carrying out the method of diagnosing a hepatitis B virus (HBV) infection in an individual (suspected of having a HBV infection),
(v) (mentioned above) is useful for carrying out the method of diagnosing a viral induced hepatocellular carcinoma (HCC) in an individual (suspected of having a viral induced HCC) such as hepatitis C virus induced hepatocellular carcinoma (HCC-HCV) or hepatitis B virus induced hepatocellular carcinoma (HCC-HBV),
(vi) (mentioned above) is useful for carrying out the method of differentiating between at least two conditions in an individual, wherein the at least two conditions are selected from the group consisting of a hepatocellular carcinoma (HCC), hepatitis C virus (HCV) infection, hepatitis B virus (HBV) infection, and a viral induced hepatocellular carcinoma (HCC), and/or
(vii) (mentioned above) is useful for carrying out the method of diagnosing a lymphoma in an individual (suspected of having a lymphoma).

The kit may further comprise
(i) a container, and/or
(ii) a data carrier.
Said data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. The access code may also allow access to an application software that causes a computer to perform tasks for computer users or a mobile app which is a software designed to run on smartphones and other mobile devices.
Said data carrier may further comprise a reference level of the at least one elF referred to herein. In case that the data carrier comprises an access code which allows the access to a database, said reference level is deposited in this database.
In addition, the data carrier may comprise information or instructions on how to carry out the above-described methods.

Described herein, but not encompassed by the present invention, is the use of at least one elF selected from the group consisting of eIF2AK4, eIF2B4/eIF-2B subunit delta, eIF2C 3, eIF2d, eIF-2A/alpha/a/eIF2S1, eIF-2-beta/eIF2S2, eIF3b, eIF3c, eIF3d, eIF3f, eIF3g, eIF31, eIF-4B, 4E-BP1, eIF-4G1, eIF-5A, eIF2AK3/HsPEK, eIF-4E3, eIF-5, eIF1AD, eIF1AX/eIF-1A X isoform, eIF1AY/eIF-1A Y isoform, eIF-2A, eIF2B5, eIF3j, and eIF4A2/eIF4A-II in the provision of a prognosis to an individual suffering from a tumor.

Described herein, but not encompassed by the present invention, is the use of at least one elF selected from the group consisting of eIF2α, preferably peIF2α, eIF3C, eIF3D, eIF3H, eIF3I, eIF4E, eIF4G, and eIF5 for diagnosing a hepatocellular carcinoma (HCC) in an individual (suspected of having a HCC).

Described herein, but not encompassed by the present invention, is the use of at least one elF selected from the group consisting of eIF3B and eIF3D for diagnosing a hepatitis C virus (HCV) infection in an individual (suspected of having a HCV infection).

Described herein, but not encompassed by the present invention, is the use of at least one elF selected from the group consisting of eIF2α, preferably peIF2α, eIF3H, eIF3I, eIF4B, preferably peIF4B, eIF4G, and eIF5 for diagnosing a hepatitis B virus (HBV) infection in an individual (suspected of having a HBV infection).

Described herein, but not encompassed by the present invention, is the use of at least one elF selected from the group consisting of peIF2α, eIF2α, eIF3B, eIF3D, eIF3H, eIF3I, eIF3J, eIF4B, preferably peIF4B, eIF4G, eIF5, and eIF6 for diagnosing a viral induced hepatocellular carcinoma (HCC) in an individual (suspected of having a viral induced HCC). The viral induced hepatocellular carcinoma (HCC) may be hepatitis C virus induced hepatocellular carcinoma (HCC-HCV) or hepatitis B virus induced hepatocellular carcinoma (HCC-HBV).

Described herein, but not encompassed by the present invention, is the use of at least one elF selected from the group consisting of eIF2α, preferably peIF2α, eIF3B, eIF3D, eIF3H, eIF3I, eIF4B, preferably peIF4B, eIF4G, eIF5, and eIF6 for differentiating between at least two conditions in an individual, wherein the at least two conditions are selected from the group consisting of a hepatocellular carcinoma (HCC), hepatitis C virus (HCV) infection, hepatitis B virus (HBV) infection, and a viral induced hepatocellular carcinoma (HCC). The viral induced hepatocellular carcinoma (HCC) may be hepatitis C virus induced hepatocellular carcinoma (HCC-HCV) or hepatitis B virus induced hepatocellular carcinoma (HCC-HBV).

Described herein, but not encompassed by the present invention, is the use of at least one elF selected from the group consisting of eIF1AX/eIF-1A X isoform, eIF1AY/eIF-1A Y isoform, eIF2AK3/HsPEK, eIF2B4/eIF-2B subunit delta, eIF2B5, and eIF4A2/eIF4A-II for diagnosing a lymphoma in an individual (suspected of having a lymphoma). Preferably, the lymphoma is a Diffuse Large B-cell Lymphoma (DLBCL). More preferably, the DLBCL is a germinal center B-cell (GCB) subtype of DLBCL or a non-germinal center B-cell (nGCB) subtype of DLBL. Even more preferably, the germinal center B-cell (GCB) subtype is a primary germinal center B-cell (pGCB) disease or a secondary germinal center B-cell (FLIII-GCB) disease. In particular, the secondary germinal center B-cell disease arises/goes out from a Follicular Lymphoma grade III.

In this respect, it should be noted that peIF2α is the phosphorylated form of eIF2α and peIF4B is the phosphorylated form of eIF4B. Further, eIF4G preferably comprises the isoforms eIF4G1, eIF4G2, and/or eIF4G3. Furthermore, eIF4E preferably comprises the isoforms eIF4E1, eIF4E2, and/or eIF4E3.

As to the Gene IDs of the elFs, it is referred to the above-described methods.

For the above mentioned uses, the level of the above mentioned eIFs is determined in a sample, in particular in a biological sample, from an individual to be tested. It is preferred that the biological sample is a body fluid sample or a (tumor) tissue sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.

Regarding the specific (preferred) elF combinations, it is referred to the above-described methods.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

The present invention is further illustrated by the following examples, however, without being restricted thereto.

### EXAMPLES

### Example 1: Prognosis of individuals suffering from lymphoma

Blood cancer is one of the most important cancers in Europe. Within the wide-spread group of blood cancer, malignant lymphoma are a heterogeneous group of neoplastic disorders affecting the lymphatic system. 95% are of B-cell origin. Within B-cell lymphomas a further distinction can be made into Hodgkin's (HL) and non-Hodgkin's lymphoma (NHL). NHL comprise neoplasms with diverse biological and clinical manifestations, including the most common lymphoma subtype, the Diffuse Large B-cell Lymphoma (DLBCL). The prognosis for these lymphatic neoplasms is still bad with 35% of affected patients dying of the disease within the first year after diagnosis. Treatment options are limited, mostly focusing on chemotherapeutic approaches.
So far several publications about the impact of eIFs on lymphomagenesis and lymphoma progression exist. However, the involved research groups have mainly focused on the eIF4F complex and its contribution.
Importantly, there are no publications so far analyzing the relationship between the expression of the whole range of eIF-subunits and patient outcome. As mentioned above, the research focus has been mainly concentrated on the eIF4F-complex. Thus, research studies, investigating the complete range of elFs, are lacking. Our research group intended to fill this gap.

### Materials and methods

The survival analysis between respective elF expression and patient survival was performed based on the Lenz-dataset which was published by Lenz G et al. (New Engl J Med 359(2008):2313-2323). Gene expression of 56 elFs (see Table 1 for a list of the elFs) was analyzed in 200 Diffuse Large B-cell Lymphoma patients which were treated with R-CHOP-chemotherapy (Combination therapy composed of the active ingredients: **Rituximab, C**yclophosphamide, **H**ydroxydaunorubicin, **V**incristine, **P**redniso(lo)ne). R-CHOP-chemotherapy is the standard treatment approach to treat this kind of lymphatic cancers. A panel of expert hematopathologists confirmed the diagnosis of DLBCL using current World Health Organization criteria. The gene expression was investigated on mRNA level by using Affymetrix U133 plus 2.0 microarrays (Affymetrix, USA).

**Table 1: List of the total range of eIFs analyzed by our bioinformatic analysis.**

| | | | |
|---|---|---|---|
| EIF1 | EIF3I | EIF2B5 | EIF4EBP2 |
| EIF1AD | EIF3J | EIF2D | EIF4EBP3 |
| EIF1AX | EIF3K | EIF2S1 | EIF4ENIF1 |
| EIF1AY | EIF3L | EIF2S2 | EIF4G1 |
| EIF1B | EIF3M | EIF2S3 | EIF4G2 |
| EIF2A | EIF4A1 | EIF2S3L | EIF4G3 |
| EIF2AK1 | EIF4A2 | EIF3A | EIF4H |
| EIF2AK2 | EIF4A3 | EIF3B | EIF5 |
| EIF2AK3 | EIF4B | EIF3C | EIF5A |
| EIF2AK4 | EIF4E | EIF3CL | EIF5A2 |
| EIF2B1 | EIF4E1B | EIF3D | EIF5AL1 |
| EIF2B2 | EIF4E2 | EIF3E | EIF5B |
| EIF2B3 | EIF4E3 | EIF3F | EIF6 |
| EIF2B4 | EIF4EBP1 | EIF3G | EIF3H |

DLBCL is caused by the abnormal multiplication of B-cells, which are very important parts of the lymphatic immune system. Like in other human cancers, this abnormal increase in the cell number of specific cells has detrimental effects on the body - leading eventually to the death of affected patients. Because the lymphatic system includes a great variety of different cell types the to be investigated B-cells have to be first of all isolated to be analyzed:
Cell suspensions from three biopsy specimens were separated by means of flow cytometry into a CD19+ malignant subpopulation and a CD19- nonmalignant subpopulation. Before the gene expression of the isolated B-cells could be investigated by microarray analysis, the RNA samples had to be prepared: Gene expression profiling was performed after two rounds of linear amplification from total RNA. To interpret the microarray results regarding gene expression the following adaptations were performed: After normalization to a median signal of 500, provided in the Affymetrix Microarray Suite software, version 5.0 (MAS5.0, Affymetrix, USA), genes were selected that had a signal value greater than 128 in either the CD19+ or CD19-fractions in at least two of the sorted samples.

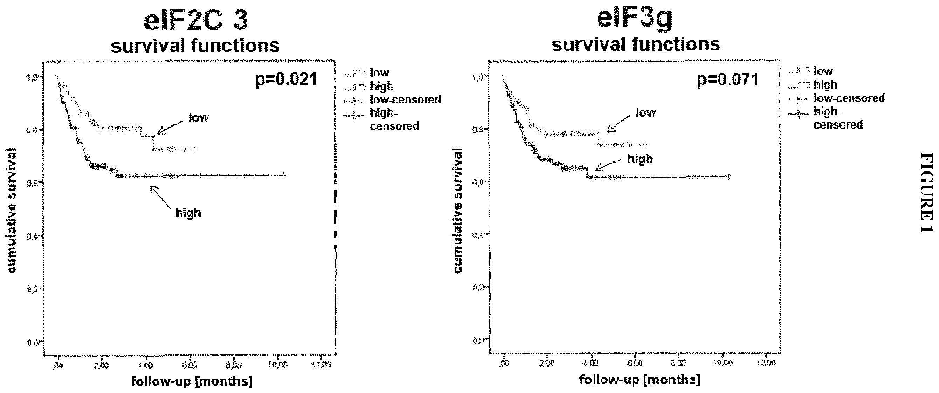

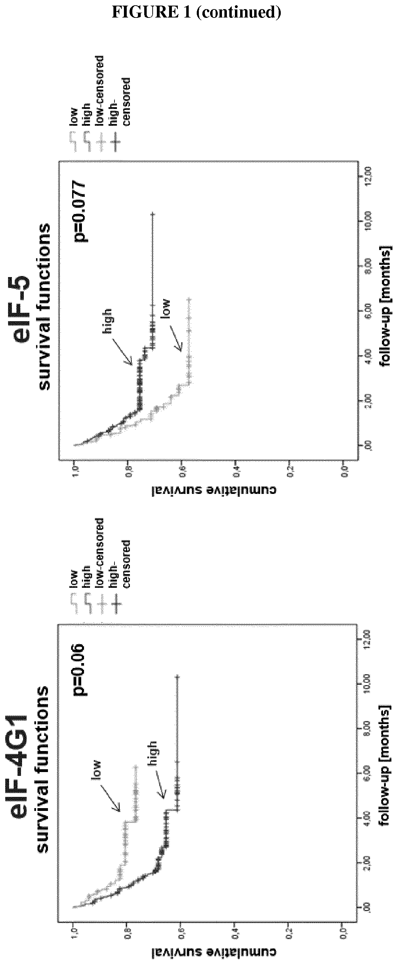

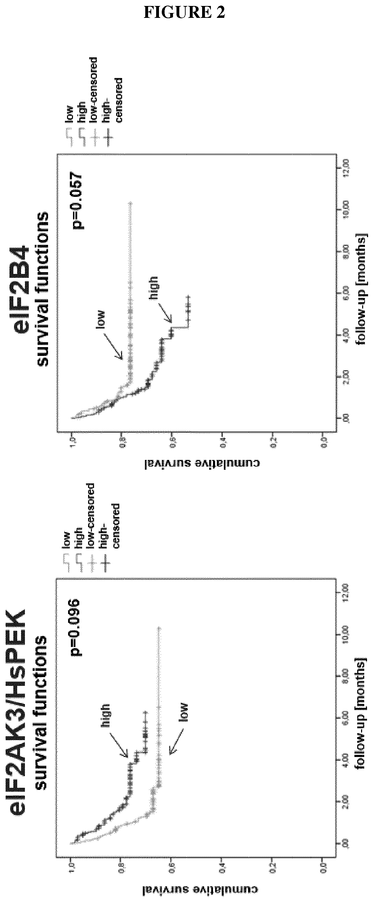

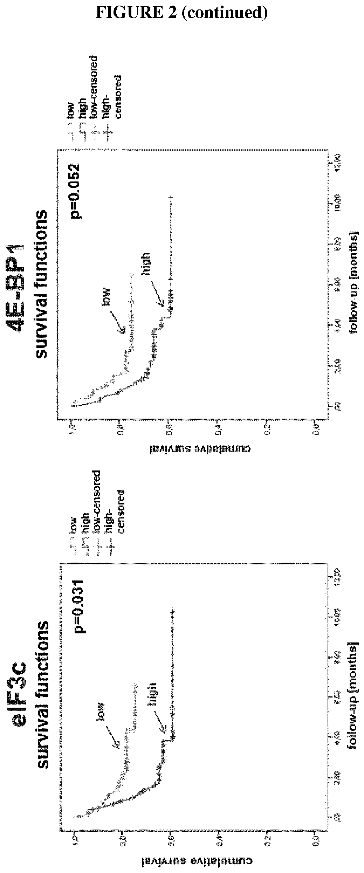

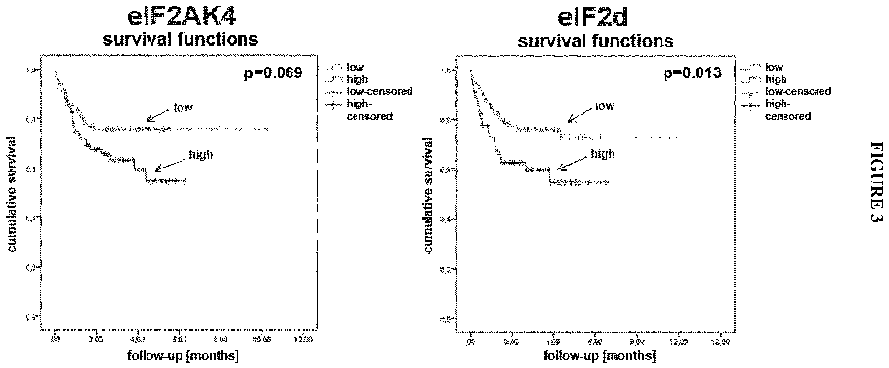

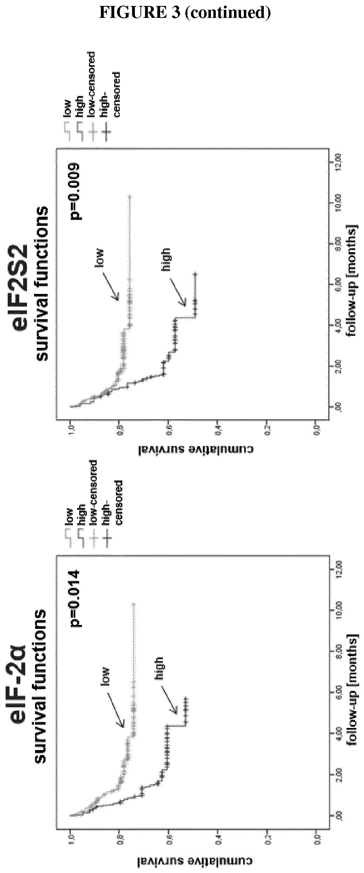

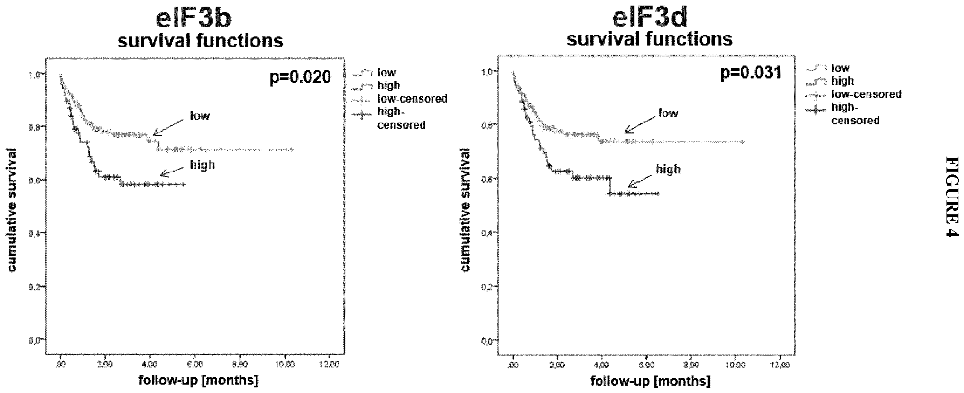

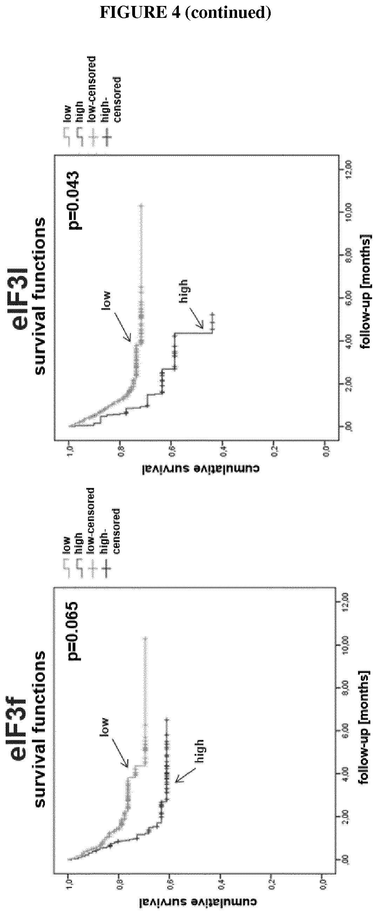

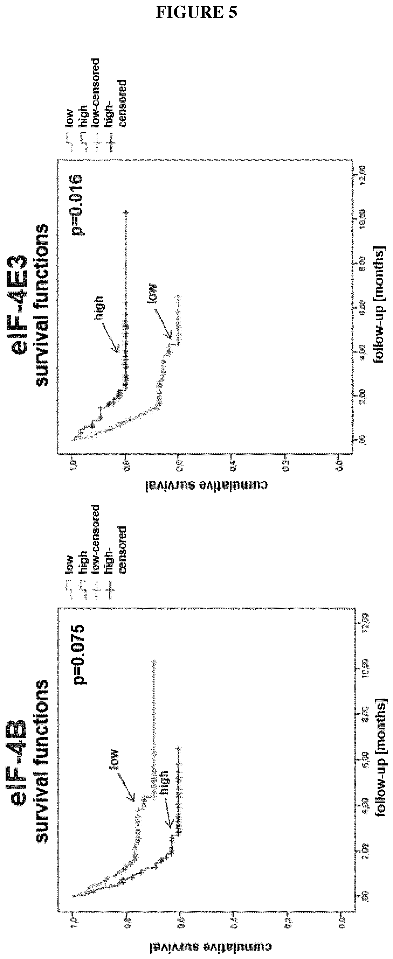

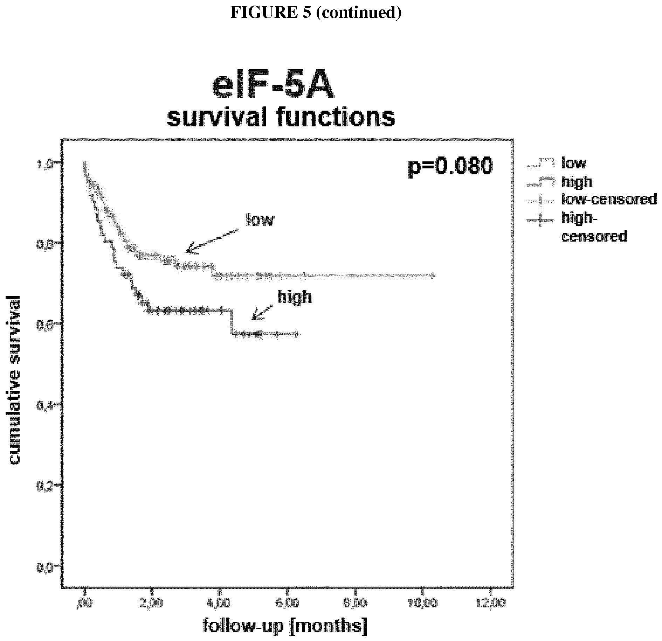

The 1^{st} quartile, median and 3^{rd} quartile refers to the cut off level for distinguishing high and low expression (see also Figure 1-5 and Table 2). This means that a patient with an elF expression higher than the 1^{st} quartile has a higher elF expression than the lowest elF expressing quarter of the complete range of patients tested. In contrast, a patient with an elF expression higher than the 3^{rd} quartile has an elF expression higher than three quarters of the tested patients (therefore a very high expression). To define significance a p-value of 0,05 was defined as significant.

### Results

As described above a previously published data set comprising gene expression and patient survival profiles in DLBCL (Lenz G et al.) was analyzed for correlations between elF mRNA expression and patient survival.
Indeed, we detected for several eIF-subunits a link between altered expression and better or worse patient outcome. The expression/survival correlations are illustrated in Figure 1-5.
In the case of 9 eIF-subunits there was even a significant correlation between lower subunit expression and better patient outcome. In contrast, for eIF-4E3 the data indicate that higher gene expression seems to be significantly more beneficial for patient survival. 10 further eIF-subunits showed expression-survival correlations too, which however were not statistically significant (p > 0,05).
The results of the survival analysis are summarized in Table 2:

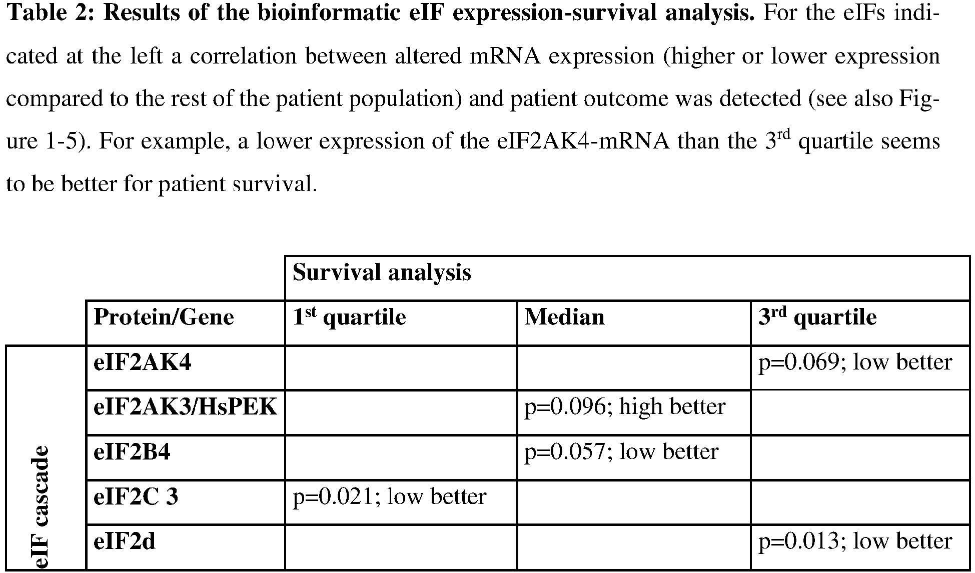

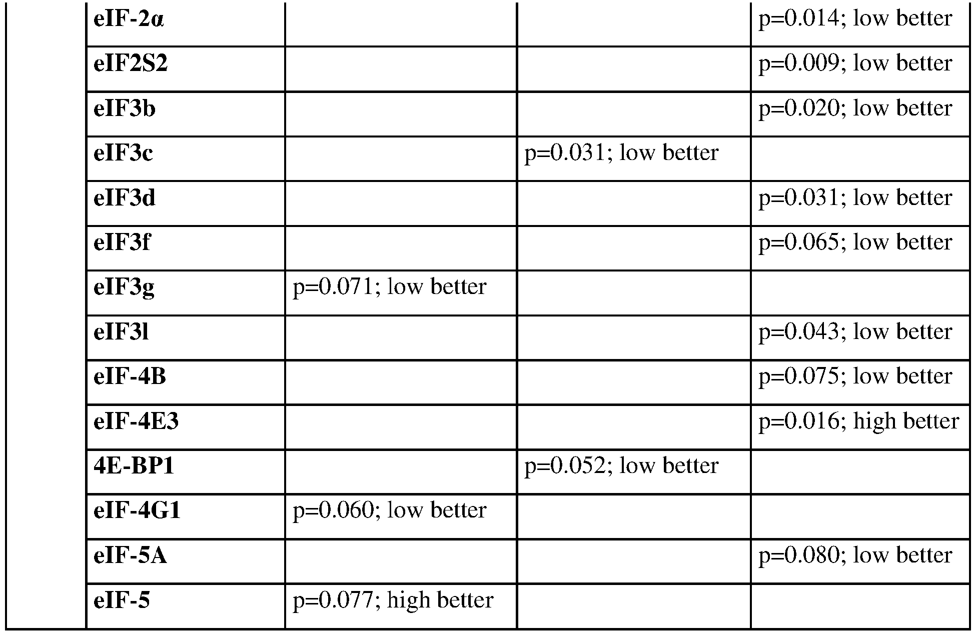

### Discussion

Till now, the research in the lymphoma area focused mainly on the eIF4F-complex. Therefore, research studies, investigating the complete range of eIFs, are lacking. This is the first analysis showing the relationship between the expression of a big range of various eIF-subunits and patient outcome.
We detected a potential for in the field previously unstudied eIFs to serve as biomarkers in lymphoma (Figs. 1 to 5 and Table 2).
By determining the expression levels of these eIFs in affected patients one could therefore give a prognosis for the patients analyzed. This would enable to divide a patient cohort in a high risk and a low risk group. According to the personal risk the patient could then be treated with stronger or more moderate chemotherapy, respectively. Similarly, patients of the high risk group could accordingly be supervised in a close meshed way for possible disease relapses after treatment and this would probably in order save lives.

### Example 2: Prognosis of individuals suffering from hepatocellular carcinoma (HCC) Materials and methods

### Human hepatocellular carcinoma samples

Formalin fixed paraffin embedded HCC samples and respective healthy control tissues from a total of 234 patients were collected and were used to generate 10 tissue microarrays (TMAs). The histological diagnosis, differentiation, and stage were classified according to the WHO classification (Hamilton S et al. Pathology and Genetics of Tumors of the Digestive System. World Health Organization Classification of Tumours International Agency for Research on Canccer (IARC) 2000; IARC Press).

### Tissue Microarrys (TMA)

All tumor tissue samples were acquired at the time of surgery, and immediately frozen in liquid nitrogen and stored at -80°C.
Every sample was stained for haematoxylin-eosin and examined to find relevant tumor areas which were marked on the slide. Tissue cones of the chosen tumor regions were punched out, assembled in an array structure and embedded into a fresh paraffin block, according to a specific pattern. The sections taken were 5µm thick, mounted on a specific adhesive-coated glass slide, compatible for immunohistochemical staining and analysis.

### Immunohistochemistry (IHC)

A summary of all used antibodies and the dilution to determine the expression of different **eIFs** is shown in the following Table 3:

**Table 3: Summary of all used antibodies and the dilution to determine the expression of different eIFs**

| **Primary Antibody** | **Company** | **Dilution** | **Second Antibody** |
|---|---|---|---|
| eIF4G | Cell Signaling (#2498) | 1:25 | Rabbit |
| eIF4E | Cell Signaling (#9742) | 1:100 | Rabbit |
| eIF2α (D7D3) XP | Cell Signaling (#5324) | 1:2000 | Rabbit |
| eIF3P110 (B-6) | Santa Cruz (sc-74507) | 1:250 | Mouse |
| eIF6 | Gene Tex (GTX63642) | 1:75 | Rabbit |
| eIF3M (V-21) | Santa Cruz (sc-133541) | 1:30 | Rabbit |
| eIF1 | Sigma Aldrich (HPA043003) | 1:50 | Rabbit |
| eIF3A | Cell Signaling (#2538) | 1:50 | Rabbit |
| eIF3B (eIF3η D-9) | Santa Cruz (sc-137215) | 1:100 | Mouse |
| eIF3H (D9C1) XP | Cell Signaling (#3413) | 1:750 | Rabbit |

Staining was performed using the Ventana Immunostainer XT (Ventana Medical Systems, USA). The endogenous peroxidase activity was inactivated in 3% hydrogen peroxide for 5 minutes. The primary antibodies were applied at different dilutions (see Table A) for 60 minutes, followed by incubation with a peroxidase-labelled secondary antibody for 30 minutes and substrate-chromogen 3,3'-diaminobenzidine tetrahydrochloride for 8 minutes. Counterstaining was performed with haematoxylin.
The intensity of IHC staining was evaluated by light microscopy. Density and intensity of each TMA spot was scored in a semi-quantitative manner by differentiating nuclear and cytoplasmic staining. The Total Immunostaining Score (TIS) was calculated in percent. No staining was termed as 0, weak staining as 1, moderate staining as 2 and strong staining as 3.

### Western Blot

Total protein extracts were collected, and lysed in NP-40 Lysis buffer (0.05 M Tris-HCl, 5 M NaCl, 0.5% NP-40, 0.1 M Pefabloc, 1 M DTT, complete Mini, PhosSTOP). The tissue samples were homogenized with a Potter tissue homogenizer (Kontes Glass Co, Duall 20). The protein concentration was determined using Bradford protein assay (Biorad Protein Assay Dye Reagent, 500-0006; BioRad Laboratories GmbH, Germany). Equal amounts of 30µg protein were loaded onto SDS-PAGE gels (30% Acrylamid/ Bisacrylamid solution; ROTH), subjected to electrophoresis in Mini-vertical electrophoresis units (Hoefer Inc, USA) and blotted onto PVDF membranes (Immobilin-P Transfer Membrane; Millipore, USA) using a Semi Dry Blotting Unit (SCIE-PLAS; Cambridge, England). The membranes were blocked in TBST with 5% non-fat milk (AppliChem; Germany) for 1h at room temperature. The primary antibodies were diluted in TBST, 5% BSA overnight at 4°C. The membranes were washed with TBST, the secondary antibody solutions for anti-mouse and anti-rabbit were acquired from Amersham. The interest proteins were detected using ECL Plus Western Blotting Detection Reagent (GE Healthcare; Buckinghamshire, England), followed by exposure on the Multilmage™ Light Cabinet (Alpha Innotech Corporation, USA).

### Statistical analysis

All values are represented as mean ± standard error of the mean if not indicated otherwise. Statistical significance was evaluated using Decision Trees and Kaplan Meier Curves.

### Results

### Immunohistochemistry of Tissue Microarrays

Density of the IHC staining was predominantly evaluated as 100%. In comparison to healthy liver tissue, several eIFs were highly upregulated in HCC tissue.
IHC staining for eIF2α, eIF3H, eIF3C, eIF4E and eIF6 revealed a weak to strong staining in the Healthy liver tissue and also in the HCC tissue.
For eIF5 the IHC staining displayed a high to moderate staining intensity in the HCC samples, whereas the intensity in healthy liver tissue was weak.

### Expression of eIF2α, eIF3C, eIF4E and eIF5 in HCC

Protein expression of eIF2α, eIF3C, eIF4E and eIF5 was significantly upregulated in HCC samples and HCC samples with a HCV infection compared to healthy liver tissue. The p-value for this calculation was 0.051.

### Statistical analysis

The Survival Curve according to t-Stage displayed a better survival with a lower score ≤ 2 than with a score of 3 with a p-value of 0,179. The survival is also better when the patient has one tumor compared to patients with two or more. Microvessel invasion is associated with a poor clinical outcome compared to patients without a microvessel invasion with a p-value of 0,067. The survival according to sex is better for women compared to man with a p-vale of 0.6. The differences between patients with and without HBV (Hepatitis B virus) infection showed no changes in the survival. In comparison to HCV (Hepatitis C virus) patient survival for these are poor compared to patients without HCV infection.

### Discussion

Liver cancer is the second leading cause of cancer mortality worldwide, with approximatley 600,000 cancer releated deaths. Altered translation initiation and abnormal gene expression increase the risk of cancer development. Previous studies displayed, that deregulation along the elF cascade disassociated with malignant transformation and progression of cancer. The goal in this example was to analyze the contribution of various eIFs to find a link between translation initiation and carcinogenesis.
eIF5A, an indispensable member of the translation initiation process, is found to be aberrantly expressed in different malignancies including HCC, ovarian cancer, and lung cancer. One of its isoforms, eIF5A2, is overexpressed in HCC tissues, and this up-regulation may be a result of chromosome 3q amplification where the eIF5A2 gene resides. Clinical studies have demonstrated a correlation between up-regulation of eIF5A2 level with tumor metastasis and venous infiltration. Therefore, eIF5A2 has been proposed as an indicator of tumor invasiveness in HCC. In addition, targeting eIF5A2 by siRNA and combined treatment with GC7 effectively reduces the migration ability of tumor cells, suggesting that targeting eIF5A2 and hypusination could be a potential treatment for HCC.
eIF4E is involved in the regulation of the mRNA translation process. It can enhance the translation of some important growth factors and cell growth regulators and affect protein synthesis, the cell cycle, cancer gene activation, and apoptosis; it also play an important role in malignant transformation and metastasis. eIF4E regulates the translation of cancer-related mRNAs that are involved in tumor occurrence and development.
The involvements of eIFs in cancer formation has been suggested and already, at least in part, have been proven for many elF subunits and various tumor entities. eIFs can play a role, depending on the particular subunit and the respectively evaluated tissue types, in tumor development. The network of eIFs seems to display all elements of an entire oncogenic as well as tumor suppressive cascade. This thereby implicates enhanced elF activation in HCC progression and suggests that eIFs may be an attractive target for HCC therapy.

### Example 3: Prognosis of individuals suffering from lymphoma

We further adjusted the patient cohort of example 1 and performed an additional survival analysis (for a description of the used methods see example 1). The results are depicted in Table 4.
Table 4 includes besides further elF subunits, showing expression-survival correlations too, also an update of some of the factors shown in Table 2.

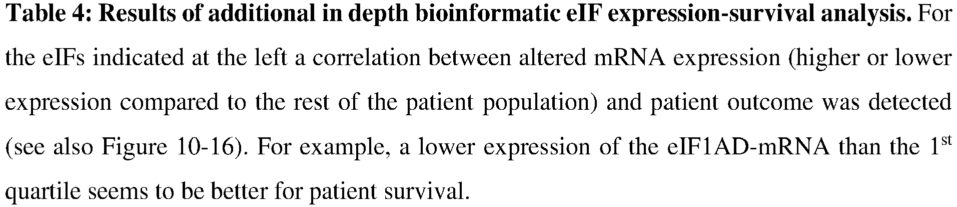

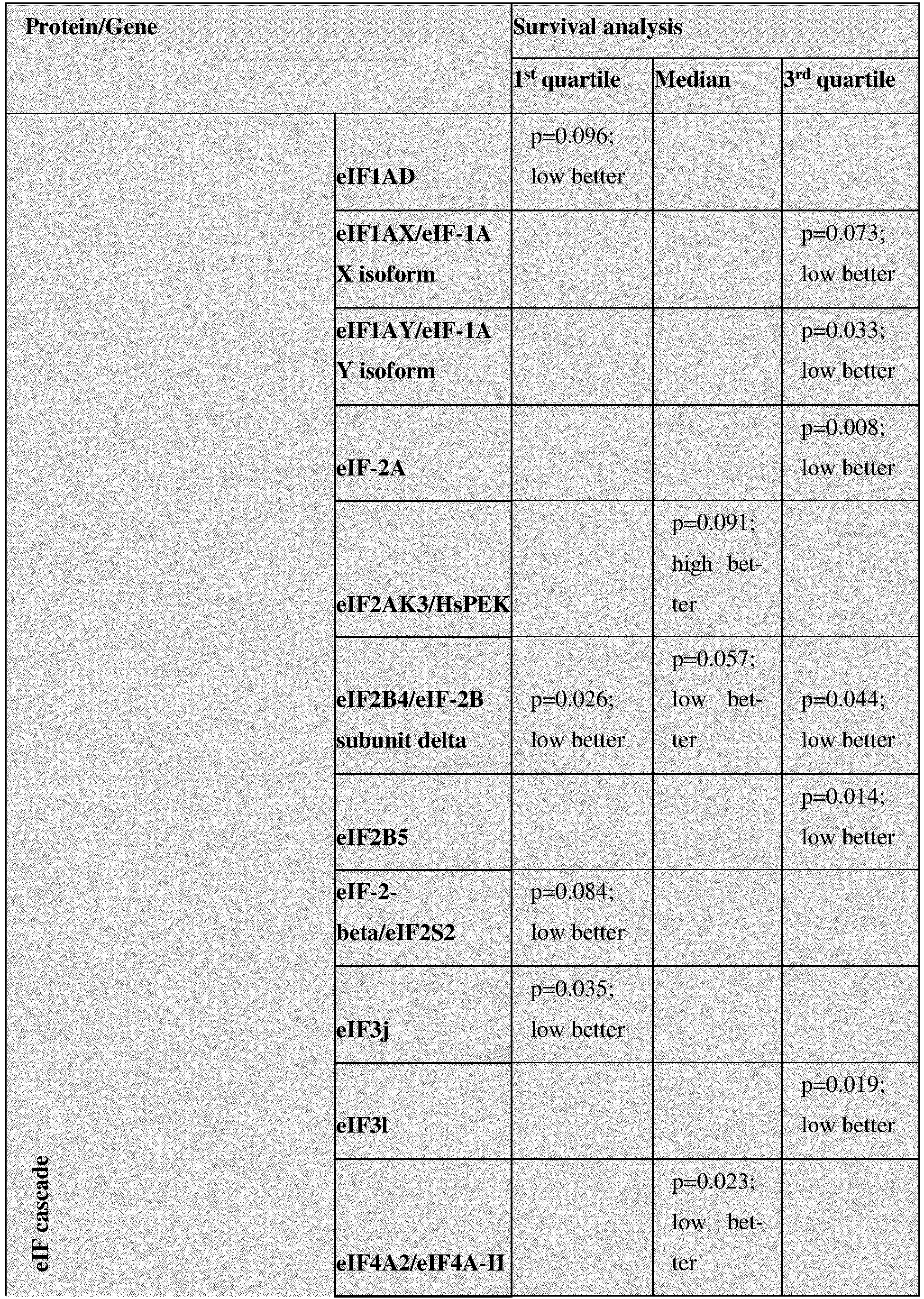

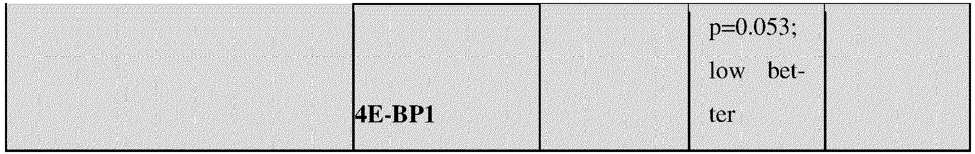

### Example 4: Diagnosis of a HCC, a HCV infection, a HBV infection, a viral induced HCC and differential diagnosing between these diseases or conditions

peIF2α, eIF2α, eiF3B, eIF3D, eIF3J, peIF4B, eIF4G and eIF6 were upregulated in HCV-associated HCC. eIF2α, peIF4B, eIF5 and various eIF3 subunits were significantly increased in HBV-associated HCC. HCC without viral background displayed a significant increase for the elF subunits p2α, 3C, 31, 4E and 4G. We noticed dramatic differences in the expression pattern between chronic hepatitis B and C, HBV- and HCV associated HCC and non-virus related HCC. In this respect, it is referred to Figures 17 and 18.
Patients with HBV and HCV infection with high eIF2α expression and as well patients without HBV and HCV showed a better survival. For eIF3H, we observed the best survival in patients with a low eIF3H score and without HBV. HCV infected patients with a high eIF3H score had a poor prognosis. For eIF3C we did not observed any differences in the survival with a high or low score and no significant differences neither with nor without HBV background. The survival according to eIF3C in HCV showed no significant differences with or without HCV infection. For eIF4E we did not observed any differences in the survival with a high or low score and no significantly differences with or without HBV infection background. Patients with HCV displayed a shorter survival compared to patients without HCV infection, but eIF4E did not influence the survival in these patients. A high score of eIF5 showed a significantly influence on survival in HBV infected patients and in patients without HBV infection. The eIF5 score did not have so much influence in HCV positive patients. The survival in HCV infected patients is poor compared to patients without HCV induced hepatitis .Taking into account eIF6 expression and HBV positivity showed no significant differences in the patient overall survival. HCV infected patients displayed a poor survival with a high score of eIF6.

We noticed dramatic differences in the elF expression and chronic hepatitis C but no changes in chronic hepatitis B.

### Example 4: Diagnosis of a lymphoma

### Material and methods

In addition to the survival analysis based on the Lenz-dataset, we also analyzed a local DLBCL patient cohort consisting of 56 patients on mRNA level for patient survival. Furthermore, we also investigated the DLBCL tissue specimens in comparison with their cells of origin, non-neoplastic germinal center B-cells.

### Tissue specimens

DLBCL patient tissue was gathered during routine diagnostics. Non-neoplastic germinal center B-cells were isolated from non-neoplastic tonsils gathered in course of routine tonsillectomies. For this purpose, FACS cell sort was used to select the high CD20⁺ and high CD38⁺ B-cell population.

### qRT-PCR

RNA extraction from fresh frozen DLBCL patient tissue and germinal center B-cells, respectively, was performed using QIAzol Lysis reagent (Qiagen) and miRNeasy Mini Kit (Qiagen) according to the manufacturer's instructions. RNA concentration and quality were determined with the BioPhotometer (Eppendorf). The isolated RNA was then transcribed into cDNA using the cDNA-synthesis kits RevertAid H Minus First Strand cDNA Synthesis Kit (Thermo Fisher) and High Capacity cDNA Reverse Transcription Kit (Applied Biosystems), respectively, according to the manufacturer's instructions.

Obtained cDNA was afterwards used for quantitative real-time PCR (qRT-PCR) approaches on the C1000 Touch Thermal Cycler CFX 384 Real-Time System (Bio-Rad) using the GoTaq qPCR Master Mix (Promega). Each real-time PCR reaction was composed of 5µl Master Mix (Promega), 0,01µl forward and reverse primer for respective gene detection, 1µl aqua dest and 4µl of 1:20 diluted cDNA. The used primer pairs were synthesized by Eurofins Genomics: eIF1AX/Y fwd: AACAGACGCAGGGGTAAGAAT (SEQ ID NO: 1), eIF1AX/Y rev: CCTGAGCATACTCCTGACCAT (SEQ ID NO: 2), eIF2AK3 fwd: TGATGTTGTTTT-GGTTGGAGGA (SEQ ID NO: 3), eIF2AK3 rev: TACCTCACCTTTCCACTATATGC (SEQ ID NO: 4), eIF2B4 fwd: GGTGTATTGCCCTGCTTCGT (SEQ ID NO: 5), eIF2B4 rev: CAG-GAAGCTCATGTAGGGTTTT (SEQ ID NO: 6), eIF2B5 fwd: TTCTGGTGGCCGA-TAGCTTC (SEQ ID NO: 7), eIF2B5 rev: AGCTTTCCAGCAACAAAAGACA (SEQ ID NO: 8), eIF4A2 fwd: GAAGCCTTCCGCTATTCAGCA (SEQ ID NO: 9), eIF4A2 rev: CTT-GGGTCTCCTTGAACTCAATC (SEQ ID NO: 10). Note that the primer pair for eIFIA detection was specific for the eIF1AX and eIF1AY gene and therefore allows a detection of both mRNAs at the same time. The following parameters were used for the qRT-PCR program: Hot-Start Activation (95°C for 2 minutes), 40 cycles of denaturation and annealing/extension (95°C for 15 seconds, 60°C for 30 seconds), followed by a dissociation phase (60-95°C). Threshold cycles (C(t)) were automatically calculated by the C1000 Touch Thermal Cycler CFX 384 Real-Time System software (Bio-Rad). RNA expression was evaluated using the ΔΔC(t)-method. As housekeeping gene a combination of Actin and GAPDH was used. Therefore the geometric mean of the C(t) values of both housekeeping genes was calculated and used for the ΔΔC(t)-calculation.

### Results

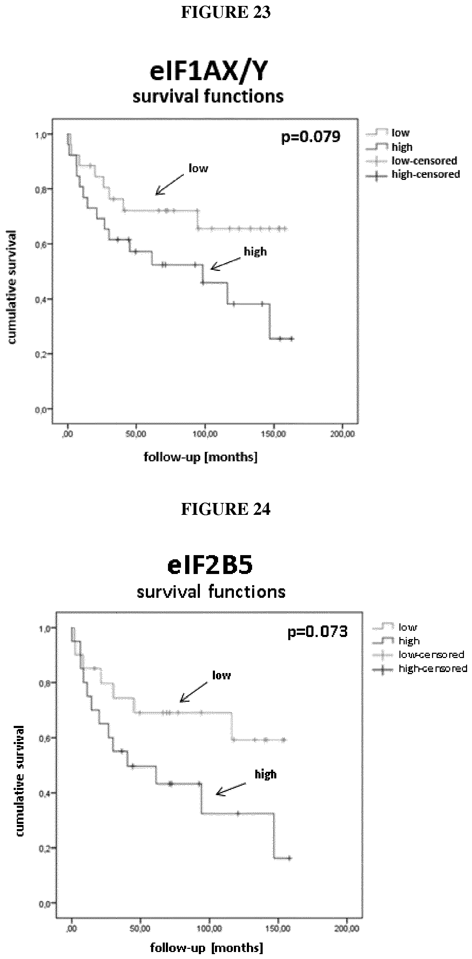

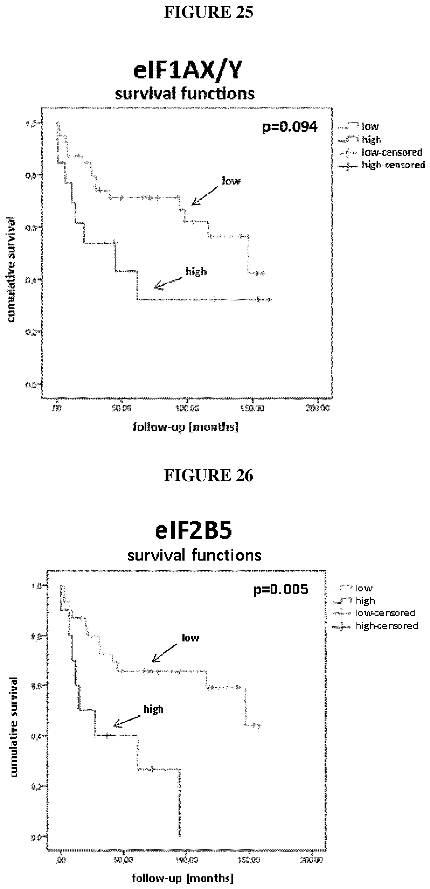

Like in the Lenz-dataset eIF2B5 showed also in our local patient cohort a significant link between lower expression and better patient survival (Figure 24 and Figure 26). In addition, also eIF1AX/Y showed a (however non-significant) association between lower factor expression and better patient survival (similar to the Lenz-dataset, where we analyzed eIF1AX and eIF1AY, however, separately; Figure 23 and Figure 25).

DLBCL arises from B-cells within the so called germinal centers of lymphatic organs that have undergone a neoplastic transformation, leading to uncontrolled cellular growth. We compared elF expression levels of healthy, non-neoplastic germinal center B-cells (GCs) with those of their diseased counterparts, the DLBCL cells of the patients of our local cohort, to test, whether elF expression allows discrimination between healthy B-cells and diseased DLBCL cells (Figure 27).

Thereby we also distinguished between the different subtypes of DLBCL: the germinal center B-cell subtype and the non-germinal center B-cell subtype (nGCB). We further classified the germinal center B-cell subtype into the primary germinal center B-cell disease (pGCB) and the secondary germinal center B-cell disease, that arose going out from a Follicular lymphoma grade III (FLIII-GCB).

The analysis showed indeed a significant overexpression of eIF1AX/Y, eIF2AK3 and eIF4A2 in all different subtypes of DLBCL in comparison with the non-neoplastic germinal center B-cells. eIF2B4 and eIF2B5 showed at least a significantly higher expression in the FLIII-GCB subtype and the nGCB subtype, but no significant overexpression in comparison with the pGCB-subtype, although also here a higher expression was visibile. Nevertheless, this data strongly indicates that the mentioned eIFs could be not only used to predict patient outcome but also in diagnostics to distinguish non-neoplastic, healthy from tumor tissue.

## Claims

1. A method of providing a prognosis to an individual suffering from a tumor comprising the steps of:
a) determining the level of eIF2AK4 in a sample from an individual, and
b) comparing the level of eIF2AK4 in said sample to a reference level A of eIF2AK4 determined in samples of patients suffering from the same tumor or to a reference level B of eIF2AK4 determined in samples of healthy individuals, wherein a decreased level of eIF2AK4 in the sample of said individual compared to the reference level A and/or B indicates a good prognosis,
wherein the tumor is a lymphoma or a hepatocellular carcinoma.

2. The method of claim 1, wherein the sample is a tumor tissue or a body fluid.

3. The method of claims 1 or 2, wherein the level of eIF2AK4 is determined by measuring mRNA or protein levels.

4. Use of a kit comprising means for determining the level of eIF2AK4 in a sample from an individual for providing a prognosis to the individual suffering from a tumor, wherein the tumor is a lymphoma or a hepatocellular carcinoma.

5. The use of claim 4, wherein the sample is a tumor tissue or a body fluid.

6. The use of claims 4 or 5, wherein the level eIF2AK4 is determined by measuring mRNA or protein levels.

## Patentansprüche

1. Verfahren zur Bereitstellung einer Prognose eines an einem Tumor leidenden Individuums, umfassend die Schritte:
a) Bestimmen des Levels von eIF2AK4 in einer Probe eines Individuums, und
b) Vergleichen des Levels von eIF2AK4 in der Probe mit einem Referenzlevel A von eIF2AK4, bestimmt in Proben von Patienten, die an demselben Tumor leiden, oder mit einem Referenzlevel B von eIF2AK4, das in Proben von gesunden Individuen bestimmt wurde,
wobei ein vermindertes Level von eIF2AK4 in der Probe des Individuums gegenüber dem Referenzlevel A und/oder B eine gute Prognose anzeigt, wobei der Tumor ein Lymphom oder ein hepatozelluläres Karzinom ist.

2. Verfahren nach Anspruch 1, wobei die Probe ein Tumorgewebe oder eine Körperflüssigkeit ist.

3. Verfahren nach Ansprüchen 1 oder 2, wobei das Level von eIF2AK4 durch Messung des mRNA- oder Proteinlevels bestimmt wird.

4. Verwendung eines Kits zur Bereitstellung einer Prognose eines an einem Tumor leidenden Individuum, umfassend Mittel zur Bestimmung des Levels von eIF2AK4 in einer Probe des Individuums, wobei der Tumor ein Lymphom oder ein hepatozelluläres Karzinom ist.

5. Verwendung nach Anspruch 4, wobei die Probe ein Tumorgewebe oder eine Körperflüssigkeit ist.

6. Verwendung nach Ansprüchen 4 oder 5, wobei das Level von eIF2AK4 durch Messung des mRNA- oder Proteinlevels bestimmt wird.

## Revendications

1. Méthode permettant de fournir un pronostic à un individu souffrant d'une tumeur comprenant les étapes de :
a) détermination du taux d'eIF2AK4 dans un échantillon provenant d'un individu, et
b) comparaison du taux d'eIF2AK4 dans ledit échantillon à un taux de référence A d'eIF2AK4 déterminé dans des échantillons de patients souffrant de la même tumeur ou à un taux de référence B d'eIF2AK4 déterminé dans des échantillons d'individus en bonne santé, un taux réduit d'eIF2AK4 dans l'échantillon dudit individu par rapport au taux de référence A et/ou B indiquant un pronostic favorable,
ladite tumeur étant un lymphome ou un carcinome hépatocellulaire.

2. Méthode selon la revendication 1, l'échantillon étant un tissu tumoral ou un fluide corporel.

3. Méthode selon la revendication 1 ou 2, le taux d'eIF2AK4 étant déterminé en mesurant les taux d'ARNm ou de protéines.

4. Utilisation d'un kit comprenant un moyen permettant de déterminer le taux d'eIF2AK4 dans un échantillon provenant d'un individu pour fournir un pronostic à l'individu souffrant d'une tumeur, ladite tumeur étant un lymphome ou un carcinome hépatocellulaire.

5. Utilisation selon la revendication 4, ledit échantillon étant un tissu tumoral ou un fluide corporel.

6. Utilisation selon la revendication 4 ou 5, ledit taux d'eIF2AK4 étant déterminé en mesurant les taux d'ARNm ou de protéines.


### SEQUENCE LISTING

<110> CBmed GmbH
<120> METHOD FOR PROGNOSING AND DIAGNOSING TUMORS
<130> 973-8 PCT
<160> 10
<170> PatentIn version 3.5
<210> 1
   <211> 21
   <212> DNA
   <213> synthetic
<400> 1
   aacagacgca ggggtaagaa t 21
<210> 2
   <211> 21
   <212> DNA
   <213> synthetic
<400> 2
   cctgagcata ctcctgacca t 21
<210> 3
   <211> 22
   <212> DNA
   <213> synthetic
<400> 3
   tgatgttgtt ttggttggag ga 22
<210> 4
   <211> 23
   <212> DNA
   <213> synthetic
<400> 4
   tacctcacct ttccactata tgc 23
<210> 5
   <211> 20
   <212> DNA
   <213> synthetic
<400> 5
   ggtgtattgc cctgcttcgt 20
<210> 6
   <211> 22
   <212> DNA
   <213> synthetic
<400> 6
   caggaagctc atgtagggtt tt 22
<210> 7
   <211> 20
   <212> DNA
   <213> synthetic
<400> 7
   ttctggtggc cgatagcttc 20
<210> 8
   <211> 22
   <212> DNA
   <213> synthetic
<400> 8
   agctttccag caacaaaaga ca 22
<210> 9
   <211> 21
   <212> DNA
   <213> synthetic
<400> 9
   gaagccttcc gctattcagc a 21
<210> 10
   <211> 23
   <212> DNA
   <213> synthetic
<400> 10
   cttgggtctc cttgaactca atc 23